# EUROPEAN PATENT SPECIFICATION

(11) **EP 2 804 631 B1**
(45) Date of publication and mention of the grant of the patent: **17.03.2021**
(21) Application number: 13701560.8
(22) Date of filing: 18.01.2013
(51) Int. Cl.: A61K 38/05, A61K 31/5383, A61K 47/68, A61P 35/00

(54) **SURROBODY CONJUGATES**
SURROBODY-KONJUGATE
SURROBODY CONJUGUÉS

(30) Priority: 20.01.2012 US 201261589272 P; 02.01.2013 US 201361848379 P
(43) Date of publication of application: 26.11.2014
(73) Proprietor: i2 Pharmaceuticals, Inc., Boulder, CO 80301 (US)
(72) Inventor: HOROWITZ, Lawrence, C., Atherton, CA 94027 (US); BHATT, Ramesh, Belmont, CA 94002 (US); HANNUM, Charles, Atherton, CA 94027 (US); FOREMAN, Pamela, Atherton, CA 94027 (US); CAI, Danying, Atherton, CA 94027 (US); GORE, Medini, Atherton, CA 94027 (US); XU, Li, Atherton, CA 94027 (US); KOBEL, Phil, Atherton, CA 94027 (US)
(74) Representative: Lavoix
(86) International application number: PCT/US2013/022308
(87) International publication number: WO 2013/109994

(56) References cited:
- WO-A1-2011/071957
- WO-A2-2011/153431
- XU L ET AL: "Surrobodies with Functional Tails", JOURNAL OF MOLECULAR BIOLOGY, ACADEMIC PRESS, UNITED KINGDOM, vol. 397, no. 1, 19 March 2010 (2010-03-19), pages 352-360, XP026929320, ISSN: 0022-2836, DOI: 10.1016/J.JMB.2010.01.036 [retrieved on 2010-01-25] cited in the application
- XU LI ET AL: "Combinatorial surrobody libraries", PROCEEDINGS OF THE NATIONAL ACADEMY OF SCIENCES, NATIONAL ACADEMY OF SCIENCES, US, vol. 105, no. 31, 5 August 2008 (2008-08-05), pages 10756-10761, XP002498064, ISSN: 0027-8424, DOI: 10.1073/PNAS.0805293105

## Description

### Related Applications

This application claims the benefit of U.S. Provisional Application Nos. 61/589,272 filed January 20, 2012 and 61/848,379 filed January 2, 2013.

### Field of the Invention

The present application discloses methods for identifying, producing, and engineering binding molecule conjugates, and the conjugates produced. In particular, the application discloses Surrobody conjugates composed of an antibody heavy chain variable domain and a surrogate light chain, wherein the surrogate light chain or the antibody heavy chain variable domain is conjugated to a therapeutic or diagnostic agent. The invention concerns a binding molecule conjugate comprising a surrogate light chain (SLC) polypeptide having at least one agent linked to the SLC polypeptide.

### Background of the Invention

Significant efforts have been directed to the development of antibodies conjugated to a variety of therapeutic or diagnostic agents. The use of antibody-drug conjugates or immunoconjugates for the targeted delivery of an anti-cancer agent is an area of great interest. Because many anti-cancer drugs target the cell cycle and kill rapidly proliferating cells, they do not discriminate between healthy and tumorous tissue. This lack of discrimination results in narrow therapeutic indices and causes severe treatment-related side effects that limit efficacy, because concentrations of drug that would significantly impact tumor growth are intolerable. Conjugating such potent molecules to specific antibodies has proven successful in delivering these potent molecules to the tumor site via the selective nature of the antibodies, at levels that effectively counter tumor growth and importantly limit exposure to noncancerous tissues/cells.

Linking such anti-cancer drugs to antibodies is achieved by several types of compatible amino acid side chain chemistry, typically through lysine amines and cysteine sulfhydryls. Aside from selecting appropriate cytotoxic agents and linkers, the challenge for all approaches remains at least two-fold. The first challenge is due to the fact that each new targeting antibody can be vastly different from the previous, with respect to the nature of their heavy and light chains, and the resulting composition of naturally occurring side chain chemistries can vary substantially. The net result is that distinct conjugating efforts are expected for nearly every new antibody. Secondly, as the heavy chain is the most likely target of most conjugation chemistries and that it is predominantly responsible for the specificity and stability of antibodies, chemical derivatization of this chain is inherently more likely to have deleterious effects on the specificity and stability of the antibody. There remains a need for alternative formats for immunoconjugates.

SURROBODIES™ (Surrobglobulins) are a new class of binding molecules which utilize surrogate light chain sequences. Surrobodies are based on the pre-B cell receptor (pre-BCR), which is produced during normal development of antibody repertoire. Precursors of B cells (pre-B cells) have been identified in the bone marrow by their production of a set of genes called VpreB(1-3) and λ5, instead of the fully developed light chains, and coexpression of µ heavy chains. The VpreB and λ5 polypeptides together form a non-covalently associated, Ig light chain-like structure, which is called the surrogate light chain or pseudo light chain. Both VpreB and λ5 are encoded by genes that do not undergo gene rearrangement and are expressed in early pre-B cells before V(D)J recombination begins. The pre-BCR is structurally different from a mature immunoglobulin in that it is composed of a heavy chain and two non-covalently associated proteins: VpreB and λ5, i.e., they have three components as opposed to two in antibodies.

A κ-like B cell receptor (κ-like BCR) has also been identified, utilizing a κ-like surrogate light chain (κ-like SLC) (Frances et al., EMBO J 13:5937-43 (1994); Thompson et al., Immunogenetics 48:305-11 (1998); Rangel et al., J Biol Chem 280:17807-14 (2005)). Rangel et al., *supra* report the identification and molecular characterization of a Vκ-like protein that is the product of an unrearranged *V*κ gene, which turned out to the be identical to the cDNA sequence previously reported by Thompson et al., *supra.* Whereas, Frances et al., *supra* reported the identification and characterization of a rearranged germline JCk that has the capacity to associate with µ heavy chains at the surface of B cell precursors, thereby providing an alternative to the λ5 pathway for B cell development. It has been proposed that κ-like and λ-like pre-BCRs work in concert to promote light chain rearrangement and ensure the maturation of B cell progenitors. For a review, see McKeller and Martinez-Valdez Seminars in Immunology 18:4043 (2006).

Further details of the design and production of Surrobodies® (Surroglobulins) are provided in Xu et al., Proc. Natl. Acad. Sci. USA 2008, 105(31):10756-61, Xu et al., J. Mol. Biol. 2010, 397, 352-360, and in PCT Publication Nos. WO 2008/118970 published on October 2, 2008; WO/2010/006286 published on January 14, 2010; WO/2010/151808 published on December 29, 2010; PCT/US2012/044746 filed June 28, 2012.

In the design of a traditional antibody-based immunoconjugate, the ability to avoid conjugation of the heavy chain is highly desirable; furthermore the ability to target invariant sites on the heavy chain partner would be ideal. The surrogate light chain of the Surrobody provides such an ideal opportunity. As the surrogate light chain is non-diverse the composition of opportunistic naturally occurring side chains would remain unchanged from one surrobody to the next. As a result, it would be possible to direct conjugate chemistries exclusively to the surrogate light chain and utilize a single derivatization strategy for each subsequent new Surrobody.

### Summary of the Invention

In one aspect, the present invention provides a binding molecule conjugate comprising a surrogate light chain (SLC) polypeptide. The SLC polypeptide has at least one agent linked to an amino acid residue of the SLC polypeptide at a predetermined amino acid position. The amino acid residue is (i) normally present in the SLC polypeptide at said amino acid position, and/or (ii) introduced into the amino acid sequence of said SLC polypeptide at said amino acid position, wherein the agent is a therapeutic agent and is selected from the group consisting of a maytansinoid, a monomethyl auristatin E (MMAE) and a monomethyl auristatin F (MMAF).

In another aspect, the binding molecule conjugate comprises a binding molecule that is a Surrobody. The Surrobody comprises an SLC polypeptide. In another embodiment, the SLC polypeptide comprises a VpreB sequence and/or λ5 sequence conjugated to a heterologous amino acid sequence. In an additional embodiment, the SLC polypeptide comprises a VpreB sequence. In another embodiment, the heterologous amino acid sequence comprises a λ5 sequence. In other embodiments, the λ5 sequence is fused to the VpreB sequence. In some embodiments, the SLC polypeptide comprises a fusion of a VpreB sequence to a heterologous amino acid sequence. In one embodiment, the heterologous amino acid sequence is a λ5 sequence or an antibody light chain variable region sequence. In one other embodiment, the antibody light chain variable region sequence is fused to said VpreB sequence at a site analogous to an antibody light chain CDR3 region, or the CDR3 region of said antibody light chain variable region sequence is fused to said VpreB sequence at a site analogous to said CDR3 region. In other embodiments, the antibody light chain is a λ chain or a a κ chain. In one other embodiment, the heterologous amino acid sequence further comprises an antibody light chain constant region sequence. In an additional embodiment, the constant region sequence is the entire constant region of an antibody light chain.

In one other embodiment, the Surrobody comprises an SLC polypeptide, wherein the SLC polypeptide comprises a VpreB sequence and/or λ5 sequence conjugated to a heterologous amino acid sequence, wherein the SLC polypeptide comprises a λ5 sequence. In one embodiment, the heterogeneous amino acid sequence is a VpreB sequence conjugated to said λ5 sequence. In another embodiment, the conjugation is a fusion of said λ5 and VpreB sequences. In other embodiments, the conjugation is a covalent linkage between said λ5 and VpreB sequences. In some embodiments, the covalent linkage is formed by a connecting peptide or polypeptide sequence.

In one additional embodiment, the SLC polypeptide comprises a VpreB sequence and a λ5 sequence, wherein said conjugation is non-covalent association. In another embodiment, at least one of said VpreB and λ5 sequences is a fragment or variant of a native VpreB and λ5 sequence, respectively.

In one embodiment, the Surrobody comprises an SLC polypeptide, wherein the SLC polypeptide comprises a VpreB sequence and/or λ5 sequence conjugated to a heterologous amino acid sequence, wherein the SLC polypeptide is further conjugated to a second heterologous amino acid sequence. In one other embodiment, the SLC polypeptide comprises a fusion of a VpreB sequence to a λ5 sequence, covalently associated with said second heterologous amino acid sequence. In another embodiment, the second heterogeneous polypeptide sequence is an antibody heavy chain sequence comprising a variable region. In one embodiment, the antibody heavy chain sequence is conjugated to the fusion of said VpreB sequence and said λ5 sequence. In another embodiment, the conjugation of said VpreB sequence and said λ5 sequence is (i) by a peptide linker; or (ii) by non-covalent association, to form a dimeric complex. In some embodiments, the antibody heavy chain variable region sequence binds to the same target as said SLC polypeptide, or the antibody heavy chain variable region sequence binds to a target different from the target to which said SLC polypeptide binds. In another embodiment, the Surrobody further comprises a polypeptide comprising one or more functionally null binding regions conjugated to the dimeric complex. In another embodiment, the polypeptide comprises an antibody heavy chain amino acid sequence.

In one other embodiment, the Surrobody includes an SLC polypeptide that comprises a VpreB sequence and a λ5 sequence, wherein said conjugation is non-covalent association, and wherein the Surrobody further comprises an antibody heavy chain sequence comprising a variable region, non-covalently associated with the non-covalently associated VpreB sequence and λ5 sequences, to form a trimeric complex. In another embodiment, the antibody heavy chain comprises variable region sequences binding to the same target as said SLC polypeptide, or the antibody heavy chain comprises variable region sequences binding to a target different from the target to which said SLC polypeptide binds. In one other embodiment, the Surrobody further comprises a polypeptide comprising one or more functionally null binding regions conjugated to the trimeric complex. In another embodiment, the polypeptide comprises an antibody heavy chain amino acid sequence.

The binding molecule conjugate is based upon a binding molecule that comprises a surrogate light chain (SLC) polypeptide. The SLC polypeptide has at least one agent linked to an amino acid residue of the SLC polypeptide at a predetermined amino acid position. The amino acid residue is (i) normally present in the SLC polypeptide at said amino acid position, and/or (ii) introduced into the amino acid sequence of said SLC polypeptide at said amino acid position, wherein the agent is a therapeutic agent and is selected from the group consisting of a maytansinoid, a monomethyl auristatin E (MMAE) and a monomethyl auristatin F (MMAF). In one other embodiment, the amino acid residue is introduced into the amino acid sequence of the SLC polypeptide by a substitution or an insertion. In an additional embodiment, the amino acid residue is a cysteine or a lysine. In other embodiments, the amino acid residue is selected from the group consisting of a cysteine, a lysine, and a para-acetyl-phenylalanine (pAcF). In one other embodiment, the binding molecule is capable of binding at least one target or at least two targets. In some embodiments, the linker is a non-cleavable linker or a cleavable linker. In other embodiments, the non-cleavable linker comprises a maleimido-based moiety or a haloacetyl-based moiety. In one embodiment, the cleavable linker is selected from the group consisting of a peptidyl linker, a pH-sensitive linker, and a linker cleavable under reducing conditions. In another embodiment, the peptidyl linker comprises a dipeptide linker. In one other embodiment, the linker is selected from the group consisting of N-succinimidyl 4-(maleimidomethyl)cyclohexanecarboxylate (SMCC), N-succinimidyl-4-(N-maleimidomethyl)-cyclohexane-1-carboxy-(6-amidocaproate), (LC-SMCC), κ-maleimidoundecanoic acid N-succinimidyl ester (KMUA), γ-maleimidobutyric acid N-succinimidyl ester (GMBS), ε-maleimidocaproic acid N-hydroxysuccinimide ester (EMCS), m-maleimidobenzoyl-N-hydroxysuccinimide ester (MBS), N-(α-maleimidoacetoxy)-succinimide ester [AMAS], succinimidyl-6-(β-maleimidopropionamido)hexanoate (SMPH), N-succinimidyl 4-(p-maleimidophenyl)-butyrate (SMPB), N-(p-maleimidophenyl)isocyanate (PMPI), 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), maleimidocaproyl-L-valine-L-citrulline-p-aminobenzyl alcohol p-nitrophenylcarbonate (MC-val-cit-PAB), N-succinimidyl-4-(iodoacetyl)-aminobenzoate (SIAB), N-succinimidyl iodoacetate (SIA), N-succinimidyl bromoacetate (SBA), N-succinimidyl 3-(bromoacetamido)propionate (SBAP), N-succinimidyl-5-acetylthioacetate (SATA), N-succinimidyl-3-(2-pyridyldithio)propionate (SPDP), N-succinimidyl-3-(2-pyridyldithio)butyrate) (SPDB), and N-succinimidyl-oxycarbonyl-alpha-methyl-alpha-(2-pyridyl-dithio)toluene) (SMPT).

The binding molecule conjugate is based on a binding molecule that comprises an SLC polypeptide having at least one agent linker to an amino acid residue of the SLC polypeptide at a predetermined amino acid position. The amino acid residue is (i) normally present in the SLC polypeptide at said amino acid position, and/or (ii) introduced into the amino acid sequence of said SLC polypeptide at said amino acid position, wherein the agent is a therapeutic agent and is selected from the group consisting of a maytansinoid, a monomethyl auristatin E (MMAE) and a monomethyl auristatin F (MMAF). In one other embodiment, the amino acid residue is located (i) at position 16 and/or 21 of a mature VpreB1 sequence; and/or (ii) at one or more positions selected from the group consisting of 60, 74, 78, 79, 85, 91, 110, 123, 131, 133, 166, and 170 of a mature λ5 sequence.

In another aspect, the present invention provides a Surrobody comprising a surrogate light chain (SLC) polypeptide having a particular amino acid sequence. In one embodiment, the SLC comprises at least one amino acid sequence selected from the group consisting of: SSALG**C**TIRLT (SEQ ID NO: 37), TTIRL**C**CTLRN (SEQ ID NO: 38), SSVTH**C**FGSGT (SEQ ID NO: 39), VLSQP**C**ATPSV (SEQ ID NO: 40), PKATP**C**VTLFP (SEQ ID NO: 41), KATPS**C**TLFPP (SEQ ID NO: 42), TLFPP**C**SEELQ (SEQ ID NO: 43), SEELQ**C**NKATL (SEQ ID NO: 44), PGILT**C**TWKAD (SEQ ID NO: 45), PITQG**C**EMTTP (SEQ ID NO: 46), TTPSK**C**SNNKY (SEQ ID NO: 47), PSKQS**C**NKYAA (SEQ ID NO: 48), HEGST**C**EKTVA (SEQ ID NO: 49), and TCEKT**C**APAEC (SEQ ID NO: 50).

The Surrobody comprises a surrogate light chain (SLC) having at least one amino acid residue at a predetermined amino acid position for conjugation to an agent. In one embodiment, the amino acid residue is located (i) at position 16 and/or 21 of a mature VpreB1 sequence; and/or (ii) at one or more positions selected from the group consisting of 60, 74, 78, 79, 85, 91, 110, 123, 131, 133, 166, and 170 of a mature λ5 sequence. In another embodiment, the amino acid residue is introduced into the amino acid sequence of the SLC polypeptide. In one other embodiment, the amino acid residue is introduced into the amino acid sequence of the SLC polypeptide by a substitution. In one embodiment, the introduced amino acid is a cysteine or a lysine.
The invention is defined by the claims.

### Brief Description of the Drawings

The file of this patent contains at least one drawing executed in color. Copies of this patent with color drawing(s) will be provided by the Patent and Trademark Office upon request and payment of the necessary fees.
Figure 1 depicts a Surrobody™ as a Platform for Drug Conjugation.
Figure 2 depicts Protein Conjugates: Surrobody Can Target Multiple Types and Sizes of Proteins to Specific Sites.
Figure 3 depicts Site Directed Optimization of Invariant 2 Piece SLC for Drug Conjugation.
Figure 4 shows the human VpreB1 amino acid sequence of SEQ ID NO: 1 with a native leader sequence; the mouse VpreB2 sequences of SEQ ID NOS: 2 and 3; the human VpreB3-like sequence of SEQ ID NO: 4, the sequence of the truncated VpreB1 sequence in the "trimer" designated as "VpreB dTail" (SEQ ID NO: 5); and the human VpreB1 amino acid sequence of SEQ ID NO:6 with a murine Ig κ leader sequence. Underlining indicates the leader sequences within the VpreB amino acid sequences.
Figure 5 shows the murine λ5 sequence of SEQ ID NO: 7; the human λ5 sequence of SEQ ID NO: 8; the sequence of the truncated λ5 sequence in the "trimer" designated in Figure 11 as "λ5 dTail" (SEQ ID NO: 9); and the human λ5 dTail sequence of SEQ ID NO: 10 with a murine Ig κ leader sequence. Underlining indicates the leader sequences within the λ5 amino acid sequences.
Figure 6 shows human VpreB1-λ5 chimeric amino acid sequences (SEQ ID NOS:35 and 36) with a murine Ig κ leader sequence underlined.

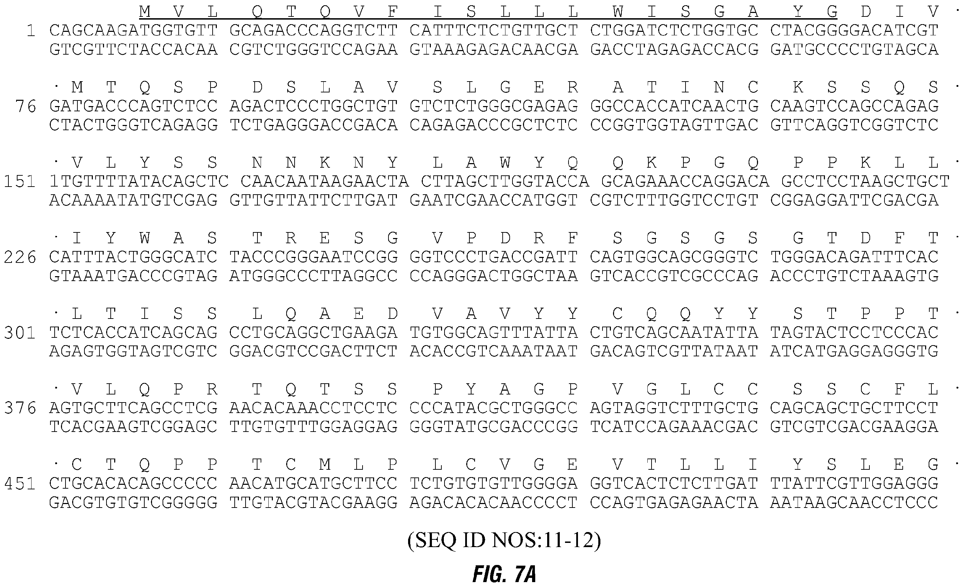

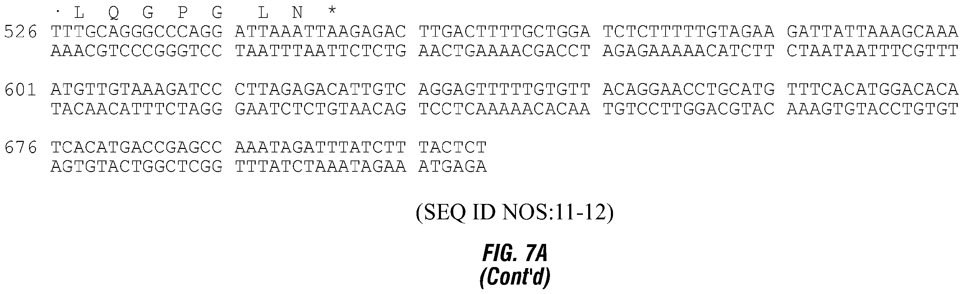

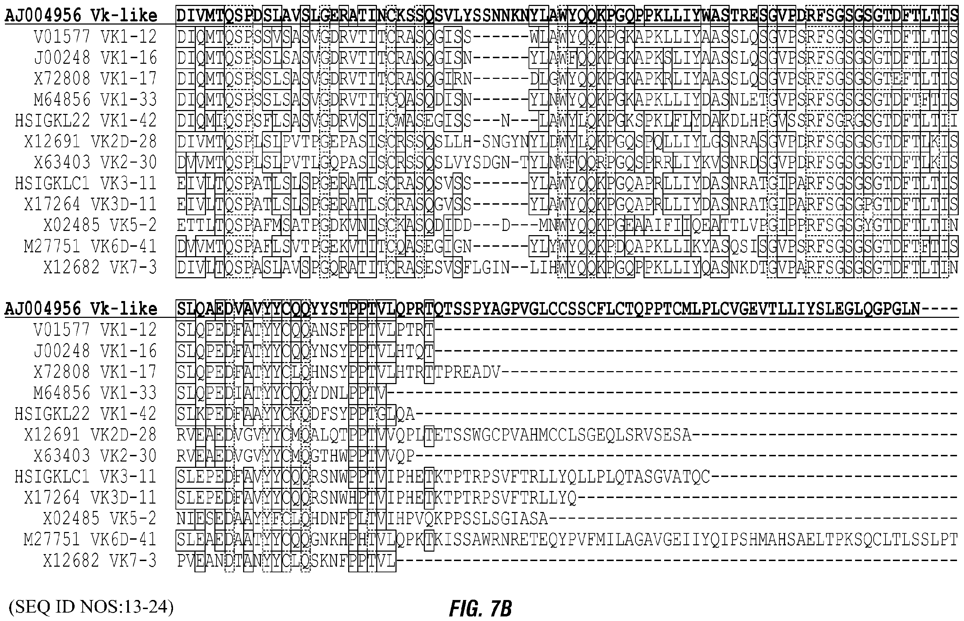

Figures 7A and 7B show (A) the human Vκ-like nucleotide sequence of SEQ ID NO:11 and the amino acid sequence of the encoded protein (AJ004956; SEQ ID NO:12) (native leader sequence underlined), and (B) the predicted mature amino acid sequences of Vκ-like proteins possible from all Vκ families, each bearing different lengths of extensions (SEQ ID NOS: 13-24) aligned with AJ004956 Vκ-like prototype sequence (residues 21-180 of SEQ ID NO:12).

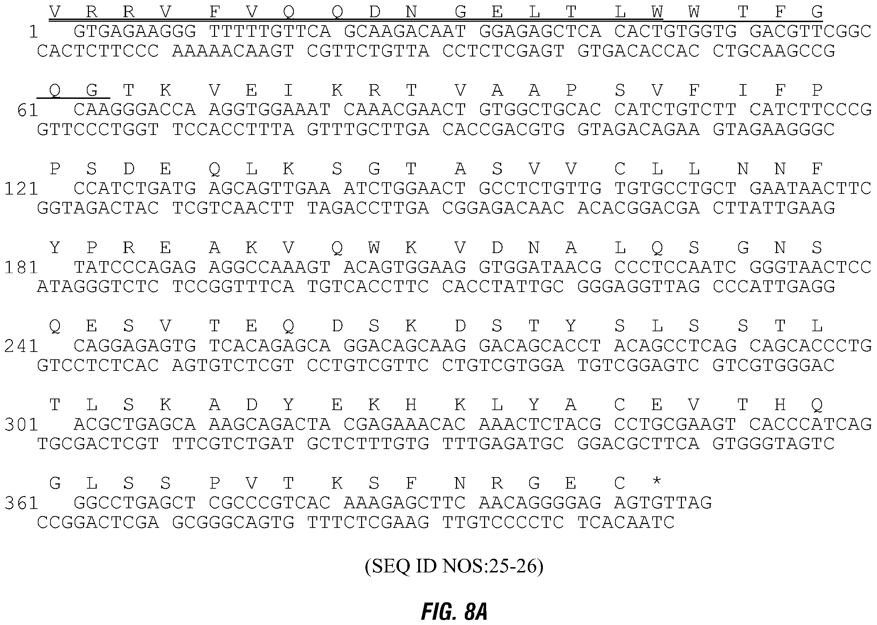

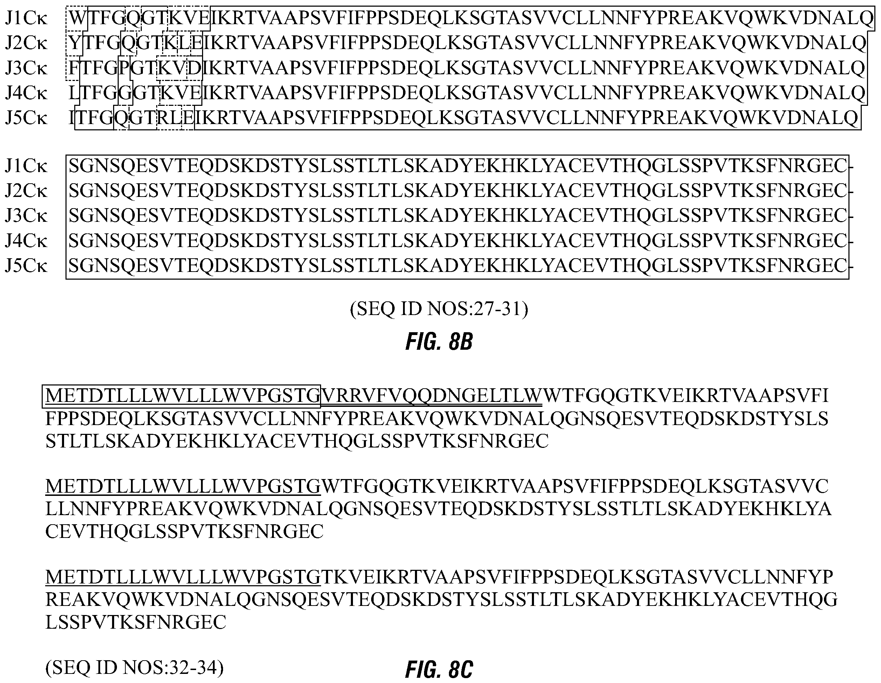

Figures 8A-C show (A) the human JCκ nucleotide sequence of SEQ ID NO:25 and the amino acid sequence of the encoded protein (SEQ ID NO:26) (unique sequence compared to predicted mature JCk proteins is doubly underlined and potential leader cleavage sequence singly underlined), (B) the predicted JCκ-like amino acid sequences from the remaining kappa J-constant region rearrangements (J1-J5Cκ) (SEQ ID NOS:27-31), and (C) the JCk engineered secretion optimized variants, including JCκ with an appended murine Ig κ leader sequence underlined (SEQ ID NO:32), a recombined JCκ only with an appended murine Ig κ leader sequence underlined (SEQ ID NO:33), and a predicted processed JCκ with an appended murine Ig κ leader sequence underlined (SEQ ID NO:34).
Figure 9 shows the results of an analysis of inhibition of cellular proliferation (*in vitro*) by Surrobody-drug conjugates.
Figure 10 shows the % conjugation for a number of Surrobody-drug conjugates (bispecific and monospecific).
Figure 11A-B depicts (A) a number of Surrobody-drug conjugate constructs (bivalent, bispecific, and monovalent), and (B) the results of an analysis of the conjugates for potency and for the ability to inhibit cell proliferation of cell lines with high receptor expression. Figure 11A (lower panel) depicts two monovalent Surrobody constructs (R1 and R2), wherein part of the molecule is functionally null.
Figure 12A-B shows the results of a serum stability analysis of (A) Surrobody-drug conjugates, and (B) Herceptin™ conjugates.
Figure 13A-D shows the results of an aggregation analysis following concentration for (A-C) Surrobody constructs, and (B) Herceptin™.
Figure 14A-C shows various positions in a surrogate light chain amino acid sequences that are suitable for introduction of an amino acid (e.g., substitution). A: VpreB1 sequence; B: λ5 sequence; and C: VpreB1-λ5 sequence. (SEQ ID NOS: 54-56, respectively).
Figure 15A-B shows various surrogate light chain variants. A: SEQ ID NOS: 57-66, respectively, in order of appearance; B: SEQ ID NOS: 67-70, respectively, in order of appearance.

### Detailed Description of the Invention

The present applications discloses methods for identifying, producing, and engineering binding molecule conjugates, and to the conjugates produced. In particular, the application discloses Surrobody conjugates composed of an antibody heavy chain variable domain and a surrogate light chain, wherein the the surrogate light chain and/or the heavy chain variable domain is conjugated to a therapeutic or diagnostic agent. The invention concerns a binding molecule conjugate comprising a surrogate light chain (SLC) polypeptide having at least one agent linked to the SLC polypeptide. The invention also discloses the polypeptide chains, nucleic acids, recombinant expression vectors, host cells, and methods for making such binding molecule conjugates. Also disclosed are pharmaceutical compositions containing the molecules and therapeutic or diagnostic methods using the same.

In one aspect, the present application also discloses a library or collection of polypeptides or peptides (*e.g.*, and antibody heavy chain variable domain), sharing common structural elements (*e.g.*, one or more framework regions) that can be manipulated to provide additional sites for conjugation (*e.g*., a cysteine or lysine) to serve as scaffolds for added functionality across the entire subset of the collection sharing said structural elements. The library or collection may be an antibody or Surrobody library or collection. In one embodiment, the common structural elements suitable for manipulation may be in one or more of the framework regions of an antibody or Surrobody.

In another aspect, the present application discloses Surrobody conjugates where certain polypeptides are conjugated to an agent while other polypeptides are not conjugated to an agent. In one embodiment, the Surrobody conjugate comprises an antibody heavy chain variable domain that is not conjugated to an agent and a surrogate light chain that is conjugated to an agent. In one other embodiment, the antibody heavy chain variable domain is conjugated to an agent in one or more of the framework regions (FR1-FR4). Those of ordinary skill in the art will appreciate that there are multiple additional areas for site specific optimization over monoclonal antibodies. Optimization will have likely provide broad application for whole classes of molecules due to conserved nature of surrogate light chains and restricted number of frameworks incorporated into a synthetic Surrobody library collection. For example, the following technologies may be applied: drug conjugate (highly toxic small molecule); radioisotopes (chelated or pretargeted); targeted drug activation (e.g., ADEPT), and immunoliposomes or Immuno targeted nanoparticles (targeted). Those of ordinary skill in the art will appreciate other suitable agents for conjugation according to the present invention.

In one aspect, the present invention provides binding molecule conjugates. In one embodiment, the binding molecule is an antibody or a Surrobody™. In anotheraspect described herein, the binding molecule conjugate comprises an antibody heavy chain variable domain, wherein binding molecule is conjugated to an agent. In one embodiment, the binding molecule further comprises a surrogate light chain (SLC). In one aspect described herein, the heavy chain variable domain is conjugated to the agent. In another embodiment, the SLC is conjugated to the agent. In some embodiments, the binding molecule is conjugated to a therapeutic agent or to a diagnostic agent. In one other embodiment, the binding molecule is conjugated to the agent via a linker. In another embodiment, at least one amino acid residue of the binding molecule is conjugated to the agent. In some embodiments, the amino acid residue comprises a naturally occurring amino acid or a non-naturally occurring amino acid. In one other embodiment, the therapeutic agent is an anti-cancer agent. In another embodiment, the diagnostic agent comprises a detectable label.

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), provides one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

Throughout this application, the use of singular includes the plural unless expressly stated otherwise.

In this application, the use of "or" includes "and/or", unless expressly stated otherwise.

Furthermore, the terms, "include," "including," and "included," are not limiting.

In the context of the present invention, the term "antibody" (Ab) is used to refer to a native antibody from a classically recombined heavy chain derived from V(D)J gene recombination and a classically recombined light chain also derived from VJ gene recombination, or a fragment thereof.

The term "binding molecule conjugate" in the broadest sense, is used to refer to a binding molecule comprising a light chain sequence. In another embodiment, the light chain sequence is an antibody λ or κ light chain sequence. In one embodiment, the light chain sequence is a surrogate light chain sequence. In one other embodiment, the surrogate light chain sequence comprises a VpreB sequence and/or a λ5 sequence. In yet another embodiment, the surrogate light chain sequence comprises a VpreB sequence fused to a λ5 sequence. In another embodiment, the surrogate light chain sequence is a κ-like surrogate light chain (SLC) construct comprising a Vκ-like and/or a JCK sequence. In one embodiment, the binding molecule may be a Surrobody in which case the light chain sequence is a surrogate light chain. The binding molecule may be an antibody in which the light chain sequence is a light chain variable domain.

The term "surrogate light chain polypeptide" or "SLC polypeptide" is used herein to refer to a VpreB polypeptide, a λ5 polypeptide, a Vκ-like polypeptide, a JCK polypeptide, and variants thereof.

The term "surrogate light chain sequence" or "SLC sequence" is used herein to refer to amino acid sequences from a native-sequence or variant VpreB polypeptide, a λ5 polypeptide, a Vκ-like polypeptide, and/or a JCK polypeptide. SLC sequences specifically include amino acid sequences from isoforms, including splice variants and variants formed by posttranslational modifications, other mammalian homologues thereof, as well as variants of one or more of such native sequence polypeptides.

The term "VpreB" is used herein in the broadest sense and refers to any native sequence or variant VpreB polypeptide, specifically including, without limitation, human VpreB 1 of SEQ ID NO: 1, mouse VpreB2 of SEQ ID NOS: 2 and 3, human VpreB3-like sequence of SEQ ID NO: 4, human VpreB dT of SEQ ID NO:5 and isoforms, including splice variants and variants formed by posttranslational modifications, other mammalian homologues thereof, as well as variants of such native sequence polypeptides.

The term "λ5" is used herein in the broadest sense and refers to any native sequence or variant λ5 polypeptide, specifically including, without limitation, murine λ5 sequence of SEQ ID NO: 7, human λ5 sequence of SEQ ID NO: 8, and the human λ5 dT shown as SEQ ID NO: 9, the human VpreB 1 amino acid sequence of SEQ ID NO: 10 and their isoforms, including splice variants and variants formed by posttranslational modifications, other mammalian homologous thereof, as well a variants of such native sequence polypeptides.

The terms "variant VpreB polypeptide" and "a variant of a VpreB polypeptide" are used interchangeably, and are defined herein as a polypeptide differing from a native sequence VpreB polypeptide at one or more amino acid positions as a result of an amino acid modification. The "variant VpreB polypeptide," as defined herein, will be different from a native antibody λ or κ light chain sequence, or a fragment thereof. The "variant VpreB polypeptide" will preferably retain at least about 65%, or at least about 70%, or at least about 75%, or at least about 80%, or at least about 85%, or at least about 90%, or at least about 95%, or at least about 98% sequence identity with a native sequence VpreB polypeptide. In another preferred embodiment, the "variant VpreB polypeptide" will be less than 95%, or less than 90%, or less than 85%, or less than 80%, or less than 75%, or less than 70%, or less than 65%, or less than 60% identical in its amino acid sequence to a native antibody λ or κ light chain sequence. Variant VpreB polypeptides specifically include, without limitation, VpreB polypeptides in which the non-Ig-like unique tail at the C-terminus of the VpreB sequence is partially or completely removed.

The terms "variant λ5 polypeptide" and "a variant of a λ5 polypeptide" are used interchangeably, and are defined herein as a polypeptide differing from a native sequence λ5 polypeptide at one or more amino acid positions as a result of an amino acid modification. The "variant λ5 polypeptide," as defined herein, will be different from a native antibody λ or κ light chain sequence, or a fragment thereof. The "variant λ5 polypeptide" will preferably retain at least about 65%, or at least about 70%, or at least about 75%, or at least about 80%, or at least about 85%, or at least about 90%, or at least about 95%, or at least about 98% sequence identity with a native sequence λ5 polypeptide. In another preferred embodiment, the "variant λ5 polypeptide" will be less than 95%, or less than 90%, or less than 85%, or less than 80%, or less than 75%, or less than 70%, or less than 65%, or less than 60% identical in its amino acid sequence to a native antibody λ or κ light chain sequence. Variant λ5 polypeptides specifically include, without limitation, λ5 polypeptides in which the unique tail at the N-terminus of the λ5 sequence is partially or completely removed.

The terms "variant Vκ-like polypeptide" and "a variant of a Vκ-like polypeptide" are used interchangeably, and are defined herein as a polypeptide differing from a native sequence Vκ-like polypeptide at one or more amino acid positions as a result of an amino acid modification. The "variant Vκ-like polypeptide," as defined herein, will be different from a native antibody λ or κ light chain sequence, or a fragment thereof. The "variant Vκ-like polypeptide" will preferably retain at least about 65%, or at least about 70%, or at least about 75%, or at least about 80%, or at least about 85%, or at least about 90%, or at least about 95%, or at least about 98% sequence identity with a native sequence Vκ-like polypeptide. In another preferred embodiment, the "variant Vκ-like polypeptide" will be less than 95%, or less than 90%, or less than 85%, or less than 80%, or less than 75%, or less than 70%, or less than 65%, or less than 60% identical in its amino acid sequence to a native antibody λ or κ light chain sequence. Variant Vκ-like polypeptides specifically include, without limitation, Vκ-like polypeptides in which the non-Ig-like unique tail at the C-terminus of the Vκ-like sequence is partially or completely removed.

The terms "variant JCK polypeptide" and "a variant of a JCK polypeptide" are used interchangeably, and are defined herein as a polypeptide differing from a native sequence JCK polypeptide at one or more amino acid positions as a result of an amino acid modification. The "variant JCK polypeptide," as defined herein, will be different from a native antibody λ or κ light chain sequence, or a fragment thereof. The "variant JCK polypeptide" will preferably retain at least about 65%, or at least about 70%, or at least about 75%, or at least about 80%, or at least about 85%, or at least about 90%, or at least about 95%, or at least about 98% sequence identity with a native sequence JCK polypeptide. In another preferred embodiment, the "variant JCK polypeptide" will be less than 95%, or less than 90%, or less than 85%, or less than 80%, or less than 75%, or less than 70%, or less than 65%, or less than 60% identical in its amino acid sequence to a native antibody λ or κ light chain sequence. Variant JCK polypeptides specifically include, without limitation, JCK polypeptides in which the unique tail at the N-terminus of the JCK sequence is partially or completely removed.

Percent amino acid sequence identity may be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

The term "VpreB sequence" is used herein to refer to the sequence of "VpreB," as hereinabove defined, or a fragment thereof.

The term "λ5 sequence" is used herein to refers to the sequence of "λ5," as hereinabove defined, or a fragment thereof.

The term "Vκ-like sequence" is used herein to refer to the sequence of "Vκ-like," as hereinabove defined, or a fragment thereof.

The term "JCκ sequence" is used herein to refer to the sequence of "JCκ," as hereinabove defined, or a fragment thereof.

The term "λ-like surrogate light chain," as used herein, refers to a dimer formed by the non-covalent association of a VpreB and a λ5 protein.

The term "κ-like surrogate light chain," as used herein, refers to a dimer formed by the non-covalent association of a Vκ-like and a JCκ protein.

The term "λ-like surrogate light chain sequence," as defined herein, means any polypeptide sequence that comprises a "VpreB sequence" and/or a "λ5 sequence," as hereinabove defined. The "λ-like surrogate light chain sequence," as defined herein, specifically includes, without limitation, the human VpreB 1 sequence of SEQ ID NO 1, the mouse VpreB2 sequences of SEQ ID NOS: 2 and 3, and the human VpreB3 sequence of SEQ ID NO: 4, the human VpreB dT shown as SEQ ID NO: 5; and the human VpreB 1 amino acid sequence of SEQ ID NO:6 and their various isoforms, including splice variants and variants formed by post-translational modifications, homologues thereof in other mammalian species, as well as fragments and variants thereof. The term "λ-like surrogate light chain sequence" additionally includes, without limitation, the murine λ5 sequence of SEQ ID NO: 7, the human λ5 sequence of SEQ ID NO: 8, the human λ5 dTail shown as SEQ ID NO: 9, the human λ5 dTail sequence of SEQ D NO: 10 and their isoforms, including splice variants and variants formed by posttranslational modifications, homologues thereof in other mammalian species, as well as fragments and variants thereof. The term "λ-like surrogate light chain sequence" additionally includes a sequence comprising both VpreB and λ5 sequences as hereinabove defined.

The term "κ-like surrogate light chain sequence," as defined herein, means any polypeptide sequence that comprises a "Vκ-like sequence" and/or a "JCκ," as hereinabove defined. The "κ-like surrogate light chain sequence," as defined herein, specifically includes, without limitation, the human Vκ-like sequence of any of SEQ ID NOS: 12-24, and their various isoforms, including splice variants and variants formed by posttranslational modifications, homologues thereof in other mammalian species, as well as fragments and variants thereof. The term "κ-like surrogate light chain sequence" additionally includes, without limitation, the human Vκ-like sequence of any of SEQ ID NOS: 12-24, the human JCκ sequence of any of SEQ ID NO:25-35, and their isoforms, including splice variants and variants formed by posttranslational modifications, homologues thereof in other mammalian species, as well as fragments and variants thereof. The term "κ-like surrogate light chain sequence" additionally includes a sequence comprising both Vκ-like and JCκ sequences as hereinabove defined.

The term "surrogate light chain construct" is used in the broadest sense and includes any and all additional heterologous components, including a heterologous amino acid sequence, nucleic acid, and other molecules conjugated to a surrogate light chain sequence, wherein "conjugation" is defined below.

A "surrogate light chain construct" is also referred herein as a "Surrobody™," or "Surrobody" and the two terms are used interchangeably. Certain Surrobody™ λ-like surrogate light chain constructs are disclosed in Xu et al., Proc. Natl. Acad. Sci. USA 2008, 105(31):10756-61 and in PCT Publication WO 2008/118970 published on October 2, 2008. Also contemplated are κ-like surrogate light chain constructs as described in U.S. Patent Publication No. 2010-0062950, and Xu et al., J. Mol. Biol. 2010, 397, 352-360. In addition, Surrobodies comprising Stacked Variable Domains as described in PCT/US2012/044746 filed on June 28, 2012.

In the context of the polypeptides of the present invention, the term "heterologous amino acid sequence," relative to a first amino acid sequence, is used to refer to an amino acid sequence not naturally associated with the first amino acid sequence, at least not in the form it is present in the surrogate light chain constructs herein. Thus, a "heterologous amino acid sequence" relative to a VpreB, λ5, Vκ-like, or JCκ is any amino acid sequence not associated with native VpreB, λ5, Vκ-like, or JCκ in its native environment. These include, without limitation, i) λ5 sequences that are different from those λ5 sequences that, together with VpreB, form the surrogate light chain on developing B cells, such as amino acid sequence variants, e.g. truncated and/or derivatized λ5 sequences; ii) VpreB sequences that are different from those VpreB sequences that, together with λ5, form the surrogate light chain on developing B cells, such as amino acid sequence variants, e.g. truncated and/or derivatized VpreB sequences, iii) Vκ-like sequences that are different from those Vκ-like sequences that, together with JCκ, form the κ-like surrogate light chain on developing B cells, such as amino acid sequence variants, e.g. truncated and/or derivatized Vκ-like sequences; and iv) JCκ sequences that are different from those JCκ sequences that, together with Vκ-like, form the κ-like surrogate light chain on developing B cells, such as amino acid sequence variants, e.g. truncated and/or derivatized JCκ sequences.

A "heterologous amino acid sequence" relative to a VpreB or λ5 also includes VpreB or λ5 sequences covalently associated with, e.g. fused to, a corresponding VpreB or λ5, including native sequence VpreB or λ5, since in their native environment, the VpreB and λ5 sequences are not covalently associated, e.g. fused, to each other. Similarly, a "heterologous amino acid sequence" relative to a Vκ-like or JCκ also includes Vκ-like or JCκ sequences covalently associated with, e.g. fused to, a corresponding Vκ-like or JCκ, including native sequence Vκ-like or JCκ, since in their native environment, the Vκ-like or JCκ sequences are not covalently associated, e.g. fused, to each other.

A "heterologous amino acid sequence" relative to a VpreB or Vκ-like also includes VpreB or Vκ-like sequences covalently associated with, e.g. fused to, a sequence providing additional functionality (e.g., a cytokine or antibody fragment amino acid sequence), or any fragment or variant thereof, since in their native environment, the VpreB or Vκ-like and the sequence providing additional functionality are not covalently associated, e.g. fused, to each other. The antibody fragment amino acid sequence may be a single chain variable fragment (scFv).

Heterologous amino acid sequences also include, without limitation, antibody sequences, including antibody and heavy chain sequences and fragments or variants thereof, such as, for example, antibody light and heavy chain variable region sequences, and antibody light and heavy chain constant region sequences.

The terms "conjugate," "conjugated," and "conjugation" refer to any and all forms of covalent or non-covalent linkage, and include, without limitation, direct genetic or chemical fusion, coupling through a linker or a cross-linking agent, and non-covalent association, for example through Van der Waals forces, or by using a leucine zipper. In the present invention, conjugation particuarly refers to the linkage of a therapeutic or diagnostic agent to a polypeptide that is part of a binding molecule, such as a Surrobody or antibody.

The term "flexible linker" is used herein to refer to any linker that is not predicted, based on its chemical structure, to be fixed in three-dimensional space in its intended context and environment.

The term "fusion" is used herein to refer to the combination of amino acid sequences of different origin in one polypeptide chain by in-frame combination of their coding nucleotide sequences. The term "fusion" explicitly encompasses internal fusions, i.e., insertion of sequences of different origin within a polypeptide chain, in addition to fusion to one of its termini.

As used herein, the terms "peptide," "polypeptide" and "protein" all refer to a primary sequence of amino acids that are joined by covalent "peptide linkages." In general, a peptide consists of a few amino acids, typically from about 2 to about 50 amino acids, and is shorter than a protein. The term "polypeptide," as defined herein, encompasses peptides and proteins.

A "native antibody" is heterotetrameric glycoprotein of about 150,000 daltons, composed of two identical light (L) chains and two identical heavy (H) chains. Each light chain is linked to a heavy chain by covalent disulfide bond(s), while the number of disulfide linkages varies between the heavy chains of different immunoglobulin isotypes. Each heavy and light chain also has regularly spaced intrachain disulfide bridges. Each heavy chain has, at one end, a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light- and heavy-chain variable domains, Chothia et al., J. Mol. Biol. 186:651 (1985); Novotny and Haber, Proc. Natl. Acad. Sci. U.S.A. 82:4592 (1985).

The term "variable" with reference to antibody chains is used to refer to portions of the antibody chains which differ extensively in sequence among antibodies and participate in the binding and specificity of each particular antibody for its particular antigen. Such variability is concentrated in three segments called hypervariable regions both in the light chain and the heavy chain variable domains. The more highly conserved portions of variable domains are called the framework region (FR). The variable domains of native heavy and light chains each comprise four FRs (FR1, FR2, FR3 and FR4, respectively), largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991), pages 647-669). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" (*i.e.,* residues 30-36 (L1), 46-55 (L2) and 86-96 (L3) in the light chain variable domain and 30-35 (H1), 47-58 (H2) and 93-101 (H3) in the heavy chain variable domain; MacCallum et al,. J Mol Biol. 262(5):732-45 (1996).

The term "framework region" refers to the art recognized portions of an antibody variable region that exist between the more divergent CDR regions. Such framework regions are typically referred to as frameworks 1 through 4 (FR1, FR2, FR3, and FR4) and provide a scaffold for holding, in three-dimensional space, the three CDRs found in a heavy or light chain antibody variable region, such that the CDRs can form an antigen-binding surface.

Depending on the amino acid sequence of the constant domain of their heavy chains, antibodies can be assigned to different classes. There are five major classes of antibodies IgA, IgD, IgE, IgG, and IgM, and several of these may be further divided into subclasses (isotypes), e.g., IgG1, IgG2, IgG3, IgG4, IgA, and IgA2. In a preferred embodiment, the immunoglobulin sequences used in the construction of the immunoadhesins of the described herein are from an IgG immunoglobulin heavy chain domain. For human immunoadhesins, the use of human IgG1 and IgG3 immunoglobulin sequences is preferred. A major advantage of using the IgG1 is that IgG1 immunoadhesins can be purified efficiently on immobilized protein A. However, other structural and functional properties should be taken into account when choosing the Ig fusion partner for a particular immunoadhesin construction. For example, the IgG3 hinge is longer and more flexible, so that it can accommodate larger "adhesin" domains that may not fold or function properly when fused to IgG1. Another consideration may be valency; IgG immunoadhesins are bivalent homodimers, whereas Ig subtypes like IgA and IgM may give rise to dimeric or pentameric structures, respectively, of the basic Ig homodimer unit. For VEGF receptor Ig-like domain/immunoglobulin chimeras designed for in vivo applications, the pharmacokinetic properties and the effector functions specified by the Fc region are important as well. Although IgG1, IgG2 and IgG4 all have in vivo half-lives of 21 days, their relative potencies at activating the complement system are different. Moreover, various immunoglobulins possess varying numbers of allotypic isotypes.

The heavy-chain constant domains that correspond to the different classes of immunoglobulins are called α, δ, ε, γ, and µ, respectively.

The "light chains" of antibodies from any vertebrate species can be assigned to one of two clearly distinct types, called kappa (κ) and lambda (λ), based on the amino acid sequences of their constant domains. Any reference to an antibody light chain herein includes both κ and λ light chains.

"Antibody fragments" comprise a portion of a full length antibody, generally the antigen binding or a variable domain thereof. Examples of antibody fragments include, but are not limited to, Fab, Fab', F(ab')₂, scFv, and (scFv)₂ fragments.

As used herein the term "antibody binding region" refers to one or more portions of an immunoglobulin or antibody variable region capable of binding an antigen(s). Typically, the antibody binding region is, for example, an antibody light chain (VL) (or variable region thereof), an antibody heavy chain (VH) (or variable region thereof), a heavy chain Fd region, a combined antibody light and heavy chain (or variable region thereof) such as a Fab, F(ab')₂, single domain, or single chain antibody (scFv), or a full length antibody, for example, an IgG (*e.g.*, an IgG1, IgG2, IgG3, or IgG4 subtype), IgA1, IgA2, IgD, IgE, or IgM antibody.

The term "epitope" as used herein, refers to a sequence of at least about 3 to 5, preferably at least about 5 to 10, or at least about 5 to 15 amino acids, and typically not more than about 500, or about 1,000 amino acids, which define a sequence that by itself, or as part of a larger sequence, binds to an antibody generated in response to such sequence. An epitope is not limited to a polypeptide having a sequence identical to the portion of the parent protein from which it is derived. Indeed, viral genomes are in a state of constant change and exhibit relatively high degrees of variability between isolates. Thus the term "epitope" encompasses sequences identical to the native sequence, as well as modifications, such as deletions, substitutions and/or insertions to the native sequence. Generally, such modifications are conservative in nature but non-conservative modifications are also contemplated. The term specifically includes "mimotopes," i.e. sequences that do not identify a continuous linear native sequence or do not necessarily occur in a native protein, but functionally mimic an epitope on a native protein. The term "epitope" specifically includes linear and conformational epitopes.

The term "amino acid" or "amino acid residue" typically refers to an amino acid having its art recognized definition such as an amino acid selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val) although modified, synthetic, or rare amino acids may be used as desired. Thus, modified and unusual amino acids listed in 37 CFR 1.822(b)(4) are specifically included within this definition. Amino acids can be subdivided into various sub-groups. Thus, amino acids can be grouped as having a nonpolar side chain (*e.g.*, Ala, Cys, Ile, Leu, Met, Phe, Pro, Val); a negatively charged side chain (*e.g.*, Asp, Glu); a positively charged side chain (*e.g*., Arg, His, Lys); or an uncharged polar side chain (*e.g.*, Asn, Cys, Gln, Gly, His, Met, Phe, Ser, Thr, Trp, and Tyr). Amino acids can also be grouped as small amino acids (Gly, Ala), nucleophilic amino acids (Ser, His, Thr, Cys), hydrophobic amino acids (Val, Leu, Ile, Met, Pro), aromatic amino acids (Phe, Tyr, Trp, Asp, Glu), amides (Asp, Glu), and basic amino acids (Lys, Arg).

The term "Stacked Variable Domain," or "SVD," in the broadest sense, is used to refer to tandem arrangements in which variable domain sequences from two different sources are conjugated to each other. In one embodiment, the conjugation takes place by direct fusion. In another embodiment, the conjugation is provided by covalent linkage through a linker sequence, such as, for example, a short peptide sequence. The reference to two different sources does not mean, however, that the variable domain sequence have to be obtained from the source from which they derive. The variable domain sequences and the tandem arrangements can be produced by any means, such as recombinant methods and/or chemical synthesis. The terms "Stacked Variable Domain" or "SVD" specifically include multi-specific (e.g. bispecific, trispecific, etc.) Surrobody- or antibody-based polypeptides comprising at least one "outer binding domain" and at least one "inner binding domain", each specifically binding to a different target. The term specifically includes bispecific, trispecific, and other multi-specific constructs, where the variable domains may be present ("stacked") in a single polypeptide chain ("single-chain stacked variable domains") or two or more polypeptide chains. Thus, the terms specifically include, without limitation, monomeric, dimeric and tetrameric structures, and monovalent bispecific and bivalent bispecific structures. Stacked Variable Domains are further described in PCT/US2012/044746 filed on June 28, 2012.

The term "polynucleotide(s)" refers to nucleic acids such as DNA molecules and RNA molecules and analogs thereof (*e.g.*, DNA or RNA generated using nucleotide analogs or using nucleic acid chemistry). As desired, the polynucleotides may be made synthetically, *e.g.*, using art-recognized nucleic acid chemistry or enzymatically using, *e.g.*, a polymerase, and, if desired, be modified. Typical modifications include methylation, biotinylation, and other art-known modifications. In addition, the nucleic acid molecule can be single-stranded or double-stranded and, where desired, linked to a detectable moiety.

The term "variant" with respect to a reference polypeptide refers to a polypeptide that possesses at least one amino acid mutation or modification (i.e., alteration) as compared to a native polypeptide. Variants generated by "amino acid modifications" can be produced, for example, by substituting, deleting, inserting and/or chemically modifying at least one amino acid in the native amino acid sequence.

An "amino acid modification" refers to a change in the amino acid sequence of a predetermined amino acid sequence. Exemplary modifications include an amino acid substitution, insertion and/or deletion.

An "amino acid modification at" a specified position, refers to the substitution or deletion of the specified residue, or the insertion of at least one amino acid residue adjacent the specified residue. By insertion "adjacent" a specified residue is meant insertion within one to two residues thereof. The insertion may be N-terminal or C-terminal to the specified residue.

An "amino acid substitution" refers to the replacement of at least one existing amino acid residue in a predetermined amino acid sequence with another different "replacement" amino acid residue. The replacement residue or residues may be "naturally occurring amino acid residues" (i.e. encoded by the genetic code) and selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). Substitution with one or more non-naturally occurring amino acid residues is also encompassed by the definition of an amino acid substitution herein.

A "non-naturally occurring amino acid residue" refers to a residue, other than those naturally occurring amino acid residues listed above, which is able to covalently bind adjacent amino acid residues(s) in a polypeptide chain. Examples of non-naturally occurring amino acid residues include norleucine, ornithine, norvaline, homoserine and other amino acid residue analogues such as those described in Ellman et al. Meth. Enzym. 202:301 336 (1991). To generate such non-naturally occurring amino acid residues, the procedures of Noren et al. Science 244:182 (1989) and Ellman et al., supra, can be used. Briefly, these procedures involve chemically activating a suppressor tRNA with a non-naturally occurring amino acid residue followed by in vitro transcription and translation of the RNA.

An "amino acid insertion" refers to the incorporation of at least one amino acid into a predetermined amino acid sequence. While the insertion will usually consist of the insertion of one or two amino acid residues, the present application contemplates larger "peptide insertions", e.g. insertion of about three to about five or even up to about ten amino acid residues. The inserted residue(s) may be naturally occurring or non-naturally occurring as disclosed above.

An "amino acid deletion" refers to the removal of at least one amino acid residue from a predetermined amino acid sequence.

The term "mutagenesis" refers to, unless otherwise specified, any art recognized technique for altering a polynucleotide or polypeptide sequence. Preferred types of mutagenesis include error prone PCR mutagenesis, saturation mutagenesis, or other site directed mutagenesis.

"Site-directed mutagenesis" is a technique standard in the art, and is conducted using a synthetic oligonucleotide primer complementary to a single-stranded phage DNA to be mutagenized except for limited mismatching, representing the desired mutation. Briefly, the synthetic oligonucleotide is used as a primer to direct synthesis of a strand complementary to the single-stranded phage DNA, and the resulting double-stranded DNA is transformed into a phage-supporting host bacterium. Cultures of the transformed bacteria are plated in top agar, permitting plaque formation from single cells that harbor the phage. Theoretically, 50% of the new plaques will contain the phage having, as a single strand, the mutated form; 50% will have the original sequence. Plaques of interest are selected by hybridizing with kinased synthetic primer at a temperature that permits hybridization of an exact match, but at which the mismatches with the original strand are sufficient to prevent hybridization. Plaques that hybridize with the probe are then selected, sequenced and cultured, and the DNA is recovered.

The term "vector" is used to refer to a rDNA molecule capable of autonomous replication in a cell and to which a DNA segment, e.g., gene or polynucleotide, can be operatively linked so as to bring about replication of the attached segment. Vectors capable of directing the expression of genes encoding for one or more polypeptides are referred to herein as "expression vectors. "The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers. A vector may be a "plasmid" referring to a circular double-stranded DNA loop into which additional DNA segments may be ligated. A vector may be a phage vector or a viral vector, in which additional DNA segements may be ligated into the viral genome. Suitable vectors are capable of autonomous replication in a host cell into which they are introduced, e.g., bacterial vector with a bacterial origin or replication and episomal mammalian vectors. A vector may be integrated into the host cell genome, e.g., a non-episomal mammalian vector, upon introduction into the host cell, and replicated along with the host genome.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

A "phage display library" is a protein expression library that expresses a collection of cloned protein sequences as fusions with a phage coat protein. Thus, the phrase "phage display library" refers herein to a collection of phage (e.g., filamentous phage) wherein the phage express an external (typically heterologous) protein. The external protein is free to interact with (bind to) other moieties with which the phage are contacted. Each phage displaying an external protein is a "member" of the phage display library.

The term "filamentous phage" refers to a viral particle capable of displaying a heterogeneous polypeptide on its surface, and includes, without limitation, f1, fd, Pf1, and M13. The filamentous phage may contain a selectable marker such as tetracycline (e.g., "fd-tet"). Various filamentous phage display systems are well known to those of skill in the art (see, e.g., Zacher et al. Gene 9: 127-140 (1980), Smith et al. Science 228: 1315-1317 (1985); and Parmley and Smith Gene 73: 305-318 (1988)).

The term "panning" is used to refer to the multiple rounds of screening process in identification and isolation of phages carrying compounds, such as antibodies, with high affinity and specificity to a target.

A "leader sequence," "signal peptide," or a "secretory leader," which terms are used interchangeably, contains a sequence comprising amino acid residues that directs the intracellular trafficking of the polypeptide to which it is a part. Polypeptides contain secretory leaders, signal peptides or leader sequences, typically at their N-terminus. These polypeptides may also contain cleavage sites where the leader sequences may be cleaved from the rest of the polypeptides by signal endopeptidases. Such cleavage results in the generation of mature polypeptides. Cleavage typically takes place during secretion or after the intact polypeptide has been directed to the appropriate cellular compartment.

A "host cell" includes an individual cell or cell culture which can be or has been a recipient for transformation of nucleic acid(s) and/or vector(s) containing nucleic acids encoding the molecules described herein. In methods of the described herein, a host cell can be a eukaryotic cell, such as a Chinese Hamster Ovary (CHO) cell, or a human embryonic kidney (HEK) 293 cell. Other suitable host cells are known to those skilled in the art.

### B. Detailed Description

Techniques for performing the methods described herein are well known in the art and described in standard laboratory textbooks, including, for example, Ausubel et al., Current Protocols of Molecular Biology, John Wiley and Sons (1997); Molecular Cloning: A Laboratory Manual, Third Edition, J. Sambrook and D. W. Russell, eds., Cold Spring Harbor, New York, USA, Cold Spring Harbor Laboratory Press, 2001; O'Brian et al., Analytical Chemistry of Bacillus Thuringiensis, Hickle and Fitch, eds., Am. Chem. Soc., 1990; Bacillus thuringiensis: biology, ecology and safety, T.R. Glare and M. O'Callaghan, eds., John Wiley, 2000; Antibody Phage Display. Methods and Protocols, Humana Press, 2001; and Antibodies, G. Subramanian, ed., Kluwer Academic, 2004. Mutagenesis can, for example, be performed using site-directed mutagenesis (Kunkel et al., Proc. Natl. Acad. Sci USA 82:488-492 (1985)). PCR amplification methods are described in U.S. Pat. Nos. 4,683,192, 4,683,202, 4,800,159, and 4,965,188, and in several textbooks including "PCR Technology: Principles and Applications for DNA Amplification", H. Erlich, ed., Stockton Press, New York (1989); and PCR Protocols: A Guide to Methods and Applications, Innis et al., eds., Academic Press, San Diego, Calif. (1990).

The present invention provides binding molecules conjugated to an agent, which is a therapeutic agent. The binding molecule conjugate comprises a surrogate light chain (SLC). In one aspect, the binding molecule conjugate is based upon antibodies or Surrobodies.

### 1. Surrogate Light Chains (SLCs)

Surrobody constructs are based on the pre-B cell receptor (pre-BCR), which is produced during normal development of an antibody repertoire. Unlike antibodies, pre-BCR is a trimer, that is composed of an antibody heavy chain paired with two surrogate light chain components, VpreB and λ5. Both VpreB and λ5 are encoded by genes that do not undergo gene rearrangement and are expressed in early pro-B cells before V(D)J recombination begins. The pre-BCR is structurally different from a mature immunoglobulin in that it is composed of a heavy chain and two non-covalently associated proteins: VpreB and λ5, i.e., they have three components as opposed to two in antibodies. Furthermore, although VpreB is homologous to the Vλ Ig domain, and λ5 is homologous to the Cλ domain of antibodies, each has noncanonical peptide extensions: VpreB 1 has additional 21 residues on its C terminus; λ5 has a 50 amino acid extension at its N terminus.

Similarly, the κ-like surrogate light chain constructs described herein are based on the pre-B cell receptor (pre-BCR). The κ-like light chain is the germline VκIV gene partnered with a JCK fusion gene. In each of these genes a peptidic extension exists in the vicinity surrounding a site analogous for CDR3. As these two proteins do not appear to recombine at the genomic level it is likely their association to a heavy chain are mutually exclusive of each other and analogous to the associations described for the λ-like surrogate light chain.

Further details of the design and production of Surrobodies are provided in Xu et al., Proc. Natl. Acad. Sci. USA 2008, 105(31):10756-61 and in PCT Publications WO 2008/118970, published on October 2, 2008; WO/2010/006286, published on January 1, 2010; WO/2010/151808, published on December 29, 2010, WO/2011/071957 published on June 16, 2011; and PCT/US2012/044746 filed on June 28, 2012.

### (i) λ-like surrogate light chains

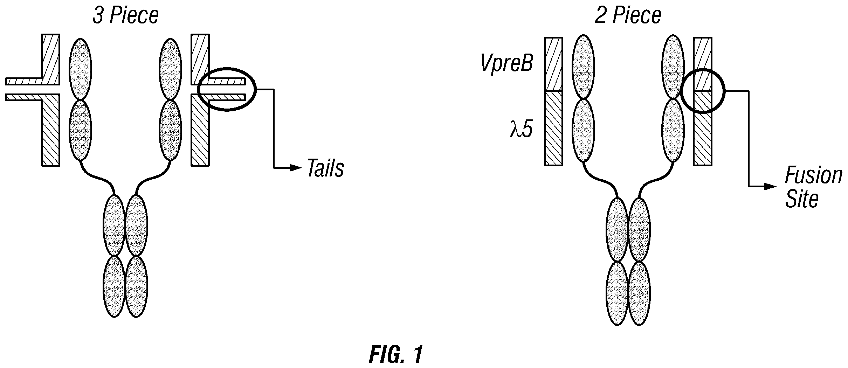

The present invention contemplates Surrobody conjugates comprising surrogate light chains that have a VpreB sequence conjugated to a λ5 sequence. Figure 1 depicts a Surrobody™ as a platform for conjugation, which takes advantage of the invariant Surrogate Light Chain (SLC) partner as a candidate site(s) for consistent conjugation. Two invariant proteins: lambda5 (λ5) and VpreB make up the Surrogate Light Chain, which can be utilized by Surrobodies™ as two native proteins with endogenous "tails" ready for conjugation ("3 piece) or with the two proteins fused together into single protein ("2 piece") (Figure 1). In addition, the Surrobody library is a synthetic library with constrained frameworks made up of 4 possible combinations. Conserved framework regions also provide candidate site(s) for consistent conjugation.

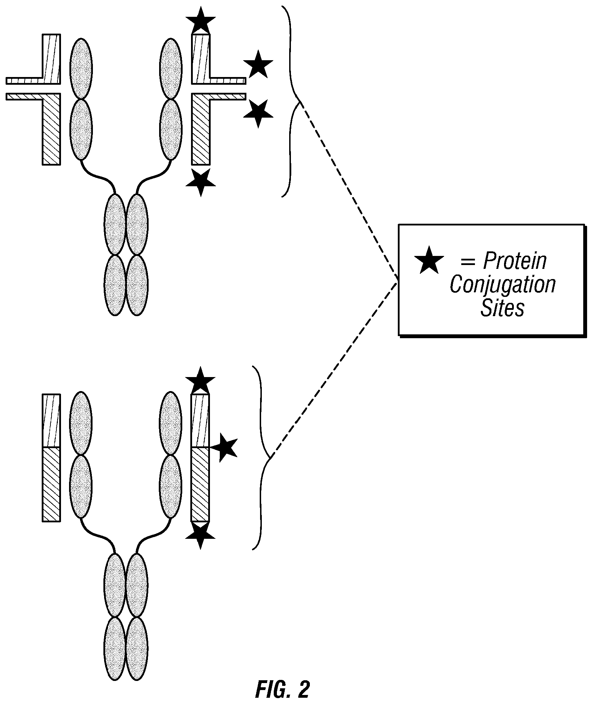

Figure 2 illustrates, in general, how a Surrobody can target multiple types and sizes of proteins to specific sites. Multiple proteins have been conjugated to various components of the SLC with high fidelity including, without limitation, immunoproteins (e.g., IL-2 at 16 kDa), bispecific immune recruitment (e.g., CD3), medium to large proteins (e.g., FGF21 at 20 kDa, scFv Abs at 25 kDa - influenza virus antibody and VEGF, and Fab constructs), and small proteins (e.g., bioactive proteins such as GLP-1 and Exendin-1 at ∼6kDa).

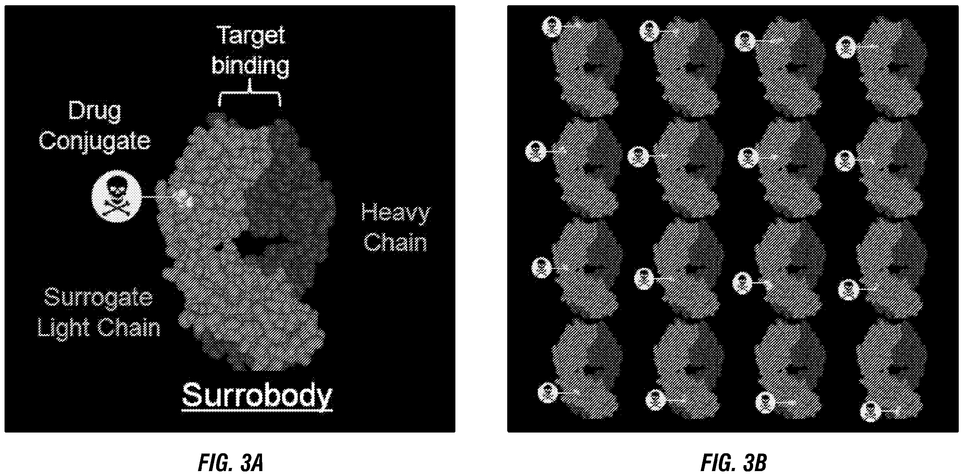

The present invention relates to additional areas for specific optimization of conjugation in Surrobodies™ components. Such optimization has the potential for a variety of applications due to the conserved nature of SLC and restricted number of frameworks derived from a synthetic collection. One important area of application is the formation of Surrobody™-drug conjugates. Figure 3A depicts an example of site directed optimization of an invariant 2-piece SLC for drug conjugation. Once favorable sites are identified on the SLC (e.g., series of possible cysteine, lysine, or other single or multiple chemical substitutions in SLC constructs), they can can be used for subsequent Surrobodies™ (Figure 3B).

In one embodiment, the VpreB sequence is selected from the group consisting of a native VpreB 1 sequence, a native VpreB2 sequence, a native VpreB3 sequence and fragments and variants thereof. In one other embodiment, the native VpreB sequence is selected from the group consisting of human VpreB1 of SEQ ID NO: 1, mouse VpreB2 of SEQ ID NOS: 2 and 3, human VpreB3 of SEQ ID NO: 4, VpreB-like polypeptide of SEQ ID NO:5, human VpreB dTail polypeptide of SEQ ID NO:6 and fragments and variants thereof. In other embodiments, the λ5 sequence comprises all or part of a murine λ5 of SEQ ID NO: 7; a human λ5 polypeptide of SEQ ID NO: 8, or a human λ5 dTail polypeptide of SEQ ID NO:9.

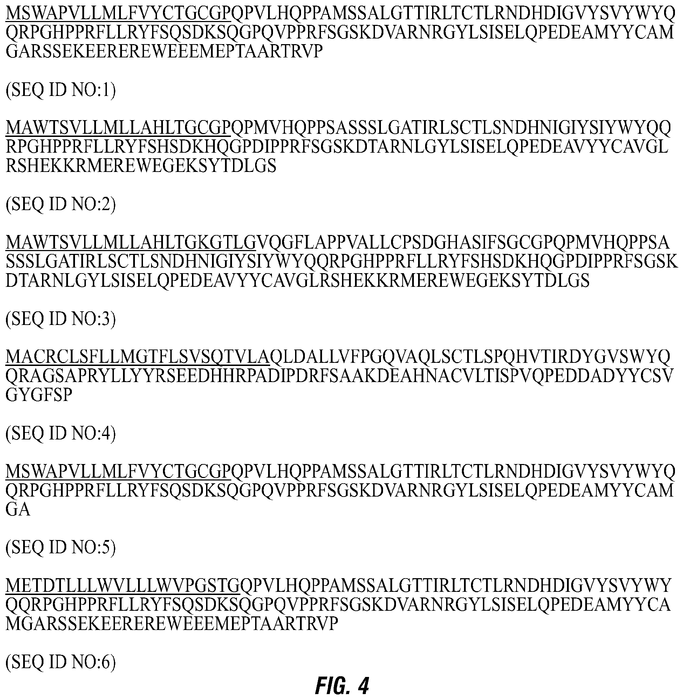

The main isoform of human VpreB 1 (CAG30495) is a 145 amino acid long polypeptide (SEQ ID NO: 1 in Figure 4), including a 19 amino acid leader sequence. Similar leader sequences are present in other VpreB polypeptides. The human truncated VpreB 1 sequence (lacking the characteristic "tail" at the C-terminus of native VpreB 1), is also referred to as the "VpreB 1 dTail sequence" and shown as SEQ ID NO:5.

The main isoform of murine λ5 (CAA10962) is a 209-amino acid polypeptide (SEQ ID NO:7), including a 30 amino acid leader sequence. A human λ5-like protein has 213 amino acids (NP_064455; SEQ ID NO: 8) and shows about 84% sequence identity to the antibody λ light chain constant region. Similar leader sequences are present in other λ5 polypeptides. The human truncated λ5 sequence (lacking the characteristic "tail" at the N-terminus of native λ5), is also referred to as the "λ5 dTail sequence" and shown as SEQ ID NO:9.

In one other embodiment, the invention provides binding molecule conjugates that contain an SLC construct comprising a VpreB sequence shown as SEQ ID NO:6. In another embodiment, the invention provides an SLC construct comprising a λ5 sequence shown as SEQ ID NO:10. In one embodiment, the SLC construct comprises a polypeptide shown as SEQ ID NO:35.

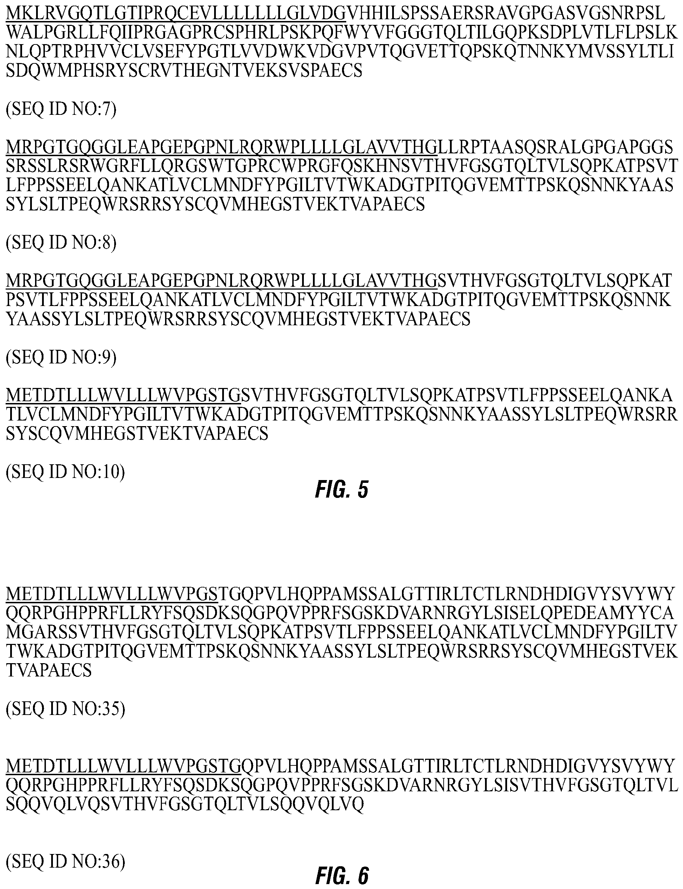

Specific examples of λ-like Surrobodies include polypeptides in which a VpreB sequence, such as a VpreB 1, VpreB2, or VpreB3 sequence, including fragments and variants of the native sequences, is conjugated to a λ5 sequence, including fragments and variants of the native sequence. Representative fusions of this type are provided in PCT Publication WO 2008/118970 published on October 2, 2008; WO/2010/006286 published on January 14, 2010; WO/2010/151808 published on December 29, 2010; PCT/US2012/044746 filed June 28, 2012. An example of a fusion with a heterologous leader sequence is illustrated in Figure 6 (SEQ ID NOS: 35 and 36). In a direct fusion, typically the C-terminus of a VpreB sequence (e.g. a VpreB 1, VpreB2 or VpreB3 sequence) is fused to the N-terminus of a λ5 sequence. While it is possible to fuse the entire length of a native VpreB sequence to a full-length λ5 sequence, typically the fusion takes place at or around a CDR3 analogous site in each of the two polypeptides. In this embodiment, the fusion may take place within, or at a location within about 10 amino acid residues at either side of the CDR3 analogous region. In a preferred embodiment, the fusion takes place between about amino acid residues 116-126 of the native human VpreB 1 sequence (SEQ ID NO: 1) and between about amino acid residues 82 and 93 of the native human λ5 sequence (SEQ ID NO: 8).

As noted above, in addition to direct fusions, the polypeptide constructs of the present invention include non-covalent associations of a VpreB sequence (including fragments and variants of a native sequence) with a heterologous sequence, such as a λ5 sequence (including fragments and variants of the native sequence), and/or an antibody sequence. Thus, for example, a full-length VpreB sequence may be non-covalently associated with a truncated λ5 sequence. Alternatively, a truncated VpreB sequence may be non-covalently associated with a full-length λ5 sequence.

Surrogate light chain constructs comprising non-covalently associated VpreB 1 and λ5 sequences, in association with an antibody heavy chain. The association may be covalent and/or non-covalent. The structures may include, for example, full-length VpreB 1 and λ5 sequences, a full-length VpreB 1 sequence associated with a truncated λ5 sequence ("Lambda 5dT"), a truncated VpreB 1 sequence associated with a full-length λ5 sequence (VpreB dT") and a truncated VpreB 1 sequence associated with a truncated λ5 sequence ("Short").

One of ordinary skill will appreciate that a variety of other constructs can be made and used in a similar fashion. For example, the structures can be asymmetrical, comprising different surrogate light chain sequences in each arm, and/or having trimeric or pentameric structures.

All surrogate light chain constructs (Surrobodies) herein may be associated with antibody sequences. For example, a polypeptide comprising one or more VpreB-λ5 fusions can be linked to an antibody heavy chain variable region sequence by a peptide linker. In another embodiment, a VpreB-λ5 fusion is non-covalently associated with an antibody heavy chain, or a fragment thereof including a variable region sequence to form a dimeric complex. In yet another embodiment, the VpreB and λ5 sequences are non-covalently associated with each other and an antibody heavy chain, or a fragment thereof including a variable region sequence, thereby forming a trimeric complex.

In one embodiment, the invention provides an SLC construct wherein the λ5 sequence is non-covalently associated with the VpreB sequence. In one other embodiment, the invention contemplates an SLC construct wherein the conjugate of said VpreB sequence and λ5 sequence is non-covalently associated with an antibody heavy chain sequence.

The present invention also contemplates SLC constructs wherein a λ5 sequence and a VpreB sequence are connected by a covalent linker. In one embodiment, the invention provides an SLC construct wherein the λ5 sequence is non-covalently associated with the VpreB sequence. In one other embodiment, the invention contemplates an SLC construct wherein the conjugate of said VpreB sequence and λ5 sequence is non-covalently associated with an antibody heavy chain sequence.

The binding molecule conjugates of the present invention, including those based upon multispecific Surrobody molecules, may contain VpreB/λ5 conjugates. The VpreB/λ5 conjugates may be SLC polypeptides that are fusions. Exemplary sequences suitable for use in VpreB 1 (SEQ ID NO: 1) / λ5 (SEQ ID NO: 8) conjugates include, without limitation, VpreB 1(20-121), λ5 (93-213), λ5 (93-107), and λ5 (93-108).

### (ii) κ-like surrogate light chains

Specific examples of κ-like Surrobodies include polypeptides in which a Vκ-like sequence, including fragments and variants of the native sequences, is conjugated to a JCκ sequence, including fragments and variants of the native sequence. Representative fusions of this type are illustrated in U.S. Patent Publication No. 2001-0062950, and Xu et al., J. Mol. Biol. 2010, 397, 352-360.

Various heterodimeric surrogate κ light chain deletion variants may be used as surrogage light chains. In the "full length" construct, both the VK-like and JCK sequence retains the C- and N-terminal extensions (tails), respectively. In the dJ variant, the N-terminal extension of JCK has been deleted. In the dVK tail variants, the C-terminal extension of the VK-like sequence had been removed but the N-terminal extension of JCK is retained. In the "short kappa" variant, both the C-terminal tail of the VK-like sequence and the N-terminal extension of the JCK sequence are retained. Single chain constructs may be made between the full length sequences and any of the deletion variants in any combination, e.g., full length single chain, full length VK-like and dJ single chain, full length JCK and dVK, etc.

Specific examples of the polypeptide constructs herein include polypeptides in which a VK-like and/or JCK sequence is associated with an antibody heavy chain, or a fragment thereof. In the κ-like surrogate light chain constructs of the present invention, the VK-like polypeptide and/or the JCK polypeptide may contain the C- and N-terminal extensions, respectively, that are not present in similar antibody sequences. Alternatively, part or whole of the extension(s) can be removed from the κ-like surrogate light chain constructs herein.

Other κ-like surrogate light chain constructs, which can be used individually or can be further derivatized and/or associated with additional heterologous sequences, such as antibody heavy chain sequences, such as a full-length antibody heavy chain or a fragment thereof.

While the C- and N-terminal extensions of the VK-like polypeptide and/or the JCK polypeptide do not need to be present in the constructs of the present invention, it is advantageous to retain at least a part of at least one of such appendages, because they provide a unique opportunity to create combinatorial functional diversity, either by linear extensions or, for example, in the form of constrained diversity, as a result of screening loop libraries, as described in WO/2010/006286 published on January 14, 2010. In addition, the "tail" portions of the VK-like polypeptide and/or the JCK polypeptide can be fused to other peptides and/or polypeptides, to provide for various desired properties, such as, for example, enhanced binding, additional binding specificities, enhanced pK, improved half-life, reduced half-life, cell surface anchoring, enhancement of cellular translocation, dominant negative activities, etc. Specific functional tail extensions are further discussed in WO/2010/151808 published on December 29, 2010.

If desired, the constructs of the present invention can be engineered, for example, by incorporating or appending known sequences or sequence motifs from the CDR1, CDR2 and/or CDR3 regions of antibodies, including known therapeutic antibodies into the CDR1, CDR2 and/or CDR3 analogous regions of the κ-like surrogate light chain sequences. This allows the creation of molecules that are not antibodies, but will exhibit binding specificities and affinities similar to or superior over those of a known therapeutic antibody.

As VK-like and the JCK genes encode polypeptides that can function as independent proteins and function as surrogate light chains, surrogate-like light chains can be engineered from true light chains and be used in every previous application proposed for engineered true surrogate light chains. This can be accomplished by expressing the variable light region to contain a peptidic extension analogous to either the VpreB or VK-like gene. Similarly the constant region can be engineered to resemble either the λ5 or JCK genes and their peptidic extensions. Furthermore any chimeras or heterodimeric partnered combinations are within the scope herein.

In one other aspect, the present invention contemplates multispecific Surrobody molecules comprising surrogate light chain (SLC) domains that have κ-like SLC polypeptides. In one embodiment, the κ-like SLC polypeptide comprises a Vκ-like sequence and/or a JCκ sequence. In another embodiment, the Vκ -like sequence is selected from the group consisting of SEQ ID NOS: 12-24, and fragments and variants thereof. In one other embodiment, the JCκ sequence is selected from the group consisting of SEQ ID NOS:26-39, and fragments and variants thereof. The κ-like SLC domain may be a Vκ -like sequence conjugated to a JCκ sequence. The conjugate may be a fusion. In another embodiment, the fusion takes place at or around the CDR3 analogous regions of said Vκ-like sequence and said JCκ sequence respectively. In one embodiment, the invention contemplates a κ-like SLC construct, wherein said Vκ-like sequence and said JCκ sequence are connected by a covalent linker.

In one embodiment, the invention provides a κ-like SLC construct, wherein said Vκ-like sequence is non-covalently associated with said JCκ sequence. In one embodiment, the invention provides a κ-like SLC construct wherein the conjugate of said Vκ-like sequence and JCκ sequence is non-covalently associated with an antibody heavy chain sequence.

### 2. Multispecific Stacked Variable Domain (SVD) binding molecule conjugates

In one aspect, the binding molecule conjugated described herein are based upon stacked variable domain (SVD) Surroglobulin structures, as described in PCT/US2012/044746 filed June 28, 2012. The agents, e.g., therapeutic and diagnostic agents, described herein may be attached to a surrogate light chain region that is part of an SVD structure via the linker strategies described herein.

SVD Surroglobulin structures are heteromeric binding proteins designed such that two domains from two different parental Surrobodies are covalently linked in tandem directly or via a designed linker. Specifically the first component of the complex is the tandem product of a heavy chain variable domain (VH) of the first surrobody and the surrogate light chain domain of a second surrobody linked together, which is intended to create the "outer" binding domain. The second component of the SVD complex is the tandem product of a surrogate light chain domain of the first surrobody and the heavy chain variable domain (VH) of a second surrobody linked together, which is intended to create the "inner" binding domain. This second component may be followed by a constant domain sequence (e.g. CH1) and, if desired, an Fc region to enable avid binding to both specificities. The two components, though typically single polypeptides, can be individual dimeric proteins. The agents, *e.g.*, therapeutic and diagnostic agents, described herein may be attached to at least one surrogate light chain domain or region via the linker strategies described herein.

In another aspect, the binding molecule conjugates are based upon SVD molecules that utilize different antibody heavy chain constant domain region sequences. In one embodiment, the heavy chain constant domain sequence comprises a sequence selected from the group consisting of: a CH1 sequence, a CH2 sequence, a CH3 sequence, a CH1 and a CH3 sequence, a CH2 and a CH3 sequence, an Fc region, as well as any functionally active fragment thereof.

In another embodiment, the application discloses a binding molecule conjugate based upon an SVD Surroglobulin structure, comprising a single chain product of a heavy chain variable domain (VH) of a first surrobody linked to its cognate surrogate light chain that is intended to create the "outer" binding domain, which is in turn linked to the surrogate light chain of a second surrobody. In this embodiment, the second component of the SVD complex is the heavy chain variable domain (VH) of a second surrobody, which is intended to create the "inner" binding domain. This second heavy chain may be followed by the constant domain (CH1) and if desired the Fc region for avid binding to both each distinct binding target. In this embodiment, the first binding domain specificity is created as a single chain construct fused to the surrogate light chain of a second binding specificity to restore native binding affinities of a parental Surroglobulin (SgG). However, if the second binding domain maintains native binding affinities in the presence of a fusion on the N-terminus then it is also possible to fuse the single chain construct with a similar effect. The agents, *e.g*., therapeutic and diagnostic agents, described herein may be attached to at least one surrogate light chain domain or region via the linker strategies described herein.

Furthermore it is possible to fuse distinct single chain binding domains to both the amino terminus of the surrogate light chain and the amino terminus of the heavy chain to create a trispecific, avid heteromeric binding protein.

In yet another embodiment, a panel of SVD-SgG molecules are created, composed of combinations of heavy chain variable (VH) domains of neutralizing surroglobulins and combinatorial linker diversity to identify combinations with potentiated or additional activity. The beneficial combination have the potential to be generated into a more potent agent, as well as a more consistent product than a cocktail admixture of biologics, such as antibodies.

In another example of targeting a single molecule a single heavy chain variable domain (VH) is used for each of the four binding sites of an SVD-SgG construct, to create a molecule that is capable of either binding stoichiometrically larger amounts of target or creating higher order clusters of the targeted protein.

The multispecific stacked variable domain (SVD) binding molecules, as defined herein, contain different polypeptide components. The present invention contemplates the use of fragments of these polypeptide components, in particular, functional fragments. The term "fragment" refers to a portion of a polypeptide or sequence described herein, generally comprising at least the region involved in binding a target and/or in association with another polypeptide or sequence. A "functional fragment, " as defined herein, is a portion of a polypeptide or sequence which has a qualitative biological activity in common with the original (reference) polypeptide or sequence. Thus, for example, a fragment of a surrogate light chain (SLC) polypeptide or sequence may be a functional fragment, which comprises at least a minimum sequence length required for retaining a qualitative biological activity of the SLC polypeptide or sequence. For example, the functional fragment may retain the qualitative ability to bind a target either alone or in combination with another polypeptide, e.g., an antibody heavy chain variable region sequence, and/or the ability to associate with another polypeptide, e.g., an antibody heavy chain constant region. The agents, e.g., therapeutic and diagnostic agents, described herein may be attached to at least one surrogate light chain domain or region via the linker strategies described herein.

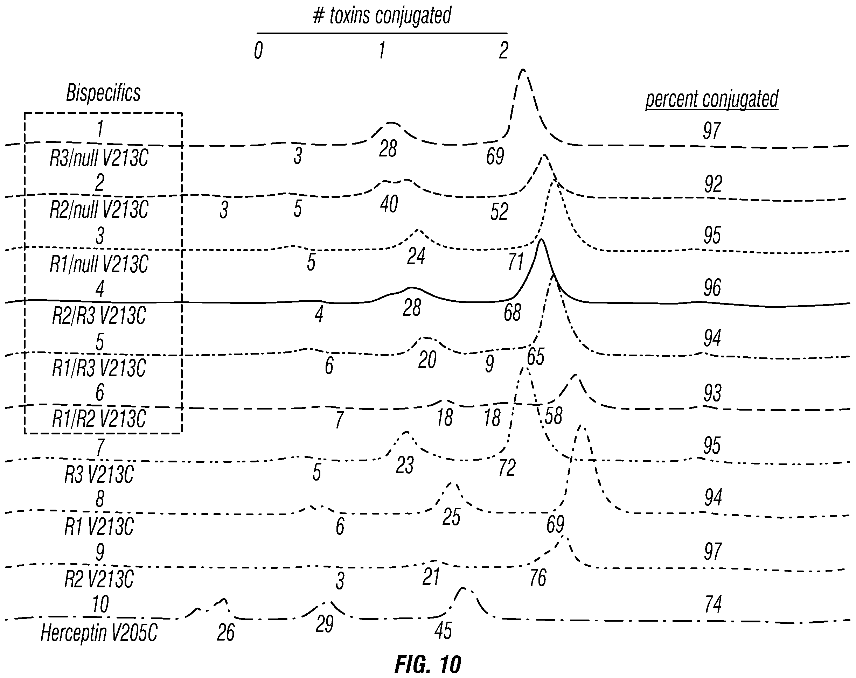

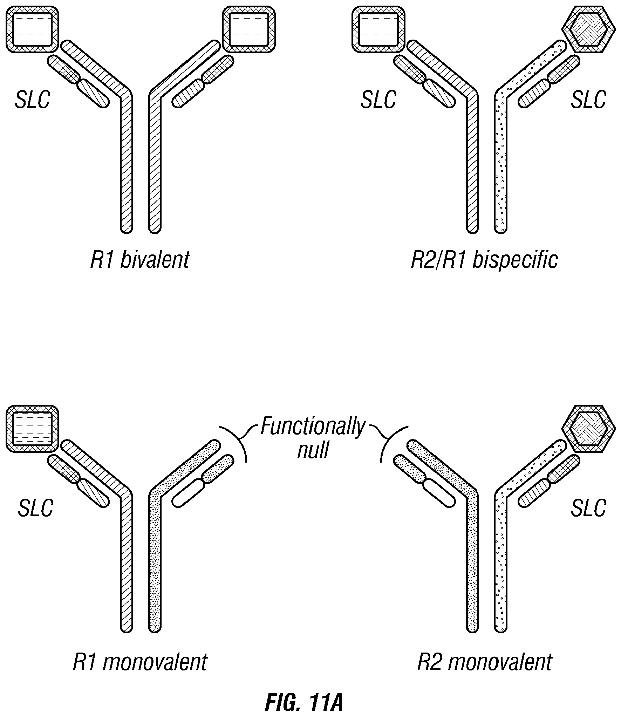

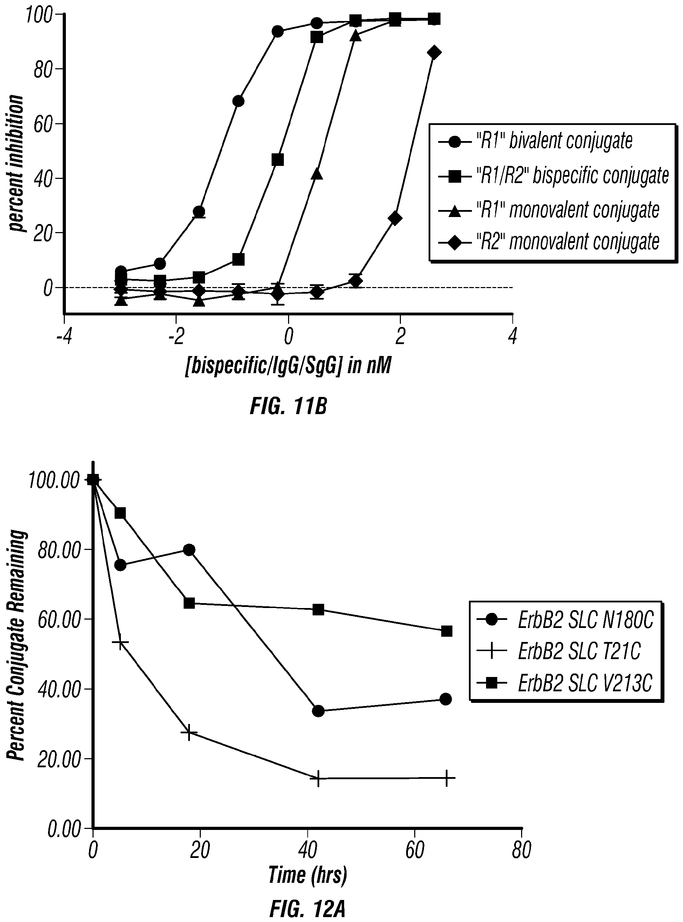

These and further embodiments are illustrated in the Examples and associated Figures of PCT/US2012/044746 filed June 28, 2012. For example, agents may be attached to an SLC region or domain as shown in (i) a bispecific Surrobody structure (Figure 1A of PCT/US2012/044746); (ii) a multispecific/bispecific single chain based Surrobody (scSv) structure (Figure 1B-1E of PCT/US2012/044746); (iii) a monomeric monovalent binder or bivalent avid binder Surrobody structure (Figure 17 of PCT/US2012/044746); (iv) a bispecific monomeric SVD Surrobody structure (Figure 18 of PCT/US2012/044746); (v) a trispecific SVD Surrobody structure; or (vi) a "Cross-complement" SVD Surrobody structure. In one embodiment, the present invention contemplates binding molecule conjugates that comprise an SLC polypeptide having an agent attached to any suitable amino acid residue of the SLC amino acid sequences depicted in Figures 10 and 11 of Figure 18 of PCT/US2012/044746 filed June 28, 2012.

### 3. Functionally null moeities

In another aspect, the present invention provides binding molecule conjugates that comprise a funtionally null moiety. It has been found that the pharmacokinetic properties of biologically active moieties, such as peptides and polypeptides, or associated peptidic and non-peptidic molecules, can be modulated by conjugation to a functionally null scaffold. Thus, for example, the in vivo half-lives of rapidly clearing peptides and polypeptides, or secondarily associated peptidic and non-peptidic molecules, can be extended by conjugation to a longer half-life functionally null scaffold, such as, for example, a functionally null antibody, Surrobody™, or other scaffold comprising a functionally null binding region, such as an Adnectin™ (hereinafter referred to as "Adnectin"), Domain Antibody™ (hereinafter referred to as "Domain Antibody" or "dAB"), DARPin, anti-calin, Affibody, or fragments thereof.

In one aspect, the present invention concerns binding molecule conjugates that comprise a first moiety conjugated to a second moiety, wherein the second moiety is a scaffold comprising one or more functionally null binding regions conjugated to and capable of modulating at least one pharmacokinetic property of the first moiety. In another aspect, the present invention concerns binding molecule conjugates that comprise a fusion molecule, wherein the fusion molecule comprises a first moiety and a second moiety, wherein said second moiety comprises one or more functionally null binding regions fused to and capable of modulating at least one pharmacokinetic property of the first moiety. In some embodiments, the first moiety is a peptide or a polypeptide. The peptide or polypeptide may be a biologically active moiety. In yet another embodiment, the biologically active moiety and the scaffold or moiety comprising one or more functionally null binding regions are fused to each other.

In other embodiments, the scaffold or moiety comprising one or more functionally null binding regions may, for example, be selected from the group consisting of antibodies, Adnectins, Domain Antibodies (Dabs), DARPins, anti-calins, Affibodies, and fragments thereof. Thus, the scaffold or moiety comprising one or more functionally null binding regions may be an antibody or an antibody fragment, or a Surrobody or a fragment thereof.

In another embodiment, the fuctionally null moieties that make up part of the binding molecule conjugates are based upon antibodies. Such antibodies are created from germline heavy and light chains from a combination of unmutated "V-J" light chain and unmutated "V-D-J" genes to encode the null antibody polypeptides. As most binding is dictated by the heavy chain CDR3 region, the possibility of binding to any foreign or non-foreign target can be further reduced by removing the D-region or using a designed minimal D-region in creating the null antibody. Further engineering or additional deletions of portions of the V and J regions are possible to further reinforce nonreactivitiy. Similar strategies can be applied to produce functionally null Surrobodies and other moieties with functionally null binding regions.

Funtionally null moieites are further described in WO/2011/071957 published on June 16, 2011.

### 4. Binding molecule conjugates comprising SLCs

The present invention is directed to binding molecule conjugates comprising a surrogate light chain, or fragment thereof, linked or conjugated to a drug or prodrug (also referred to herein as Surrobody conjugates). Suitable drugs or prodrugs are known in the art. The drugs or prodrugs can be cytotoxic agents. The cytotoxic agent used in the cytotoxic conjugate of the described herein can be any compound that results in the death of a cell, or induces cell death, or in some manner decreases cell viability, and includes, for example, maytansinoids and maytansinoid analogs. Other suitable cytotoxic agents are for example benzodiazepines, taxoids, CC-1065 and CC-1065 analogs, duocarmycins and duocarmycin analogs, enediynes, such as calicheamicins, dolastatin and dolastatin analogs including auristatins, tomaymycin derivatives, leptomycin derivaties, methotrexate, cisplatin, carboplatin, daunorubicin, doxorubicin, vincristine, vinblastine, melphalan, mitomycin C, chlorambucil and morpholino doxorubicin.

Such conjugates can be prepared by using a linking group in order to link a drug or prodrug to the surrogate light chain. Suitable linking groups are well known in the art and include, for example, disulfide groups, thioether groups, acid labile groups, photolabile groups, peptidase labile groups and esterase labile groups.

The drug or prodrug can, for example, be linked to the surrogate light chain (SLC), or fragment thereof, through a disulfide bond. The linker molecule or crosslinking agent comprises a reactive chemical group that can react with the SLC or fragment thereof. The reactive chemical groups for reaction with the surrogate light chain can be N-succinimidyl esters and N-sulfosuccinimidyl esters. Additionally the linker molecule comprises a reactive chemical group, which can be a dithiopyridyl group that can react with the drug to form a disulfide bond. Linker molecules include, for example, N-succinimidyl 3-(2-pyridyldithio) propionate (SPDP) (see, e.g., Carlsson et al., Biochem. J., 173: 723-737 (1978)), N-succinimidyl 4-(2-pyridyldithio)butanoate (SPDB) (see, e.g., U.S. Pat. No. 4,563,304), N-succinimidyl 4-(2-pyridyldithio)-2-sulfobutanoate (sulfo-SPDB) (see US Publication No. 20090274713), N-succinimidyl 4-(2-pyridyldithio) pentanoate (SPP) (see, e.g., CAS Registry number 341498-08-6), 2-iminothiolane, or acetylsuccinic anhydride. For example, the SLC, or fragment thereof, can be modified with crosslinking reagents and the SLC, or fragment thereof, containing free or protected thiol groups thus derived is then reacted with a disulfide- or thiol-containing maytansinoid to produce conjugates. The conjugates can be purified by chromatography, including but not limited to HPLC, size-exclusion, adsorption, ion exchange and affinity capture, dialysis or tangential flow filtration.

In another aspect of the present invention, the surrogate light chain (SLC), or fragment thereof, is linked to the cytotoxic drugs via disulfide bonds and a polyethylene glycol spacer in enhancing the potency, solubility or the efficacy of the conjugate. Such cleavable hydrophilic linkers are described in WO2009/0134976. The additional benefit of this linker design is the desired high monomer ratio and the minimal aggregation of the conjugate. Specifically contemplated in this aspect are conjugates of SLCs, or a fragment thereof, and drugs linked via disulfide group (--S--S--) bearing polyethylene glycol spacers ((CH₂CH₂O)ₙ₌₁₋₁₄) with a narrow range of drug load of 2-8 are described that show relatively high potent biological activity toward cancer cells and have the desired biochemical properties of high conjugation yield and high monomer ratio with minimal protein aggregation.

Binding molecule conjugates with non-cleavable linkers can also be prepared. Such crosslinkers are described in the art (see US Publication No. 20050169933) and include but are not limited to, N-succinimidyl 4-(maleimidomethyl)cyclohexanecarboxylate (SMCC). In some embodiments, a surrogagte light chain (SLC), or fragment thereof, is modified with crosslinking reagents such as succinimidyl 4-(N-maleimidomethyl)-cyclohexane-1-carboxylate (SMCC), sulfo-SMCC, maleimidobenzoyl-N-hydroxysuccinimide ester (MBS), sulfo-MBS or succinimidyl-iodoacetate, as described in the literature, to introduce 1-10 reactive groups (Yoshitake et al, Eur. J. Biochem., 101:395-399 (1979); Hashida et al, J. Applied Biochem., 56-63 (1984); and Liu et al, Biochem., 18:690-697 (1979)). The modified SLC, or fragment thereof, is then reacted with the thiol-containing maytansinoid derivative to produce a conjugate. The conjugate can be purified by gel filtration through a Sephadex G25 column or by dialysis or tangential flow filtration. The modified binding molecules are treated with the thiol-containing maytansinoid (1 to 2 molar equivalent/maleimido group) and conjugates are purified by gel filtration through a Sephadex G-25 column, chromatography on a ceramic hydroxyapatite column, dialysis or tangential flow filtration or a combination of methods thereof. Typically, an average of 1-10 maytansinoids per binding molecule are linked. One method is to modify an SLC, or fragment thereof, with succinimidyl 4-(N-maleimidomethyl)-cyclohexane-1-carboxylate (SMCC) to introduce maleimido groups followed by reaction of the modified SLC, or fragment thereof, with a thiol-containing maytansinoid to give a thioether-linked conjugate. Again conjugates with 1 to 10 drug molecules per binding molecule result.

In another aspect of the invention, the surrogate light chain (SLC), or fragment thereof, is linked to the drug via a non-cleavable bond through the intermediacy of a PEG spacer. Suitable crosslinking reagents comprising hydrophilic PEG chains that form linkers between a drug and the SLC, or fragment thereof, are also well known in the art, or are commercially available (for example from Quanta Biodesign, Powell, Ohio). Suitable PEG-containing crosslinkers can also be synthesized from commercially available PEGs themselves using standard synthetic chemistry techniques known to one skilled in the art. The drugs can be reacted with bifunctional PEG-containing cross linkers to give compounds of the following formula, Z--X₁--(--CH₂--CH₂--O--)ₙ--Yₚ-D, by methods described in detail in US Patent Publication 20090274713 and in WO2009/0134976, which can then react with the SLC, or fragment thereof, to provide a conjugate.

Alternatively, the cell binding can be modified with the bifunctional PEG crosslinker to introduce a thiol-reactive group (such as a maleimide or haloacetamide) which can then be treated with a thiol-containing maytansinoid to provide a conjugate. In another method, the cell binding can be modified with the bifunctional PEG crosslinker to introduce a thiol moiety which can then be treated with a thiol-reactive maytansinoid (such as a maytansinoid bearing a maleimide or haloacetamide), to provide a conjugate.

The present invention includes aspects wherein about 2 to about 8 drug molecules ("drug load"), for example, maytansinoid, are linked to a surrogate light chain (SLC) or fragment thereof, the anti-tumor effect of the conjugate is much more efficacious as compared to a drug load of a lesser or higher number of drugs linked to the same SLC or fragment thereof. "Drug load", as used herein, refers to the number of drug molecules (e.g., a maytansinoid) that can be attached to a binding molecule (e.g., an SLC or fragment thereof). In one aspect the number of drug molecules that can be attached to a cell binding agent can average from about 2 to about 8 (e.g., 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1). N²'-deacetyl-N²'-(3-mercapto-1-oxopropyl)-maytansine (DM1) and N²'-deacetyl-N²'-(4-mercapto-4-methyl-1-oxopentyl) maytansine (DM4) can be used.

The surrogate light chain (SLC) or fragment thereof can be modified by reacting a bifunctional crosslinking reagent with the SLC or fragment thereof, thereby resulting in the covalent attachment of a linker molecule to the SLC or fragment thereof. As used herein, a "bifunctional crosslinking reagent" is any chemical moiety that covalently links an SLC or fragment thereof to a drug, such as the drugs described herein. In another method, a portion of the linking moiety is provided by the drug. In this respect, the drug comprises a linking moiety that is part of a larger linker molecule that is used to join the SLC or fragment thereof to the drug. For example, to form the maytansinoid DM1, the side chain at the C-3 hydroxyl group of maytansine is modified to have a free sulfhydryl group (SH). This thiolated form of maytansine can react with a modified SLC or fragment thereof to form a conjugate. Therefore, the final linker is assembled from two components, one of which is provided by the crosslinking reagent, while the other is provided by the side chain from DM1.

The drug molecules can also be linked to the surrogate light chain (SLC) or fragment thereof through an intermediary carrier molecule such as serum albumin.

As used herein, the expression "linked to a surrogate light chain or fragment thereof" or "linked to an SLC or fragment thereof" refers to the binding molecule conjugate comprising at least one drug derivative bound to an SLC or fragment thereof via a suitable linking group, or a precursor thereof. One linking group is SMCC.

In certain embodiments, cytotoxic agents useful in the present invention are maytansinoids and maytansinoid analogs. Examples of suitable maytansinoids include esters of maytansinol and maytansinol analogs. Included are any drugs that inhibit microtubule formation and that are highly toxic to mammalian cells, as are maytansinol and maytansinol analogs.

Examples of suitable maytansinol esters include those having a modified aromatic ring and those having modifications at other positions. Such suitable maytansinoids are disclosed in U.S. Pat. Nos. 4,424,219; 4,256,746; 4,294,757; 4,307,016; 4,313,946; 4,315,929; 4,331,598; 4,361,650; 4,362,663; 4,364,866; 4,450,254; 4,322,348; 4,371,533; 5,208,020; 5,416,064; 5,475,092; 5,585,499; 5,846,545; 6,333,410; 7,276,497 and 7,473,796.

In a certain embodiment, the binding molecule conjugates described herein utilize the thiol-containing maytansinoid (DM1), formally termed N²'-deacetyl-N²'-(3-mercapto-1-oxopropyl)-maytansine; the thiol-containing maytansinoid N²'-deacetyl-N²'(4-methyl-4-mercapto-1-oxopentyl)-maytansine (e.g., DM4); or a maytansinoid comprising a side chain that contains a sterically hindered thiol bond is N²'-deacetyl-N²'(4-mercapto-1-oxopentyl)-maytansine (termed DM3), as the cytotoxic agent.

Each of the maytansinoids taught in U.S. Pat. Nos. 5,208,020 and 7,276,497, can also be used in the conjugate of the present invention. Many positions on maytansinoids can serve as the position to chemically link the linking moiety. For example, the C-3 position having a hydroxyl group, the C-14 position modified with hydroxymethyl, the C-15 position modified with hydroxy and the C-20 position having a hydroxy group are all expected to be useful. In some embodiments, the C-3 position serves as the position to chemically link the linking moiety, and in some particular embodiments, the C-3 position of maytansinol serves as the position to chemically link the linking moiety.

Several descriptions for producing such polypeptide-maytansinoid conjugates are provided in U.S. Pat. Nos. 6,333,410, 6,441,163, 6,716,821, and 7,368,565.

In general, a solution containing a surrogate light chain (SLC) or fragment thereof in aqueous buffer can be incubated with a molar excess of maytansinoids having a disulfide moiety that bears a reactive group. The reaction mixture can be quenched by addition of excess amine (such as ethanolamine, taurine, etc.). The maytansinoid conjugate can then be purified by gel filtration.

The number of maytansinoid molecules bound per binding molecule can be determined by measuring spectrophotometrically the ratio of the absorbance at 252 nm and 280 nm. The average number of maytansinoid molecules/binding molecule can be, for example, 1-10 or 2-5.

Anthracycline compounds, as well as derivatives, intermediates and modified versions thereof, can also be used to prepare binding molecules described herein. For example, doxorubicin, doxorubicin derivatives, doxorubicin intermediates, and modified doxorubicins can be used in binding molecule conjugates. Exemplary compounds are described in WO 2010/009124.

Conjugates comprising a surrogate light chain (SLC) or fragment thereof with maytansinoid or other drugs can be evaluated for their ability to suppress proliferation of various unwanted cell lines in vitro. For example, cell lines such NCI-H226, NCI-H292, and NCI-H322M, can easily be used for the assessment of cytotoxicity of these compounds. Cells to be evaluated can be exposed to the conjugates for 4 to 5 days and the surviving fractions of cells measured in direct assays by known methods. IC₅₀ values can then be calculated from the results of the assays.

Preferred cross-linking reagents that form non-cleavable linkers between the maytansinoid and the binding molecule comprise a maleimido- or haloacetyl-based moiety. According to the present invention, such non-cleavable linkers are said to be derived from maleimido- or haloacetyl-based moiety. Cross-linking reagents comprising a maleimido-based moiety include N-succinimidyl 4-(maleimidomethyl)cyclohexanecarboxylate (SMCC), N-succinimidyl-4-(N-maleimidomethyl)-cyclohexane-1-carboxy-(6-amidocaproa-te), which is a "long chain" analog of SMCC (LC-SMCC), κ-maleimidoundecanoic acid N-succinimidyl ester (KMUA), γ-maleimidobutyric acid N-succinimidyl ester (GMBS), ε-maleimidocaproic acid N-hydroxysuccinimide ester (EMCS), m-maleimidobenzoyl-N-hydroxysuccinimide ester (MBS), N-(α-maleimidoacetoxy)-succinimide ester [AMAS], succinimidyl-6-(β-maleimidopropionamido)hexanoate (SMPH), N-succinimidyl 4-(p-maleimidophenyl)-butyrate (SMPB), and N-(p-maleimidophenyl)isocyanate (PMPI). These cross-linking reagents form non-cleavable linkers derived from maleimido-based moieties.

Cross-linking reagents comprising a haloacetyl-based moiety include N-succinimidyl-4-(iodoacetyl)-aminobenzoate (SIAB), N-succinimidyl iodoacetate (SIA), N-succinimidyl bromoacetate (SBA) and N-succinimidyl 3-(bromoacetamido)propionate (SBAP). These cross-linking reagents form non-cleavable linkers derived from haloacetyl-based moieties.

Additional agents and linkers suitable for use are provided in U.S. 20120156217; U.S. 8198417; and U.S. 20120294853.

### Surrobody-Drug Conjugate Compounds

The present invention provides, inter alia, binding molecule-drug conjugates for targeted delivery of drugs. The inventors have made the discovery that the binding molecule-drug conjugates comprising a surrogate light chain (SLC) or fragment thereof have advantageous therapeutic properties including, without limitation, potent cytotoxic activity, serum stability, a high percentage of drug conjugation, and reduced aggregation following concentration .

In one aspect, the conjugates comprise an SLC unit covalently linked to at least one Drug unit. The Drug units can be covalently linked directly or via a Linker unit (-L-). In some embodiments, the conjugate has the following formula:

SLC-(L-D)ₚ (I)

or a pharmaceutically acceptable salt or solvate thereof; wherein:
SLC is the surrogate light chain or fragment thereof unit, and
(L-D) is a Linker unit-Drug unit moiety, wherein:
   L- is a Linker unit, and
   D is a drug unit; and
   p is an integer from 1 to about 20.

In some embodiments, p ranges from 1 to 10, 1 to 9, 1 to 8, 1 to 7, 1 to 6, 1 to 5, 1 to 4, 1 to 3, or 1 to 2. In some embodiments, p ranges from 2 to 10, 2 to 9, 2 to 8, 2 to 7, 2 to 6, 2 to 5, 2 to 4 or 2 to 3. In other embodiments, p is 1, 2, 3, 4, 5 or 6. In some embodiments, p is 2 or 4.

In some embodiments, the conjugate has the following formula:

SLC-(Aₐ-W_{w}-Y_{y}-D)ₚ (II)

or a pharmaceutically acceptable salt or solvate thereof;
wherein:
SLC is the surrogate light chain or fragment thereof unit; and
-Aₐ-W_{w}-Y_{y}- is a Linker unit (LU), wherein:
   -A- is a Stretcher unit,
   a is 0 or 1,
   each -W- is independently an Amino Acid unit,
   w is an integer ranging from 0 to 12,
   -Y- is a self-immolative spacer unit,
   y is 0, 1 or 2;
   -D is a drug unit; and
   p is an integer from 1 to about 20.

In some embodiments, a is 0 or 1, w is 0 or 1, and y is 0, 1 or 2. In some embodiments, a is 0 or 1, w is 0 or 1, and y is 0 or 1. In some embodiments, p ranges from 1 to 10, 1 to 9, 1 to 8, 1 to 7, 1 to 6, 1 to 5, 1 to 4, 1 to 3, or 1 to 2. In some embodiments, p ranges from 2 to 8, 2 to 7, 2 to 6, 2 to 5, 2 to 4 or 2 to 3. In other embodiments, p is 1, 2, 3, 4, 5 or 6. In some embodiments, p is 2 or 4. In some embodiments, when w is not zero, y is 1 or 2. In some embodiments, when w is 1 to 12, y is 1 or 2. In some embodiments, w is 2 to 12 and y is 1 or 2. In some embodiments, a is 1 and w and y are 0.

The drug loading is represented by p, the average number of drug molecules per binding molecule comprising an SLC or fragment thereof. Drug loading may range from 1 to 20 drugs (D) per binding molecule. The average number of drugs per binding molecule in preparation of conjugation reactions may be characterized by conventional means such as mass spectroscopy, ELISA assay, and HPLC. The quantitative distribution of the conjugates in terms of p may also be determined. In some instances, separation, purification, and characterization of homogeneous conjugates where p is a certain value from the conjugates with other drug loadings may be achieved by means such as reverse phase HPLC or electrophoresis. In exemplary embodiments, p is from 2 to 8.

The generation of the binding molecule-drug conjugate can be accomplished by any technique known to the skilled artisan. Briefly, the conjugate compounds comprise a binding molecule comprising a surrogate light chain (SLC) or fragment thereof as the SLC unit, a drug, and optionally a linker that joins the drug and the binding molecule. A number of different reactions are available for covalent attachment of drugs and/or linkers to binding agents. This is often accomplished by reaction of the amino acid residues of the binding molecule, e.g., SLC or fragment thereof, including the amine groups of lysine, the free carboxylic acid groups of glutamic and aspartic acid, the sulfhydryl groups of cysteine and the various moieties of the aromatic amino acids. One of the most commonly used non-specific methods of covalent attachment is the carbodiimide reaction to link a carboxy (or amino) group of a compound to amino (or carboxy) groups of a polypeptide molecule. Additionally, bifunctional agents such as dialdehydes or imidoesters have been used to link the amino group of a compound to amino groups of a polypeptide molecule. Also available for attachment of drugs to binding agents is the Schiff base reaction. This method involves the periodate oxidation of a drug that contains glycol or hydroxy groups, thus forming an aldehyde which is then reacted with the binding molecule. Attachment occurs via formation of a Schiff base with amino groups of the binding molecule. Isothiocyanates can also be used as coupling agents for covalently attaching drugs to binding molecules. Other techniques are known to the skilled artisan and within the scope of the present invention.

In certain embodiments, an intermediate, which is the precursor of the linker, is reacted with the drug under appropriate conditions. In certain embodiments, reactive groups are used on the drug and/or the intermediate. The product of the reaction between the drug and the intermediate, or the derivatized drug, is subsequently reacted with the binding molecule under appropriate conditions.

Each of the particular units of the conjugate compounds is described in more detail herein. The synthesis and structure of exemplary linker units, stretcher units, amino acid units, self-immolative spacer unit, and drug units are also described in U.S. Patent Application Publication Nos. 2003-0083263, 2005-0238649 and 2005-0009751.

### Linker Units

Typically, the binding molecule-drug conjugates comprise a linker region between the drug unit and the SLC unit. In some embodiments, the linker is cleavable under intracellular conditions, such that cleavage of the linker releases the drug unit from the binding molecule in the intracellular environment. In yet other embodiments, the linker unit is not cleavable and the drug is released, for example, by degradation of the binding molecule.

In some embodiments, the linker is cleavable by a cleaving agent that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside target cells (see, e.g., Dubowchik and Walker, 1999, Pharm. Therapeutics 83:67-123). Most typical are peptidyl linkers that are cleavable by enzymes that are present in cells. For example, a peptidyl linker that is cleavable by the thiol-dependent protease cathepsin-B, which is highly expressed in cancerous tissue, can be used (e.g., a Phe-Leu or a Gly-Phe-Leu-Gly linker (SEQ ID NO: 51)). Other examples of such linkers are described, e.g., in U.S. Pat. No. 6,214,345. In a specific embodiment, the peptidyl linker cleavable by an intracellular protease is a Val-Cit linker or a Phe-Lys linker (see, e.g., U.S. Pat. No. 6,214,345, which describes the synthesis of doxorubicin with the val-cit linker). One advantage of using intracellular proteolytic release of the therapeutic agent is that the agent is typically attenuated when conjugated and the serum stabilities of the conjugates are typically high.

In other embodiments, the cleavable linker is pH-sensitive, i.e., sensitive to hydrolysis at certain pH values. Typically, the pH-sensitive linker hydrolyzable under acidic conditions. For example, an acid-labile linker that is hydrolyzable in the lysosome (e.g., a hydrazone, semicarbazone, thiosemicarbazone, cis-aconitic amide, orthoester, acetal, ketal, or the like) can be used. (See, e.g., U.S. Pat. Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, e.g., a thioether attached to the therapeutic agent via an acylhydrazone bond (see, e.g., U.S. Pat. No. 5,622,929).

In yet other embodiments, the linker is cleavable under reducing conditions (e.g., a disulfide linker). A variety of disulfide linkers are known in the art, including, for example, those that can be formed using SATA (N-succinimidyl-5-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene)- , SPDB and SMPT (See, e.g., Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987. See also U.S. Pat. No. 4,880,935.)

In yet other specific embodiments, the linker is a malonate linker (Johnson et al., 1995, Anticancer Res. 15:1387-93), a maleimidobenzoyl linker (Lau et al., 1995, Bioorg-Med-Chem. 3(10):1299-1304), or a 3'-N-amide analog (Lau et al., 1995, Bioorg-Med-Chem. 3(10):1305-12).

In yet other embodiments, the linker unit is not cleavable and the drug is released by degradation. (See U.S. Publication No. 20050238649).

Typically, the linker is not substantially sensitive to the extracellular environment. As used herein, "not substantially sensitive to the extracellular environment," in the context of a linker, means that no more than about 20%, typically no more than about 15%, more typically no more than about 10%, and even more typically no more than about 5%, no more than about 3%, or no more than about 1% of the linkers, in a sample of binding molecule-drug conjugate, are cleaved when the binding molecule-drug conjugate presents in an extracellular environment (e.g., in plasma). Whether a linker is not substantially sensitive to the extracellular environment can be determined, for example, by incubating with plasma the conjugate for a predetermined time period (e.g., 2, 4, 8, 16, or 24 hours) and then quantitating the amount of free drug present in the plasma.

A variety of exemplary linkers that can be used with the present compositions and methods are described in WO 2004-010957, U.S. Publication No. 20060074008, U.S. Publication No. 20050238649, and U.S. Publication No. 20060024317.

A "Linker unit" (LU) is a bifunctional compound that can be used to link a Drug unit and an SLC unit to form a binding molecule-drug conjugate. In some embodiments, the Linker unit has the formula:

-Aₐ-W_{w}-Y_{y}-

wherein: -A- is a Stretcher unit,
a is 0 or 1,
each -W- is independently an Amino Acid unit,
w is an integer ranging from 0 to 12,
-Y- is a self-immolative Spacer unit, and
y is 0, 1 or 2.

In some embodiments, a is 0 or 1, w is 0 or 1, and y is 0, 1 or 2. In some embodiments, a is 0 or 1, w is 0 or 1, and y is 0 or 1. In some embodiments, when w is 1 to 12, y is 1 or 2. In some embodiments, w is 2 to 12 and y is 1 or 2. In some embodiments, a is 1 and w and y are 0.

### The Stretcher Unit

The Stretcher unit (A), when present, is capable of linking an SLC unit to an Amino Acid unit (-W-), if present, to a Spacer unit (-Y-), if present; or to a Drug unit (-D). Useful functional groups that can be present on a binding molecule, either naturally or via chemical manipulation include, but are not limited to, sulfhydryl, amino, hydroxyl, the anomeric hydroxyl group of a carbohydrate, and carboxyl. Suitable functional groups are sulfhydryl and amino. In one example, sulfhydryl groups can be generated by reduction of the intramolecular disulfide bonds of a binding molecule, e.g., a Surrobody™. In another embodiment, sulfhydryl groups can be generated by reaction of an amino group of a lysine moiety of a surrogate light chain (SLC) or fragment thereof with 2-iminothiolane (Traut's reagent) or other sulfhydryl generating reagents. In certain embodiments, the binding molecule is a Surrobody™ and is engineered to carry one or more lysines. In certain other embodiments, the Surrobody™ is engineered to carry additional sulfhydryl groups, e.g., additional cysteines.

In one embodiment, the Stretcher unit forms a bond with a sulfur atom of the SLC unit. The sulfur atom can be derived from a sulfhydryl group of an SLC or fragment thereof. Representative Stretcher units of this embodiment are depicted in U.S. Published Patent Application No. 20120294853. It is to be understood from all the exemplary embodiments that even where not denoted expressly, from 1 to 20 drug moieties can be linked to a Ligand (p=1-20). In certain embodiments, the Stretcher unit is linked to the Ligand unit via a disulfide bond between a sulfur atom of the Ligand unit and a sulfur atom of the Stretcher unit. In yet other embodiments, the Stretcher contains a reactive site that can form a bond with a primary or secondary amino group of a Ligand. Examples of these reactive sites include, but are not limited to, activated esters such as succinimide esters, 4 nitrophenyl esters, pentafluorophenyl esters, tetrafluorophenyl esters, anhydrides, acid chlorides, sulfonyl chlorides, isocyanates and isothiocyanates.

In some embodiments, the Stretcher contains a reactive site that is reactive to a modified carbohydrate's (--CHO) group that can be present on a Ligand. For example, a carbohydrate can be mildly oxidized using a reagent such as sodium periodate and the resulting (--CHO) unit of the oxidized carbohydrate can be condensed with a Stretcher that contains a functionality such as a hydrazide, an oxime, a primary or secondary amine, a hydrazine, a thiosemicarbazone, a hydrazine carboxylate, and an arylhydrazide such as those described by Kaneko et al., 1991, Bioconjugate Chem. 2:133-41.

### The Amino Acid Unit

The Amino Acid unit (-W-), when present, links the Stretcher unit to the Spacer unit if the Spacer unit is present, links the Stretcher unit to the Drug moiety if the Spacer unit is absent, and links the Ligand unit to the Drug unit if the Stretcher unit and Spacer unit are absent. -W_{w}- can be, for example, a monopeptide, dipeptide, tripeptide, tetrapeptide, pentapeptide, hexapeptide, heptapeptide, octapeptide, nonapeptide, decapeptide, undecapeptide or dodecapeptide unit. In some embodiments, the Amino Acid unit can be enzymatically cleaved by one or more enzymes, including a cancer or tumor-associated protease, to liberate the Drug unit (-D), which in one embodiment is protonated in vivo upon release to provide a Drug (D). In certain embodiments, the Amino Acid unit can comprise natural amino acids. In other embodiments, the Amino Acid unit can comprise non-natural amino acids. Useful -W_{w}- units can be designed and optimized in their selectivity for enzymatic cleavage by a particular enzyme, for example, a tumor-associated protease. In one embodiment, a -W_{w}- unit is that whose cleavage is catalyzed by cathepsin B, C and D, or a plasmin protease. In one embodiment, -W.sub.w-- is a dipeptide, tripeptide, tetrapeptide or pentapeptide.

In one aspect of the Amino Acid unit, the Amino Acid unit is valine-citrulline (vc or val-cit). In another aspect, the Amino Acid unit is phenylalanine-lysine (i.e., fk). In yet another aspect of the Amino Acid unit, the Amino Acid unit is N-methylvaline-citrulline. In yet another aspect, the Amino Acid unit is 5-aminovaleric acid, homo phenylalanine lysine, tetraisoquinolinecarboxylate lysine, cyclohexylalanine lysine, isonepecotic acid lysine, beta-alanine lysine, glycine serine valine glutamine (SEQ ID NO: 52) and isonepecotic acid.

### The Spacer Unit

The Spacer unit (-Y-), when present, links an Amino Acid unit to the Drug unit when an Amino Acid unit is present. Alternately, the Spacer unit links the Stretcher unit to the Drug unit when the Amino Acid unit is absent. The Spacer unit also links the Drug unit to the Ligand unit when both the Amino Acid unit and Stretcher unit are absent. Spacer units are of two general types: non self-immolative or self-immolative. A non self-immolative Spacer unit is one in which part or all of the Spacer unit remains bound to the Drug moiety after cleavage, particularly enzymatic, of an Amino Acid unit from the ligand-drug conjugate. Examples of a non self-immolative Spacer unit include, but are not limited to a (glycine-glycine) Spacer unit and a glycine Spacer unit. When a conjugate containing a glycine-glycine Spacer unit or a glycine Spacer unit undergoes enzymatic cleavage via an enzyme (e.g., a tumor-cell associated-protease, a cancer-cell-associated protease or a lymphocyte-associated protease), a glycine-glycine-Drug moiety or a glycine-Drug moiety is cleaved from L-Aa-Ww-. In one embodiment, an independent hydrolysis reaction takes place within the target cell, cleaving the glycine-Drug moiety bond and liberating the Drug.

In some embodiments, a non self-immolative Spacer unit (-Y-) is -Gly-. In some embodiments, a non self-immolative Spacer unit (-Y-) is -Gly-Gly-.

In one embodiment, a Drug-Linker conjugate is provided in which the Spacer unit is absent (y=0), or a pharmaceutically acceptable salt or solvate thereof.

Alternatively, a conjugate containing a self-immolative Spacer unit can release -D. As used herein, the term "self-immolative Spacer" refers to a bifunctional chemical moiety that is capable of covalently linking together two spaced chemical moieties into a stable tripartite molecule. It will spontaneously separate from the second chemical moiety if its bond to the first moiety is cleaved.

In some embodiments, -Y_{y}- is a p-aminobenzyl alcohol (PAB) unit whose phenylene portion is substituted with Qₘ wherein Q is --C₁-C₈ alkyl, --C₁-C₈ alkenyl, --C₁-C₈ alkynyl, --O--(C₁-C₈ alkyl), --O-(C₁-C₈ alkenyl), --O--(C₁-C₈ alkynyl), -halogen, -nitro or -cyano; and m is an integer ranging from 0-4. The alkyl, alkenyl and alkynyl groups, whether alone or as part of another group, can be optionally substituted with A1 as defined herein.

In some embodiments, -Y- is a PAB group that is linked to -W_{w}-- via the amino nitrogen atom of the PAB group, and connected directly to -D via a carbonate, carbamate or ether group.

Other examples of self-immolative spacers include, but are not limited to, aromatic compounds that are electronically similar to the PAB group such as 2-aminoimidazol-5-methanol derivatives (Hay et al., 1999, Bioorg. Med. Chem. Lett. 9:2237) and ortho or para-aminobenzylacetals. Spacers can be used that undergo cyclization upon amide bond hydrolysis, such as substituted and unsubstituted 4-aminobutyric acid amides (Rodrigues et al., 1995, Chemistry Biology 2:223), appropriately substituted bicyclo[2.2.1] and bicyclo[2.2.2] ring systems (Storm et al., 1972, J. Amer. Chem. Soc. 94:5815) and 2-aminophenylpropionic acid amides (Amsberry et al., 1990, J. Org. Chem. 55:5867) Elimination of amine-containing drugs that are substituted at the α-position of glycine (Kingsbury et al., 1984, J. Med. Chem. 27:1447) are also examples of self-immolative spacers.

In some embodiments, the -D moieties are the same. In yet another embodiment, the -D moieties are different.

### The Drug Unit

The drug moiety (D) is a cytotoxic drug, selected from the group consisting of a maytansinoid, a monomethyl auristatin E (MME) and a monomethyl auristatin F (MMAF). D is a Drug unit (moiety) having an atom that can form a bond with the Spacer unit, with the Amino Acid unit, with the Stretcher unit or with the Ligand unit. In some embodiments, the Drug unit D has a nitrogen atom that can form a bond with the Spacer unit. As used herein, the terms "drug unit" and "drug moiety" are synonymous and used interchangeably.

Useful classes of cytotoxic or immunomodulatory agents include, for example, antitubulin agents, auristatins, DNA minor groove binders, DNA replication inhibitors, alkylating agents (e.g., platinum complexes such as cis-platin, mono(platinum), bis(platinum) and tri-nuclear platinum complexes and carboplatin), anthracyclines, antibiotics, antifolates, antimetabolites, chemotherapy sensitizers, duocarmycins, camptothecins, etoposides, fluorinated pyrimidines, ionophores, lexitropsins, nitrosoureas, platinols, pre-forming compounds, purine antimetabolites, puromycins, radiation sensitizers, steroids, taxanes, topoisomerase inhibitors, vinca alkaloids, or the like.

Individual cytotoxic or immunomodulatory agents include, for example, an androgen, anthramycin (AMC), asparaginase, 5-azacytidine, azathioprine, bleomycin, busulfan, buthionine sulfoximine, calicheamicin, camptothecin, carboplatin, carmustine (BSNU), CC-1065, chlorambucil, cisplatin, colchicine, cyclophosphamide, cytarabine, cytidine arabinoside, cytochalasin B, dacarbazine, dactinomycin (formerly actinomycin), daunorubicin, decarbazine, docetaxel, doxorubicin, etoposide, an estrogen, 5-fluordeoxyuridine, 5-fluorouracil, gemcitabine, gramicidin D, hydroxyurea, idarubicin, ifosfamide, irinotecan, lomustine (CCNU), maytansine, mechlorethamine, melphalan, 6-mercaptopurine, methotrexate, mithramycin, mitomycin C, mitoxantrone, nitroimidazole, paclitaxel, palytoxin, plicamycin, procarbizine, rhizoxin, streptozotocin, tenoposide, 6-thioguanine, thioTEPA, topotecan, vinblastine, vincristine, vinorelbine, VP-16 and VM-26.

Cytotoxic agents include, for example, DNA minor groove binders (e.g., enediynes and lexitropsins, a CBI compound; see also U.S. Pat. No. 6,130,237), duocarmycins, taxanes (e.g., paclitaxel and docetaxel), puromycins, vinca alkaloids, CC-1065, SN-38, topotecan, morpholino-doxorubicin, rhizoxin, cyanomorpholino-doxorubicin, echinomycin, combretastatin, netropsin, epothilone A and B, estramustine, cryptophysins, cemadotin, maytansinoids, discodermolide, eleutherobin, and mitoxantrone.

In some examples, the Drug is an anti-tubulin agent. Examples of anti-tubulin agents include, but are not limited to, auristatins, taxanes (e.g., Taxol.RTM. (paclitaxel), Taxotere® (docetaxel)), T67 (Tularik) and vinca alkyloids (e.g., vincristine, vinblastine, vindesine, and vinorelbine). Other antitubulin agents include, for example, baccatin derivatives, taxane analogs (e.g., epothilone A and B), nocodazole, colchicine and colcimid, estramustine, cryptophycins, cemadotin, maytansinoids, combretastatins, discodermolide, and eleutherobin.

In certain embodiments, the cytotoxic agent is a maytansinoid, another group of anti-tubulin agents. For example, in specific embodiments, the maytansinoid is maytansine or DM-1 (ImmunoGen, Inc.; see also Chari et al., 1992, Cancer Res. 52:127-131).

In some embodiments, the Drug is an auristatin, such as auristatin E (also known in the art as a derivative of dolastatin-10) or a derivative thereof. Typically, the auristatin E derivative is, e.g., an ester formed between auristatin E and a keto acid. For example, auristatin E can be reacted with paraacetyl benzoic acid or benzoylvaleric acid to produce AEB and AEVB, respectively. Other typical auristatin derivatives include AFP, MMAF, and MMAE. The synthesis and structure of auristatin derivatives are described in U.S. Patent Application Publication Nos. 2003-0083263, 2005-0238649 and 2005-0009751; International Patent Publication No. WO 04/010957, International Patent Publication No. WO 02/088172, and U.S. Pat. Nos. 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414.

Auristatins have been shown to interfere with microtubule dynamics and nuclear and cellular division and have anticancer activity. Auristatins as described herein bind tubulin and can exert a cytotoxic or cytostatic effect on a cell. There are a number of different assays, known in the art, that can be used for determining whether an auristatin or resultant binding molecule-drug conjugate exerts a cytostatic or cytotoxic effect on a desired cell line, see e.g., Example 4.

Methods for determining whether a compound binds tubulin are known in the art. See, for example, Muller et al., Anal. Chem. 2006, 78, 4390-4397; Hamel et al., Molecular Pharmacology, 1995 47: 965-976; and Hamel et al., The Journal of Biological Chemistry, 1990 265:28, 17141-17149.The relative affinity of a compound to tubulin can be determined. Some preferred auristatins bind tubulin with an affinity ranging from 10 fold lower (weaker affinity) than the binding affinity of MMAE to tubulin to 10 fold, 20 fold or even 100 fold higher (higher affinity) than the binding affinity of MMAE to tublin. or the derivatized drug, is subsequently reacted with the binding molecule under appropriate conditions.

Each of the particular units of the binding molecule-drug conjugates is described in more detail herein. The synthesis and structure of exemplary linker units, stretcher units, amino acid units, self-immolative spacer unit, and drug units are also described in U.S. Patent Application Publication Nos. 2003-0083263, 2005-0238649 and 2005-0009751.

In certain examples, the Drug is an antimetabolite. The antimetabolite can be, for example, a purine antagonist (e.g., azothioprine or mycophenolate mofetil), a dihydrofolate reductase inhibitor (e.g., methotrexate), acyclovir, gangcyclovir, zidovudine, vidarabine, ribavarin, azidothymidine, cytidine arabinoside, amantadine, dideoxyuridine, iododeoxyuridine, poscarnet, or trifluridine.

In other examples, the Drug is tacrolimus, cyclosporine or rapamycin. In further examples, the Drug is aldesleukin, alemtuzumab, alitretinoin, allopurinol, altretamine, amifostine, anastrozole, arsenic trioxide, bexarotene, bexarotene, calusterone, capecitabine, celecoxib, cladribine, Darbepoetin alfa, Denileukin diftitox, dexrazoxane, dromostanolone propionate, epirubicin, Epoetin alfa, estramustine, exemestane, Filgrastim, floxuridine, fludarabine, fulvestrant, gemcitabine, gemtuzumab ozogamicin, goserelin, idarubicin, ifosfamide, imatinib mesylate, Interferon alfa-2a, irinotecan, letrozole, leucovorin, levamisole, meclorethamine or nitrogen mustard, megestrol, mesna, methotrexate, methoxsalen, mitomycin C, mitotane, nandrolone phenpropionate, oprelvekin, oxaliplatin, pamidronate, pegademase, pegaspargase, pegfilgrastim, pentostatin, pipobroman, plicamycin, porfimer sodium, procarbazine, quinacrine, rasburicase, Rituximab, Sargramostim, streptozocin, tamoxifen, temozolomide, teniposide, testolactone, thioguanine, toremifene, Tositumomab, Trastuzumab, tretinoin, uracil mustard, valrubicin, vinblastine, vincristine, vinorelbine and zoledronate.

In some examples, the Drug moiety is an immunomodulatory agent. The immunomodulatory agent can be, for example, gancyclovir, etanercept, tacrolimus, cyclosporine, rapamycin, cyclophosphamide, azathioprine, mycophenolate mofetil or methotrexate. Alternatively, the immunomodulatory agent can be, for example, a glucocorticoid (e.g., cortisol or aldosterone) or a glucocorticoid analogue (e.g., prednisone or dexamethasone).

In some examples, the immunomodulatory agent is an anti-inflammatory agent, such as arylcarboxylic derivatives, pyrazole-containing derivatives, oxicam derivatives and nicotinic acid derivatives. Classes of anti-inflammatory agents include, for example, cyclooxygenase inhibitors, 5-lipoxygenase inhibitors, and leukotriene receptor antagonists.

Suitable cyclooxygenase inhibitors include meclofenamic acid, mefenamic acid, carprofen, diclofenac, diflunisal, fenbufen, fenoprofen, ibuprofen, indomethacin, ketoprofen, nabumetone, naproxen, sulindac, tenoxicam, tolmetin, and acetylsalicylic acid.

Suitable lipoxygenase inhibitors include redox inhibitors (e.g., catechol butane derivatives, nordihydroguaiaretic acid (NDGA), masoprocol, phenidone, Ianopalen, indazolinones, naphazatrom, benzofuranol, alkylhydroxylamine), and non-redox inhibitors (e.g., hydroxythiazoles, methoxyalkylthiazoles, benzopyrans and derivatives thereof, methoxytetrahydropyran, boswellic acids and acetylated derivatives of boswellic acids, and quinolinemethoxyphenylacetic acids substituted with cycloalkyl radicals), and precursors of redox inhibitors.

Other suitable lipoxygenase inhibitors include antioxidants (e.g., phenols, propyl gallate, flavonoids and/or naturally occurring substrates containing flavonoids, hydroxylated derivatives of the flavones, flavonol, dihydroquercetin, luteolin, galangin, orobol, derivatives of chalcone, 4,2',4'-trihydroxychalcone, ortho-aminophenols, N-hydroxyureas, benzofuranols, ebselen and species that increase the activity of the reducing selenoenzymes), iron chelating agents (e.g., hydroxamic acids and derivatives thereof, N-hydroxyureas, 2-benzyl-1-naphthol, catechols, hydroxylamines, carnosol trolox C, catechol, naphthol, sulfasalazine, zyleuton, 5-hydroxyanthranilic acid and 4-(omega-arylalkyl)phenylalkanoic acids), imidazole-containing compounds (e.g., ketoconazole and itraconazole), phenothiazines, and benzopyran derivatives.

Yet other suitable lipoxygenase inhibitors include inhibitors of eicosanoids (e.g., octadecatetraenoic, eicosatetraenoic, docosapentaenoic, eicosahexaenoic and docosahexaenoic acids and esters thereof, PGE1 (prostaglandin E1), PGA2 (prostaglandin A2), viprostol, 15-monohydroxyeicosatetraenoic, 15-monohydroxy-eicosatrienoic and 15-monohydroxyeicosapentaenoic acids, and leukotrienes B5, C5 and D5), compounds interfering with calcium flows, phenothiazines, diphenylbutylamines, verapamil, fuscoside, curcumin, chlorogenic acid, caffeic acid, 5,8,11,14-eicosatetrayenoic acid (ETYA), hydroxyphenylretinamide, Ionapalen, esculin, diethylcarbamazine, phenantroline, baicalein, proxicromil, thioethers, diallyl sulfide and di-(1-propenyl) sulfide.

Leukotriene receptor antagonists include calcitriol, ontazolast, Bayer Bay-x-1005, Ciba-Geigy CGS-25019C, ebselen, Leo Denmark ETH-615, Lilly LY-293111, Ono ONO-4057, Terumo TMK-688, Boehringer Ingleheim BI-RM-270, Lilly LY 213024, Lilly LY 264086, Lilly LY 292728, Ono ONO LB457, Pfizer 105696, Perdue Frederick PF 10042, Rhone-Poulenc Rorer RP 66153, SmithKline Beecham SB-201146, SmithKline Beecham SB-201993, SmithKline Beecham SB-209247, Searle SC-53228, Sumitamo SM 15178, American Home Products WAY 121006, Bayer Bay-o-8276, Warner-Lambert CI-987, Warner-Lambert CI-987BPC-15LY 223982, Lilly LY 233569, Lilly LY-255283, MacroNex MNX-160, Merck and Co. MK-591, Merck and Co. MK-886, Ono ONO-LB-448, Purdue Frederick PF-5901, Rhone-Poulenc Rorer RG 14893, Rhone-Poulenc Rorer RP 66364, Rhone-Poulenc Rorer RP 69698, Shionoogi S-2474, Searle SC-41930, Searle SC-50505, Searle SC-51146, Searle SC-52798, SmithKline Beecham SK&F-104493, Leo Denmark SR-2566, Tanabe T-757 and Teijin TEI-1338.

In certain examples, the cytotoxic or cytostatic agent is a dolastatin. In certain embodiments, the cytotoxic or cytostatic agent is of the auristatin class. Thus, in a specific embodiment, the cytotoxic or cytostatic agent is monomethyl auristatin E (MMAE).

Methods of determining whether a Drug or binding molecule-drug conjugate exerts a cytostatic and/or cytotoxic effect on a cell are known. Generally, the cytotoxic or cytostatic activity of a binding molecule-drug conjugate can be measured by: exposing mammalian cells expressing a target protein of the binding molecule-drug conjugate in a cell culture medium; culturing the cells for a period from about 6 hours to about 5 days; and measuring cell viability. Cell-based in vitro assays can be used to measure viability (proliferation), cytotoxicity, and induction of apoptosis (caspase activation) of the conjugate.

For determining whether a binding molecule-drug conjugate exerts a cytostatic effect, a thymidine incorporation assay may be used. For example, cancer cells expressing a target antigen at a density of 5,000 cells/well of a 96-well plated can be cultured for a 72-hour period and exposed to 0.5 µCi of ³H-thymidine during the final 8 hours of the 72-hour period. The incorporation of ³H-thymidine into cells of the culture is measured in the presence and absence of the binding molecule-drug conjugate.

For determining cytotoxicity, necrosis or apoptosis (programmed cell death) can be measured. Necrosis is typically accompanied by increased permeability of the plasma membrane; swelling of the cell, and rupture of the plasma membrane. Apoptosis is typically characterized by membrane blebbing, condensation of cytoplasm, and the activation of endogenous endonucleases. Determination of any of these effects on cancer cells indicates that a binding molecule-drug conjugate is useful in the treatment of cancers.

Cell viability can be measured by determining in a cell the uptake of a dye such as neutral red, trypan blue, or ALAMAR™ blue (see, e.g., Page et al., 1993, Intl. J. Oncology 3:473-476). In such an assay, the cells are incubated in media containing the dye, the cells are washed, and the remaining dye, reflecting cellular uptake of the dye, is measured spectrophotometrically. The protein-binding dye sulforhodamine B (SRB) can also be used to measure cytoxicity (Skehan et al., 1990, J. Natl. Cancer Inst. 82:1107-12).

Alternatively, a tetrazolium salt, such as MTT, is used in a quantitative colorimetric assay for mammalian cell survival and proliferation by detecting living, but not dead, cells (see, e.g., Mosmann, 1983, J. Immunol. Methods 65:55-63).

Apoptosis can be quantitated by measuring, for example, DNA fragmentation. Commercial photometric methods for the quantitative in vitro determination of DNA fragmentation are available. Examples of such assays, including TUNEL (which detects incorporation of labeled nucleotides in fragmented DNA) and ELISA-based assays, are described in Biochemica, 1999, no. 2, pp. 34-37 (Roche Molecular Biochemicals).

Apoptosis can also be determined by measuring morphological changes in a cell. For example, as with necrosis, loss of plasma membrane integrity can be determined by measuring uptake of certain dyes (e.g., a fluorescent dye such as, for example, acridine orange or ethidium bromide). A method for measuring apoptotic cell number has been described by Duke and Cohen, Current Protocols in Immunology (Coligan et al. eds., 1992, pp. 3.17.1-3.17.16). Cells also can be labeled with a DNA dye (e.g., acridine orange, ethidium bromide, or propidium iodide) and the cells observed for chromatin condensation and margination along the inner nuclear membrane. Other morphological changes that can be measured to determine apoptosis include, e.g., cytoplasmic condensation, increased membrane blebbing, and cellular shrinkage.

The presence of apoptotic cells can be measured in both the attached and "floating" compartments of the cultures. For example, both compartments can be collected by removing the supernatant, trypsinizing the attached cells, combining the preparations following a centrifugation wash step (e.g., 10 minutes at 2000 rpm), and detecting apoptosis (e.g., by measuring DNA fragmentation). (See, e.g., Piazza et al., 1995, Cancer Research 55:3110-16).

The effects of binding molecule-drug conjugates can be tested or validated in animal models. A number of established animal models of cancers are known to the skilled artisan, any of which can be used to assay the efficacy of a conjugate. Non-limiting examples of such models are described infra. Moreover, small animal models to examine the in vivo efficacies of binding molecule-drug conjugates can be created by implanting human tumor cell lines into appropriate immunodeficient rodent strains, e.g., athymic nude mice or SCID mice.

### Surrogate Light Chain (SLC) Unit

The surrogate light chain (SLC) unit has at least one functional group that can form a bond with a functional group of a Linker unit. Useful functional groups that can be present on an SLC unit, either naturally, via chemical manipulation or via engineering, include, but are not limited to, sulfhydryl (--SH), amino, hydroxyl, carboxy, the anomeric hydroxyl group of a carbohydrate, and carboxyl. In some embodiments, an SLC unit functional group is a sulfhydryl group. The sulfhydryl group is typically a solvent accessible sulfhydryl group, such as a solvent accessible sulfhydryl group on a cysteine residue. Sulfhydryl groups can be generated by reduction of an intramolecular or intermolecular disulfide bond of an SLC. Sulfhydryl groups also can be generated by reaction of an amino group of a lysine moiety of an SLC using 2-iminothiolane (Traut's reagent) or another sulfhydryl generating reagent.

In some embodiments, one or more sulfhydryl groups are engineered into an SLC unit, such as by amino acid substitution. For example, a sulfhydryl group can be introduced into an SLC unit. In some embodiments, a sulfhydryl group is introduced by an amino acid substitution of serine or threonine to a cysteine residue, and/or by addition of a cysteine residue into an SLC unit (an engineered cysteine residue). In some embodiments, the cysteine residue is an internal cysteine residue, i.e., not located at the N-terminus or C-terminus of the SLC.

In an exemplary embodiment, a cysteine residue can be engineered into a surrogate light chain (SLC) or fragment thereof by amino acid substitution or insertion.

To control the number of Drug or Linker unit-Drug units attached to an SLC unit, one or more cysteine residues can be eliminated by amino acid substitution. For example, the number of solvent accessible cysteine residues can be reduced by amino acid substitution of cysteine to serine residues.

In some embodiments, an SLC unit contains 1, 2, 3, 4, 5, 6 7 or 8 solvent-accessible cysteine residues. In some embodiments, an SLC unit contains 2 or 4 solvent-accessible cysteine residues.

### Amino acid residues for drug conjugation

In one aspect, one or more amino acid residues of the Surrobody™ polypeptides are selected for drug conjugation. In one embodiment, the amino acid residue is selected from the group consisting of native residues, non-native residues, naturally occuring residues, and non-naturally occuring residues. In another embodiment, the native residue is cysteine or lysine. In other embodiments, the non-native residue or naturally occuring residue is cysteine or lysine. In one other embodiment, the non-naturally occuring residue is selected from the group consisting of a para-acetyl-phenylalanine, an O-methyl-L-tyrosine, an L-3-(2-naphthyl)alanine, a 3-methyl-phenylalanine, an O-4-alkyl-L-tyrosine, a 4-propyl-L-tyrosine, a tri-O-acetyl-GlcNAcβ-serine, an L-Dopa, a fluorinated phenylalanine, an isopropyl-L-phenylalanine, a p-azido-L-phenylalanine, a p-acyl-L-phenylalanine, a p-benzoyl-L-phenylalanine, an L-phosphoserine, a phosphonoserine, a phosphonotyrosine, a p-iodo-phenylalanine, a p-bromophenylalanine, a p-amino-L-phenylalanine, and an isopropyl-L-phenylalanine, an O-methyl-L-tyrosine, an L-3-(2-naphthyl)alanine, and an amino-, isopropyl-, or O-alkyl-containing phenylalanine analogue. Non-naturally occuring or encoded amino acids are further described in U.S. Published Patent Application Nos. 20080227205 and 20100093082.

In one aspect, thiol residues in a Surrobody™ can be introduced by a number of methods known in the art. In one embodiment, the methods include the following: a) modification of the Surrobody™ with thiol-generating reagents such as 2-iminothiolane or homocysteine thiolactone, or b) via reaction with a disulfide-containing heterobifunctional crosslinking agent such as SMCC, SPP, SPDP, SPDB, sulfo-SPDB followed by reduction of the disulfide bond with DTT or TCEP to generate a free thiol, c) mutagenesis to incorporate non-native cysteine residues, such as cysteine-engineered antibodies (US2007/0092940 A1, US 2010/0003766 A1, U.S. Pat. No. 7,723,485 B2), or d) reduction of native disulfide bonds (del Rosario, R. B. et al., Cancer Res. Suppl. 1990, 50, 804s-808s).

In one other aspect, the amino group of a lysine residue may be used for drug conjugation to a Surrobody™.

In another aspect, the Surrobodies™ described herein comprise non-native cysteine residues introduced via mutagenesis. In one embodiment, the surrogate light chain (SLC) comprises one or more non-native cysteine residues. In another embodiment, the one or more non-native cysteine residues are located in an amino acid sequence selected from the group consisting of SEQ ID NO: 1 (VpreB1); SEQ ID NO: 4 (VpreB3-like sequence); SEQ ID NO: 5 (truncated VpreB1 sequence); SEQ ID NO:6 (VpreB1 with a murine Ig κ leader sequence); SEQ ID NO: 7 (murine λ5 sequence); SEQ ID NO: 8 (human λ5 sequence); SEQ ID NO:9 (truncated λ5 sequence); SEQ ID NO: 10 (human λ5 dTail sequence with a murine Ig κ leader sequence; SEQ ID NO: 35 (human VpreB1-λ5 chimeric amino acid sequence); SEQ ID NO: 36 (human VpreB1-λ5 chimeric amino acid sequence), and any combination thereof. In another embodiment, the one or more non-native cysteine residues are located in a human Vκ-like amino acid sequence selected from the group consisting of SEQ ID NO:12; SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24. In one other embodiment, the one or more non-native cysteine residues are located in a human human JCκ amino acid sequence selected from the group consisting of SEQ ID NO:26; SEQ ID NO: 27, SEQ ID NO: 28, SEQ ID NO: 29, SEQ ID NO: 30, SEQ ID NO: 31, SEQ ID NO: 32, SEQ ID NO: 33, and SEQ ID NO: 34.

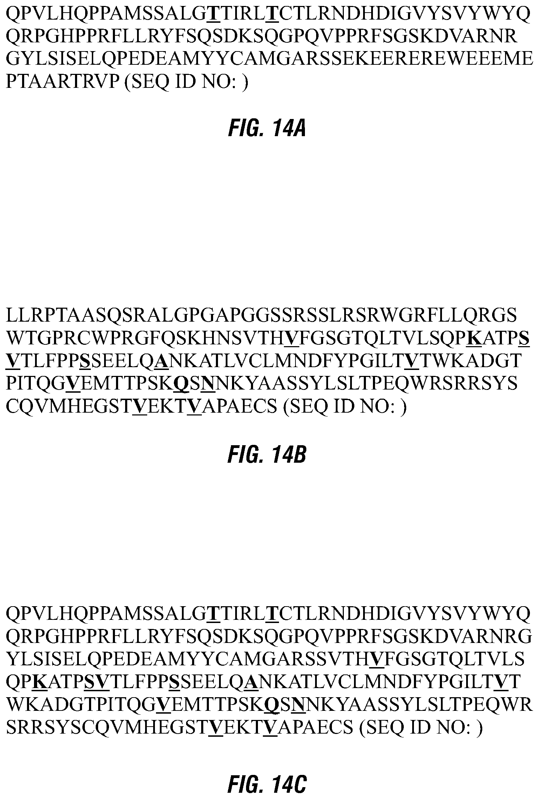

Figure 14A provides the amino acid sequence of human VpreB1 (SEQ ID NO: 54) without its leader sequence *i.e.*, a mature VpreB1 sequence. Certain amino acid positions that are suitable for the introduction of a cysteine residue are underlined and bolded. In one embodiment, the non-native cysteine residue is introduced at one or more positions selected from the group consisting of T16 and T21 (sequential numbering).

Figure 14B provides the amino acid sequence of human λ5 (SEQ ID NO: 55) without its leader sequence, *i.e.*, a mature λ5 sequence. Certain amino acid positions that are suitable for the introduction of a cysteine residue are underlined and bolded. In one embodiment, the non-native cysteine residue is introduced at one or more positions selected from the group consisting of V60, K74, S78, V79, S85, A91, V110, V123, Q131, N133, V166, and V170 (sequential numbering).

Figure 14C provides the amino acid sequence of a VpreB1-λ5 fusion (SEQ ID NO: 56) without either leader sequence. Certain amino acid positions that are suitable for the introduction of a cysteine residue are underlined and bolded. In one embodiment, the cysteine residue is introduced at one or more positions selected from the group consisting of T16 and T21, V107, K121, S125, V126, S132, A138, V157, V170, Q178, N180, V213, and V217 (sequential numbering).

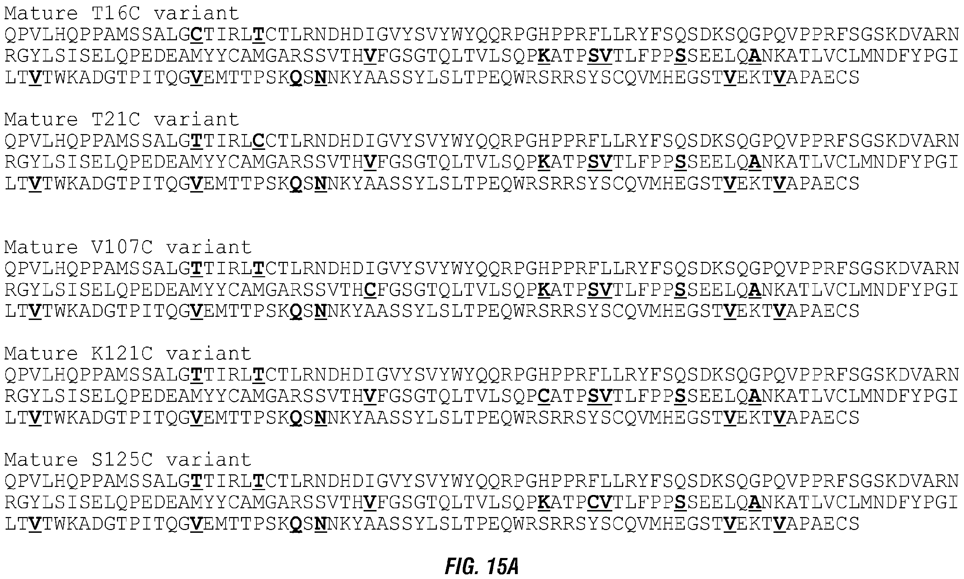

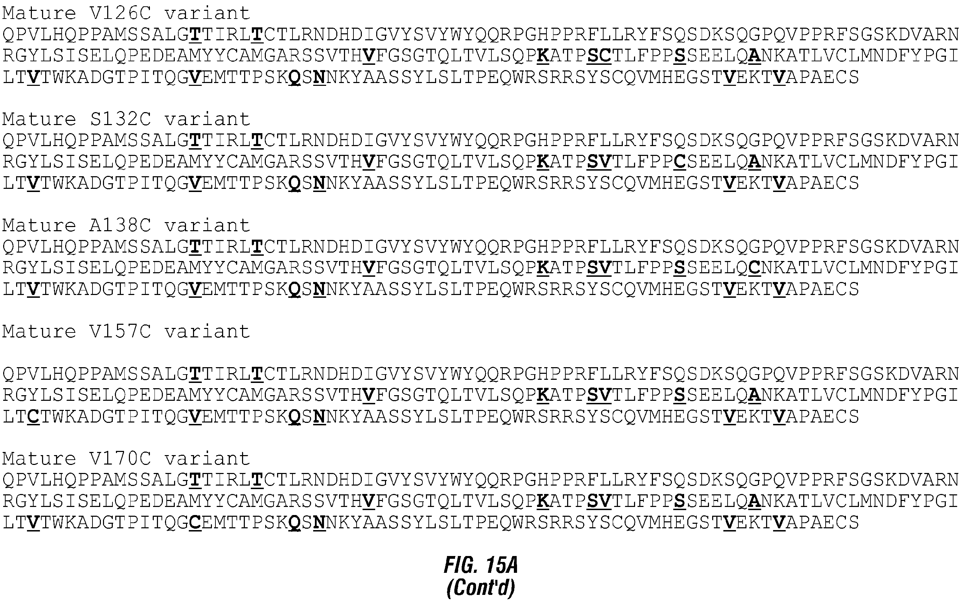

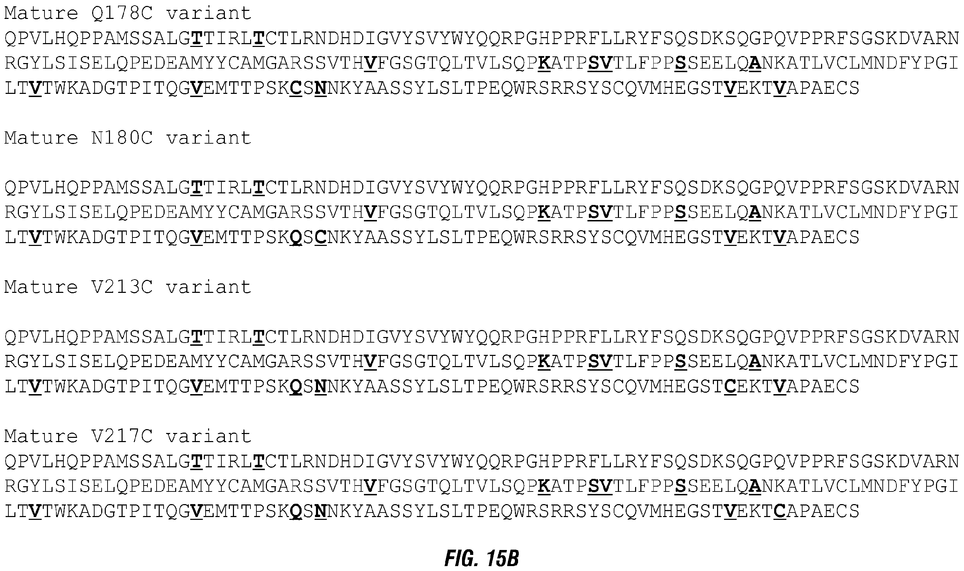

Figure 15A-B provides the amino acid sequences for various surrogate light chains with an introduced cysteine residue (SEQ ID NOS: 57-70, respectively, in order of appearance).

### Therapeutic and diagnostic agents

The binding molecule conjugates of the present invention include therapeutic agents selected from the group consisting of a maytansinoid, a monomethyl auristatin E (MME) and a monomethyl auristatin F (MMAF).

Further exemplary agents refer to a chemical compound, a mixture of chemical compounds, a biological macromolecule, or an extract made from biological materials. Therapeutic agents will be any of a wide variety of drugs, including, but not limited to, enzyme inhibitors, hormones, cytokines, growth factors, receptor ligands, antibodies, antigens, ion binding compounds including crown ethers and other chelators, substantially complementary nucleic acids, nucleic acid binding proteins including transcription factors, toxins, etc. Suitable drugs include cytokines such as erythropoietin (EPO), thrombopoietin (TPO), the interleukins (including IL-1 through IL-17), insulin, insulin-like growth factors (including IGF-1 and -2), epidermal growth factor (EGF), transforming growth factors (including TGF-α and TGF-β), human growth hormone, transferrin, epidermal growth factor (EGF), low density lipoprotein, high density lipoprotein, leptin, VEGF, PDGF, ciliary neurotrophic factor, prolactin, adrenocorticotropic hormone (ACTH), calcitonin, human chorionic gonadotropin, cotrisol, estradiol, follicle stimulating hormone (FSH), thyroid-stimulating hormone (TSH), leutinzing hormone (LH), progeterone, testosterone, toxins including ricin, and any drugs as outlined in the Physician's Desk Reference, Medical Economics Data Production Company, Montvale, N.J., 1998 and the Merck Index, 11th Edition (especially pages Ther-1 to Ther-29.

The therapeutic agent is a drug used to treat cancer. Examples of cancer drugs include, but are not limited to, antineoplastic drugs, including alkylating agents such as alkyl sulfonates (busulfan, improsulfan, piposulfan); aziridines (benzodepa, carboquone, meturedepa, uredepa); ethylenimines and methylmelamines (altretamine, triethylenemelamine, triethylenephosphoramide, triethylenethiophosphoramide, trimethylolmelamine); nitrogen mustards (chlorambucil, chlornaphazine, cyclophosphamide, estramustine, ifosfamide, mechlorethamine, mechlorethamine oxide hydrochloride, melphalan, novembichin, phenesterine, prednimustine, trofosfamide, uracil mustard); nitrosoureas (carmustine, chlorozotocin, fotenmustine, lomustine, nimustine, ranimustine); dacarbazine, mannomustine, mitobranitol, mitolactol; pipobroman; doxorubicin, carboplatin, oxaliplatin, and cisplatin, (including derivatives).

In one example, the therapeutic agent is a cytotoxic agent which includes, without limitation, pertussis toxin, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D,1-dehydrotestosterone, glucocorticoids, procaine, tetracaine, lidocaine, propranolol, and puromycin and analogs or homologs thereof.

In some examples, the therapeutic agent is an antiviral or antibacterial drug, including aclacinomycins, actinomycin, anthramycin, azaserine, bleomycins, cuctinomycin, carubicin, carzinophilin, chromomycins, ductinomycin, daunorubicin, 6-diazo-5-oxn-I-norieucine, duxorubicin, epirubicin, mitomycins, mycophenolic acid, nogalumycin, olivomycins, peplomycin, plicamycin, porfiromycin, puromycin, streptonigrin, streptozocin, tubercidin, ubenimex, zinostatin, zorubicin; aminoglycosides and polyene and macrolide antibiotics.

As will be appreciated by those in the art, any number of suitable drugs such as those found in the Physician's Desk Reference can be used.

Diagnostic agents, which are nor part of the invention, will generally include a detectable label. A "label" or "detectable label" refers to a moiety attached to a binding molecule described herein, e.g., to render the reaction between members of a specific binding pair, such as a binding molecule and an analyte, detectable. The binding molecule so labeled is referred to as "detectably labeled." A binding molecule conjugated to a diagnostic agent refers to a binding molecule with a label incorporated that provides for the identification of the binding molecule. The label is a detectable marker that can produce a signal that is detectable by visual or instrumental means, e.g., incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). Examples of labels for polypeptides include, but are not limited to, the following: radioisotopes or radionuclides (e.g., ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm); chromogens, fluorescent labels (e.g., FITC, rhodamine, lanthanide phosphors), enzymatic labels (e.g., horseradish peroxidase, luciferase, alkaline phosphatase); chemiluminescent markers; biotinyl groups; predetermined polypeptide epitopes recognized by a secondary reporter (e.g., leucine zipper pair sequences, binding sites for secondary antibodies, metal binding domains, epitope tags); and magnetic agents, such as gadolinium chelates. Representative examples of labels commonly employed for immunoassays include moieties that produce light, e.g., acridinium compounds, and moieties that produce fluorescence, e.g., fluorescein. Other labels are described herein. In this regard, the moiety itself may not be detectably labeled but may become detectable upon reaction with yet another moiety. Use of "detectably labeled" is intended to encompass the latter type of detectable labeling.

### Preparation of surrogate light chain constructs

The present application discloses methods of preparing the Surrobodies or surrrogate light chain constructs that may be used to produce the binding molecule conjugates. Nucleic acids encoding the surrogate light chain constructs, e.g. VpreB and λ5 polypeptides or VK-like or JCK polypeptides, can be isolated from natural sources, e.g. developing B cells and/or obtained by synthetic or semi-synthetic methods. Once this DNA has been identified and isolated or otherwise produced, it can be ligated into a replicable vector for further cloning or for expression.

Cloning and expression vectors that can be used for expressing the coding sequences of the polypeptides herein are well known in the art and are commercially available. The vector components generally include, but are not limited to, one or more of the following: a signal sequence, an origin of replication, one or more marker genes, an enhancer element, a promoter, and a transcription termination sequence. Suitable host cells for cloning or expressing the DNA encoding the surrogate light chain constructs in the vectors herein are prokaryote, yeast, or higher eukaryote (mammalian) cells, mammalian cells are being preferred.

Examples of suitable mammalian host cell lines include, without limitation, monkey kidney CV1 line transformed bySV40 (COS-7, ATCC CRL 1651); human embryonic kidney (HEK) line 293 (HEK 293 cells) subcloned for growth in suspension culture, Graham et al, J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and Wa human hepatoma line (Hep G2).

For use in mammalian cells, the control functions on the expression vectors are often provided by viral material. Thus, commonly used promoters can be derived from the genomes of polyoma, Adenovirus2, retroviruses, cytomegalovirus, and Simian Virus 40 (SV40). Other promoters, such as the β-actin protomer, originate from heterologous sources. Examples of suitable promoters include, without limitation, the early and late promoters of SV40 virus (Fiers et al., Nature, 273: 113 (1978)), the immediate early promoter of the human cytomegalovirus (Greenaway et al., Gene, 18: 355-360 (1982)), and promoter and/or control sequences normally associated with the desired gene sequence, provided such control sequences are compatible with the host cell system.

Transcription of a DNA encoding a desired heterologous polypeptide by higher eukaryotes is increased by inserting an enhancer sequence into the vector. The enhancer is a cis-acting element of DNA, usually about from 10 to 300 bp, that acts on a promoter to enhance its transcription-initiation activity. Enhancers are relatively orientation and position independent, but preferably are located upstream of the promoter sequence present in the expression vector. The enhancer might originate from the same source as the promoter, such as, for example, from a eukaryotic cell virus, e.g. the SV40 enhancer on the late side of the replication origin (bp 100-270), the cytomegalovirus early promoter enhancer, the polyoma enhancer on the late side of the replication origin, and adenovirus enhancers.

Expression vectors used in mammalian host cells also contain polyadenylation sites, such as those derived from viruses such as, e.g., the SV40 (early and late) or HBV.

An origin of replication may be provided either by construction of the vector to include an exogenous origin, such as may be derived from SV40 or other viral (e.g., Polyoma, Adeno, VSV, BPV) source, or may be provided by the host cell.

The expression vectors usually contain a selectable marker that encodes a protein necessary for the survival or growth of a host cell transformed with the vector. Examples of suitable selectable markers for mammalian cells include dihydrofolate reductase (DHFR), thymidine kinase (TK), and neomycin.

Suitable mammalian expression vectors are well known in the art and commercially available. Thus, for example, the surrogate light chain constructs of the present invention can be produced in mammalian host cells using a pCI expression vector (Promega), carrying the human cytomegalovirus (CMV) immediate-early enhancer/promoter region to promote constitutive expression of a DNA insert. The vector may also be the pTT5 expression vector (National Research Council, Canada). The vector can contain a neomycin phosphotransferase gene as a selectable marker.

The surrogate light chain constructs of the present invention can also be produced in bacterial host cells. Control elements for use in bacterial systems include promoters, optionally containing operator sequences, and ribosome binding sites. Suitable promoters include, without limitation, galactose (gal), lactose (lac), maltose, tryptophan (trp), β-lactamase promoters, bacteriophage λ and T7 promoters. In addition, synthetic promoters can be used, such as the tac promoter. Promoters for use in bacterial systems also generally contain a Shine-Dalgarno (SD) sequence operably linked to the DNA encoding the Fab molecule. The origin of replication from the plasmid pBR322 is suitable for most Gram-negative bacteria.

The coding sequences of the individual chains within a multi-chain construct comprising antibody surrogate light chain sequences can be present in the same expression vector, under control of separate regulatory sequences, or in separate expression vectors, used to co-transfect a desired host cells, including eukaryotic and prokaryotic hosts. Thus, multiple genes can be coexpressed using the Duet™ vectors commercially available from Novagen.

The transformed host cells may be cultured in a variety of media. Commercially available media for culturing mammalian host cells include Ham's F10 (Sigma), Minimal Essential Medium ((MEM), (Sigma), RPMI-1640 (Sigma), and Dulbecco's Modified Eagle's Medium ((DMEM), Sigma). In addition, any of the media described in Ham et al., Meth. Enz. 58:44 (1979) and Barnes et al., Anal. Biochem. 102:255 (1980) may be used as culture media for the host cells. The culture conditions, such as temperature, pH, and the like, are those previously used with the host cell selected for expression, and are included in the manufacturer's instructions or will otherwise be apparent to the ordinarily skilled artisan.

Further suitable media for culturing mammalian, bacterial (e.g. *E. coli*) or other host cells are also described in standard textbooks, such as, for example, Sambrook et al., *supra,* or Ausubel et al., *supra.*

### Heterologous leader sequences

The present application discloses heterologous leader sequences that improve the efficiency of recombinant expression of the surrogate light chain (SLC) polypeptides that may be used to form the binding molecule conjugates described herein. In one aspect, the present application discloses isolated nucleic acid molecules encoding a surrogate light chain (SLC) polypeptide or SLC construct containing an SLC polypeptide, wherein the native secretory leader sequence of the polypeptide is replaced by a heterologous secretory leader sequence. In one embodiment, the SLC polypeptide includes a VpreB polypeptide, a λ5 polypeptide, or fragments or variants thereof. In another embodiment, the VpreB polypeptide is selected from the group consisting of a native VpreB 1 sequence, a native VpreB2 sequence, a native VpreB3 sequence, and fragments and variants thereof. In some embodiments, the native VpreB sequence is selected from the group consisting of human VpreB 1 of SEQ ID NO: 1, mouse VpreB2 of SEQ ID NOS: 2 and 3, human VpreB3 of SEQ ID NO: 4, human VpreB-like polypeptide of SEQ ID NO:5, human VpreB dTail polypeptide of SEQ ID NO:6 and fragments and variants thereof. In one other embodiment, the λ5 polypeptide is selected from the group consisting of a murine λ5 of SEQ ID NO: 7; a human λ5 polypeptide of SEQ ID NO: 8, a human λ5 dTail polypeptide of SEQ ID NO:9, and fragments and variants thereof. In another embodiment, the SLC polypeptide includes a Vκ -like polypeptide, a JCκ polypeptide, or fragments or variants thereof. In one other embodiment, the Vκ -like polypeptide sequence is selected from the group consisting of SEQ ID NOS: 12-24, and fragments and variants thereof. In some embodiments, the JCκ polypeptide sequence is selected from the group consisting of SEQ ID NOS:26-39, and fragments and variants thereof.

In another aspect, the present application discloses isolated nucleic acid molecules encoding a surrogate light chain (SLC) polypeptide, wherein the native secretory leader sequence of the polypeptide is replaced by a heterologous secretory leader sequence and the SLC polypeptide includes an SLC polypeptide fusion, or fragments or variants thereof. In one embodiment, the SLC fusion includes a VpreB-λ5 polypeptide fusion, or fragments or variants thereof. In another embodiment, the fusion of the VpreB polypeptide sequence and λ5 polypeptide sequence takes place at or around the CDR3 analogous regions of the VpreB sequence and the λ5 sequence respectively. In one other embodiment, the VpreB polypeptide sequence is linked at its carboxy terminus to the amino terminus of the λ5 polypeptide sequence. In one embodiment, the SLC fusion includes a Vκ-like-JCκ polypeptide fusion, or fragments or variants thereof. In another embodiment, the fusion of the Vκ-like polypeptide sequence and JCκ polypeptide sequence takes place at or around the CDR3 analogous regions of the Vκ-like sequence and the JCκ sequence respectively. In one other embodiment, the Vκ-like polypeptide sequence is fused at its carboxy terminus to the amino terminus of the JCκ polypeptide sequence.

In all embodiments, the heterologous secretory leader sequence may be a leader sequence of a secreted polypeptide selected from the group consisting of antibodies, cytokines, lymphokines, monokines, chemokines, polypeptide hormones, digestive enzymes, and components of the extracellular matrix. In one embodiment, the cytokine may be selected from the group consisting of growth hormone, such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β (TNF-α and -β); mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1a, IL-2, IL-3, IL-4, IL-5, IL-6, IL-7, IL-8, IL-9, IL-10, IL-11, IL-12; a tumor necrosis factor such as TNF-α or TNF-β; MIP-1α; MIP-1β; and other polypeptide factors including LIF and kit ligand (KL).

In all embodiments, the secretory leader sequence may be selected from the group consisting of leader sequences of human and non-human mammalian albumin, transferrin, CD36, growth hormone, tissue plasminogen activator (t-PA), erythropoietin (EPO), and neublastin.

In all embodiments, the secretory leader sequence may be a synthetic sequence.

In all embodiments, the secretory leader sequence may be a consensus sequence of native secretory leader sequences.

The murine Ig kappa leader sequence may be used (METDTLLLWVLLLWVPGSTG - SEQ ID NO: 53) as a heterologous leader sequence.In all embodiments, the present application discloses an isolated nucleic acid molecule encoding a surrogate light chain (SLC) construct.

In one aspect, the application discloses vectors and recombinant host cells. In all embodiments, the vectors may contain a nucleic acid molecule described herein. In all embodiments, the recombinant host cells may be transformed with a nucleic acid described herein.

In another aspect, the present application discloses methods for the expression of a surrogate light chain (SLC) polypeptide or SLC construct in a recombinant host cell. In one embodiment, the method includes the step of transforming the recombinant host cell with a nucleic acid molecule encoding an SLC polypeptide or SLC construct, wherein the native secretory leader sequence of the polypeptide is replaced by a heterologous secretory leader sequence. In another embodiment, the recombinant host cell is an eukaryotic cell. In one other embodiment, the recombinant host cell is a Chinese Hamster Ovary (CHO) cell or a human embryonic kidney (HEK) 293 cell. In some embodiments, the SLC polypeptide or SLC construct is selected from the group consisting of an SLC polypeptide comprising one or more of a VpreB polypeptide, a λ5 polypeptide, a VpreB-λ5 polypeptide fusion, a Vκ -like polypeptide, a JCκ polypeptide, and a Vκ-like-JCκ polypeptide fusion.

The present application discloses nucleic acid and polypeptide constructs for producing surrogate light chain constructs in higher yields than when such constructs are produced from sequences that comprise an endogenous leader VpreB leader sequence and/or λ5 leader sequence, or an endogenous Vκ-like leader sequence and/or JCκ leader sequence. The present application discloses vectors, host cells and methods for producing surrogate light chain constructs in higher yields than when such constructs are produced from DNA sequences that include the coding sequence of the endogenous leader of VpreB and/or λ5, or the endogenous leader of Vκ-like and/or JCκ, or without an endogenous leader sequence. The higher yields are achieved by replacing at least one endogenous secretory leader sequence with a heterologous leader sequence as described herein. Accordingly, the present application discloses surrogate light chains and surrogate light chain constructs comprising heterologous leader sequences.

Preferably, the expression level achieved by a heterologous leader peptide is at least about 5% higher, at least about 10% higher, at least about 20% higher, at least about 30% higher, at least about 40% higher, or at least about 50% higher than the expression level achieved by using a homologous leader sequence, when expression is conducted under essentially the same conditions.

In the present application, a heterologous leader sequence is fused to the amino terminus of a surrogate light chain polypeptide, in place of the native VpreB leader sequence and/or the native λ5 leader sequence, or a κ-like surrogate light chain polypeptide, in place of the native Vκ-like leader sequence and/or the native JCκ leader sequence. The inventors have discovered that certain heterologous leader sequences function surprisingly well, in contrast to the native leader sequence of the surrogate light chain during the production of surrogate light chain constructs, comprising a surrogate light chain sequence (VpreB/λ5 or Vκ-like/JCκ sequences either fused together or non-covalently associated) and an antibody heavy chain sequence.

The heterologous leader sequence can be any leader sequence from a highly translated protein, including leader sequences of antibody light chains and human and non-human mammalian secreted proteins. Secreted proteins are included and their sequences are available from public databases, such as Swiss-Prot, UniProt, TrEMBL, RefSeq, Ensembl and CBI-Gene. In addition, SPD, a web based secreted protein database is a resource for such sequences, available at http://spd.cbi.pku.edu.cn. (See, Chen et al., Nucleic Acids Res., 2005, 33:D169-D173). Such secreted proteins include, without limitation, antibodies, cytokines, lymphokines, monokines, chemokines, polypeptide hormones, digestive enzymes, and components of the extracellular matrix. Further leader sequences suitable for use in the constructs described herein are included in publicly available signal peptide databases, such as, the SPdb signal peptide database, accessible at http://proline.bis.nus.edu.sq/spdb (See, Choo et al., BMC Bioinformatics 2005, 6:249).

Specific examples of suitable heterologous leader sequences include, without limitation, leader sequences of human and non-human mammalian albumin, transferrin, CD36, growth hormone, tissue plasminogen activator (t-PA), erythropoietin (EPO), neublastin leader sequences and leader peptides from other secreted human and non-human proteins.

When heterologous leader sequences are present in i) both a VpreB and a λ5 surrogate light chain construct, or ii) both a Vκ-like and a JCκ surrogate light chain construct, each heterologous leader sequence in i) or ii) may be identical to the other or may be different from the other.

In addition to signal peptides from native proteins, the heterologous leader sequences described herein include synthetic and consensus leader sequences, which can be designed to further improve the performance of leader sequences occurring in nature, and specifically adapted for best performance in the host organism used for the expression of the surrogate light chain constructs of the present invention.

In one aspect, the present application discloses a method for the expression of a surrogate light chain in a recombinant host cell. In one embodiment, the method includes the step of providing a nucleic acid encoding an SLC polypeptide or an SLC fusion polypeptide. In another embodiment, the method includes the step of transforming or transfecting the recombinant host cell with a nucleic acid encoding an SLC polypeptide or SLC fusion polypeptide. In one embodiment, the nucleic acid encoding an SLC fusion polypeptide is a chimeric molecule comprising a first SLC sequence covalently connected to a second SLC sequence, wherein the native secretory leader sequence of the first SLC sequence and/or the second SLC sequence is replaced by a heterologous secretory leader sequence. The first SLC sequence may be a VpreB sequence, a Vκ-like sequence, or a fusion polypeptide thereof. The second SLC sequence may be a λ5 sequence, a JCκ sequence, or a fusion polypeptide thereof. Heterologous leader sequences are further described in WO/2010/151808 published on December 29, 2010.

In one embodiment, a VpreB sequence is covalently connected to a λ5 sequence, wherein the native secretory leader sequence of said VpreB sequence and/or said λ5 sequence is replaced by a heterologous secretory leader sequence. In another embodiment, the VpreB sequence is fused to the λ5 sequence. In one other embodiment, the VpreB sequence is connected to the λ5 sequence through a peptide or polypeptide linker. In one other embodiment, a Vκ-like sequence is covalently connected to a JCκ sequence, wherein the native secretory leader sequence of said Vκ-like sequence and/or said JCκ sequence is replaced by a heterologous secretory leader sequence. In one other embodiment, the Vκ-like sequence is fused to the JCκ sequence. In another embodiment, the Vκ-like sequence is connected to the JCκ sequence through a peptide or polypeptide linker.

In other embodiments, the SLC sequence is covalently connected to an antibody heavy chain sequence.

In all embodiments, the methods of expression may comprise the step of transforming or transfecting a host cell with more than one nucleic acid encoding a surrogate light chain polypeptide, including surrogate light chain polypeptides and/or surrogate light chain fusion polypeptides.

In all embodiments, the methods may further comprise the step of transforming or transfecting a host cell with a nucleic acid encoding an antibody heavy chain.

In one aspect, the present application discloses methods for the expression of surrogate light chain polypeptides and/or surrogate light chain fusion polypeptides having improved yields. The methods disclosed herein utilizing heterologous leader sequences in place of native leader sequences are characterized greater polypeptide expression and yield than methods which do not replace native leader sequences with heterologous leader sequences.

Exemplarily, the recombinant host cell is bacterial cell. Exemplarily, the host cell is a eukaryotic cell. Exemplarily, the recombinant host cell is a Chinese Hamster Ovary (CHO) cell, or a human embryonic kidney (HEK) 293 cell.

In one aspect, the present application discloses host cells containing the nucleic acids described hereinExemplarily, the application discloses a recombinant host cell transformed with at least one nucleic acid described herein. The host cell can be transformed with a nucleic acid encoding an SLC fusion, which may or may not include a non-SLC molecule.

In all embodiments, the host cell is further transformed with a nucleic acid encoding an antibody heavy chain.

The present application discloses vectors that contain the nucleic acids described herein. The host cell can be transformed with at least one vector containing a nucleic acid described herein.

Purification can be performed by methods known in the art. In a preferred embodiment, the surrogate light chain constructs are purified in a 6xHis-tagged (SEQ ID NO: 71) form, using the Ni-NTA purification system (Invitrogen).

κ-like SLC molecules can be engineered from existing light chain V genes and light chain constant genes. Light chains are products of gene rearrangement and RNA processing. As the components of the κ-like SLC molecules provide alternative function from unrearranged light chain V genes and rearranged light chain JC genes, it is feasible to engineer similar translated proteins from all remaining kappa and lambda light chain V genes to make Vκ -like molecules and all combinations of the remaining kappa JC rearrangements (4 JCκ -like) and lambda JC rearrangements (4 "J" x 10 "constant" = 40 JCλ-like). Each one of these engineered molecules can serve purposes similar to those using Vκ-like and JCκ, as well as those contained in PCT Publication WO 2008/118970 published on October 2, 2008 and WO/2010/151808 published on December 29, 2010, with VpreB and λ5, and combinations and chimeras thereof.

The Surrobodies of the present invention can be conjugated to an agent by methods known to those of ordinary skill in the art.

The surrogate light chains of the present invention can be used to construct molecules for the prevention and/or treatment of disease. For such applications, molecules containing a surrogate light chain are usually used in the form of pharmaceutical compositions. Techniques and formulations generally may be found in Remington's Pharmaceutical Sciences, 18th Edition, Mack Publishing Co. (Easton, Pa. 1990). See also, Wang and Hanson "Parenteral Formulations of Proteins and Peptides: Stability and Stabilizers," Journal of Parenteral Science and Technology, Technical Report No. 10, Supp. 42-2S (1988).

Polypeptide-based pharmaceutical compositions are typically formulated in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes {e.g., Zn-protein complexes); and/or non-ionic surfactants such as TWEEN™, PLURONICS™ or polyethylene glycol (PEG).

The molecules also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, *supra.*

The molecules containing surrogate light chains disclosed herein may also be formulated as immunoliposomes. Liposomes containing the molecules are prepared by methods known in the art, such as described in Epstein et al, Proc. Natl. Acad. Sci. USA 82:3688 (1985); Hwang et al, Proc. Natl Acad. Sci. USA 77:4030 (1980); U.S. Patent Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG- derivatized phosphatidyl ethanolamine (PEG-PE). Liposomes are extruded through filters of defined pore size to yield liposomes with the desired diameter. Fragments of the molecules of the present invention can be conjugated to the liposomes via a disulfide interchange reaction (Martin et al. J. Biol. Chem. 257:286-288 (1982). A chemotherapeutic agent is optionally contained within the liposome. See Gabizon et al. J. National Cancer Inst. 81(19)1484 (1989).

For the prevention or treatment of disease, the appropriate dosage of molecule will depend on the type of infection to be treated the severity and course of the disease, and whether the antibody is administered for preventive or therapeutic purposes. The molecule is suitably administered to the patient at one time or over a series of treatments. Depending on the type and severity of the disease, about 1 µg/kg to about 15 mg/kg of antibody is a typical initial candidate dosage for administration to the patient, whether, for example, by one or more separate administrations, or by continuous infusion.

Molecules containing a surrogate light chain of the present invention are suitable for use in the treatment or prevention of diseases. In one embodiment, the present invention provides a surrogate light chain-containing molecule for use as a medicament, or for the treatment of a disease. In another embodiment, the present application discloses the use of a surrogate light chain-containing molecule for the manufacture of a medicament for treating disease. The molecule may be a nucleic acid encoding an SLC polypeptide or SLC fusion.

In one aspect, the application discloses methods useful for treating a disease in a mammal, the methods including the step of administering a therapeutically effective amount of a surrogate light chain-containing molecule to the mammal. The therapeutic compositions can be administered short term (acute) or chronic, or intermittent as directed by physician.

### SLC polypeptide containing libraries

In one aspect, the present application discloses a library of binding molecules which comprise a surrogate light chain (SLC) polypeptide. The library can be used to identify binding molecules suitable for conjugation to an agent. In one embodiment, the binding molecules comprise an SLC polypeptide conjugated to an antibody light chain polypeptide. In another embodiment, the binding molecule comprises a λ5 sequence conjugated to an antibody variable light chain sequence. In one other embodiment, the λ5 sequence-antibody variable light chain sequence is a fusion.

### Constant regions with improved stability

In another aspect, the present invention provides binding molecules with improved stability. In one embodiment, the binding molecule conjugates described herein are based upon a binding molecule comprising an SLC polypeptide and an antibody heavy chain constant region, wherein the binding molecule has improved stability based upon certain heavy chain constant region variants. In another embodiment, the binding molecule is a Surrobody, which has a heteromultimer format. In one other embodiment, the heteromultimer comprises at least one antibody heavy chain and at least one SLC. In some embodiments, the antibody heavy chain comprises a variant constant region. In other embodiments, the variant constant region is a variant CH3 and/or a variant CH2 region. In one additional embodiment, the variant constant region promotes formation of the heteromultimer with increased stability as compared to a non-variant constant region.

In another embodiment, the binding molecule or Surrobody comprises a first CH3 variant region comprising amino acid modification at positions F405 and Y407 and a second CH3 variant region comprising amino acid modification at position T394. In one embodiment, one of said first and second CH3 domain polypeptide further comprises amino acid modification of position Q347 and the other CH3 domain polypeptide comprises amino acid modification at position K360. In one other embodiment, at least one CH3 domain polypeptide further comprises amino acid modification of at least one of N390 and S400. In other embodiments, one of said first and second CH3 domain polypeptide further comprises amino acid modification of T350V. In some embodiments, the first CH3 domain polypeptide further comprises amino acid modification at position L351. In one other embodiment, the second CH3 domain polypeptide further comprises modification of at least one of positions T366 and K392.

In one embodiment, one of said first and second CH3 domain polypeptide further comprises amino acid modification of D399R or D399K and the other CH3 domain polypeptide comprises one or more of T411E, T411D, K409E, K409D, K392E and K392D. In another embodiment, one of said first and second CH3 domain polypeptide further comprises amino acid modification of T350V. In an additional embodiment, the first CH3 domain polypeptide comprises one or more amino acid modifications selected from L351Y, Y405A and Y407V, and the second CH3 domain polypeptide comprises one or more amino acid modifications selected from T366L, T3661, K392L, K392M and T394W.

In an additional embodiment, the first CH3 domain polypeptide comprises one or more amino acid modifications selected from L351Y, Y405A and Y407V, and the second CH3 domain polypeptide comprises one or more amino acid modifications selected from T366L, T3661, K392L, K392M and T394W. In one other embodiment, one of said first and second CH3 domain polypeptide further comprises amino acid modification of T350V.

In one embodiment, a first CH3 domain polypeptide comprises amino acid modifications at positions D399 and Y407 and a second CH3 domain polypeptide comprises amino acid modification at positions K409 and T411. In another embodiment, one of said first and second CH3 domain polypeptide further comprises amino acid modification of T350V. In other embodiments, the first CH3 domain polypeptide further comprises amino acid modification at position L351 and the second CH3 domain polypeptide further comprises amino acid modifications at position T366 and K392. In an additional embodiment, the first CH3 domain polypeptide further comprises amino acid modification at position S400 and the second CH3 domain polypeptide further comprises amino acid modification at position N390. In another embodiment, the first CH3 domain polypeptide comprises amino acid modifications selected from L351Y, D399R, D399K, S400K, S400R, Y407A, Y4071 and Y407V; and said second CH3 domain polypeptide comprises amino acid modifications selected from T366V, T366I, T366L, T366M, N390D, N390E, K392L, K3921, K392D, K392E, K409F, K409W, T411D and T411E. In one other embodiment, the first CH3 domain polypeptide comprises amino acid modifications selected from L351Y, D399R, D399K, Y407A, Y4071 and Y407V; and said second CH3 domain polypeptide comprises amino acid modifications selected from T366V, T366I, T366L, T366M, K392L, K392I, K392D, K392E, K409F, K409W, T411D and T411E. In one embodiment, one of said first and second CH3 domain polypeptide further comprises amino acid modification of T350V.

Additional disclosure related to variant constant regions may be found in U.S. 20120149876.

### Kits

The application also dicloses kits and articles of manufacture containing materials useful for the treatment, prevention and/or diagnosis of disease. The kit includes a container and a label, which can be located on the container or associated with the container. The container may be a bottle, vial, syringe, or any other suitable container, and may be formed from various materials, such as glass or plastic. The container holds a composition having a surrogate light chain-containing molecule as described herein, and may have a sterile access port. Examples of containers include an intravenous solution bag or a vial with a stopper that can be pierced by a hypodermic injection needle. The kits may have additional containers that hold various reagents, e.g., diluents and buffers. The label may provide a description of the composition as well as instructions for the intended use. Kits containing the molecules find use, e.g., for cellular assays, for purification or immunoprecipitation of a polypeptide from cells. For example, for isolation and purification of a protein, the kit can contain a surrogate light chain-containing molecule that binds the protein coupled to beads (e.g., sepharose beads). Kits can be provided which contain the molecules for detection and quantitation of the protein in vitro, e.g., in an ELISA or a Western blot. Such molecules useful for detection may be provided with a label such as a fluorescent or radiolabel.

The kit has at least one container that includes a molecule comprising a surrogate light chain described herein as the active agent. A label may be provided indicating that the composition may be used to treat a disease. The label may also provide instructions for administration to a subject in need of treatment. The kit may further contain an additional container having a pharmaceutically-acceptable buffer, such as bacteriostatic water for injection (BWFI), phosphate-buffered saline, Ringer's solution and dextrose solution. Finally, the kit may also contain any other suitable materials, including other buffers, diluents, filters, needles, and syringes.

Further details of the invention are provided in the following non-limiting examples.

### Examples

### Example 1. Surrogate Light Chain Protein Conjugates for enhanced therapeutic utility

Because many anti-cancer drugs target the cell cycle and kill rapidly proliferating cells, they do not discriminate between healthy and tumorous tissue. This lack of discrimination results in narrow therapeutic indices and causes severe treatment-related side effects that limit efficacy, because concentrations of drug that would significantly impact tumor growth are intolerable. Conjugating such potent molecules to specific antibodies has proven successful in delivering these potent molecules to the tumor site via the selective nature of the antibodies, at levels that effectively counter tumor growth and importantly limit exposure to noncancerous tissues/cells.

Linking such anti-cancer drugs to antibodies is achieved by several types of compatible amino acid side chain chemistry, typically through lysine amines and cysteine sulfhydryls. Aside from selecting appropriate cytotoxic agents and linkers, the challenge for all approaches remains at least two-fold. The first challenge is due to the fact that each new targeting antibody can be vastly different from the previous, with respect to the nature of their heavy and light chains, and the resulting composition of naturally occurring side chain chemistries can vary substantially. The net result is that distinct conjugating efforts are expected for nearly every new antibody. Secondly, as the heavy chain is the most likely target of most conjugation chemistries and that it is predominantly responsible for the specificity and stability of antibodies, chemical derivatization of this chain is inherently more likely to have deleterious effects on the specificity and stability of the antibody.

The ability to avoid conjugation of the heavy chain is highly desirable; furthermore the ability to target invariant sites on the heavy chain partner would be ideal. The surrogate light chain of the Surrobody™ provides such an ideal opportunity. As the surrogate light chain is non-diverse the composition of opportunistic naturally occurring side chains would remain unchanged from one surrobody to the next. As a result, it would be possible to direct conjugate chemistries exclusively to the surrogate light chain and utilize a single derivatization strategy for each subsequent new Surrobody™.

### Example 2. Derivatizing naturally occurring and specifically introduced surrogate light chain amino acid side chains

Because opportunistic amino acid side chain chemistries, namely lysine-derived amines and cysteine-derived sulfhydryls, are likely associated with elemental structural needs, introduction of new sites and elimination of naturally occurring sites may be desired. For specific introduction of reactive sites to the external face of VpreB, away from the target proximal site could be highly desirable for conjugation. The selection of such sites could be inferred by structural analysis of known pre B cell receptor structures, or empirically determined by systematic substitution of cysteines and/or lysines along the length of Vpre B sequence of the surrogate light chain construct. Similarly, it would be possible to assess naturally occurring, or systematically introduced, reactive sites on the lambda-5 derived portion of surrogate light chain constructs.

### Example 3. Specific conjugation via Surrobodies containing unnatural amino acids

It may be desirable to specifically incorporate a drug conjugate at a particular site within the surrogate light chain. It is possible that the position could be precluded from using a naturally occurring amino acid side chain chemistry because of incompatibilities from a structural or specificity standpoint. In this case it may be possible to incorporate an unnatural amino acid containing unique chemical properties that are distinctly different from all other amino acid chemistries such that specific chemical derivatization of the unnatural amino acid (or amino acids) would be possible. Depending upon the final disposition of the Surrobody one could select from numerous technologies that exist to incorporate unnatural amino acids through cell-free protein synthesis, or reprogrammed in vivo systems in e. coli, yeast, and mammalian cell lines.

### Example 4. Protein toxin surrobody conjugates with improved therapeutic utility

Numerous protein toxins exist that exert antiproliferative effects, but have narrow to nonexistent therapeutic indices. However conjugation to antibodies has proven useful in limiting the toxin's effects to the site directed by the antibodies specificity. Again, even though this process has been successful with antibodies, the ability to place the toxin in a fixed position with a heavy chain partner would be ideal. Again, the invariant nature of the surrogate light chain can be exploited for such a purpose. Toxins such as Pseudomonas Exotoxin A (PEA) could be recombinantly fused to either the VpreB or lambda-5 portions, or termini with in a surrogate light chain construct. By analogy, diphtheria toxin, ricin, and other cellular toxins could be candidates for surrogate light chain fusion. In any case surrogate light chain/toxin fusions could be generated by recombinant methods, or by chemical conjugation of toxin to Surrobody. In the case of chemical conjugation, derivatization of surrobodies would utilize opportunistic naturally occurring amino acid side chain chemistries, or rely upon recombinantly incorporated sites, or combinations of either or both.

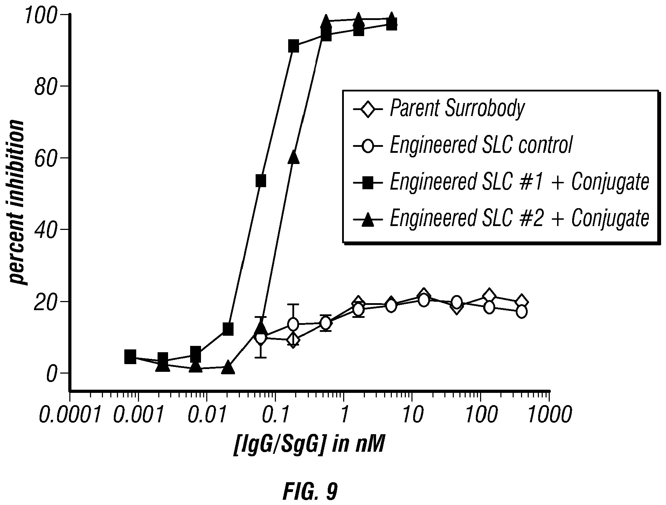

Surrobody-drug conjugates were observed to provide potent inhibition of cellular proliferation (*in vitro*)*.* The following were tested for percent inhibition: (a) a parent anti-ErbB2 Surrobody; (b) an anti-ErbB2 Surrobody using an engineered surrogate light chain (SLC) #1 (V213C); (c) an anti-ErbB2 engineered SLC#1(V213C) + MMAE conjugate; and (d) an engineered SLC#2 (T21C) + MMAE conjugate (Figure 9 and Table 4.1). Briefly, these molecules were tested for their ability, in vitro, to inhibit growth of SKBR3 cells. The assay involved plating 5,000 cells per well in a 96-well culture dish and 3-6 hours after plating the cells treating them with control surrobodies and drug conjugated surrobodies. After 6 days of treatment the cell viability was assessed by Cell titer Glo (Promega) assay and the results plotted and analyzed using Prism (Graphpad) software. Overall, the results indicate that it is possible to modify an SLC to increase a cytotoxic payload and increase both the potency and efficacy of these antiproliferative surrobodies. Specifically,the SLC + conjugates were observed to perform as well as, or better than an antibody-drug conjugate (data not shown).

**Table 4.1**

| | Potency [pM] | Efficacy |
|---|---|---|
| Parent | 0.615 | 20% |
| Engineered SLC#1 (V213C) construct | 0.382 | 19% |
| Engineered SLC#1 (V213C) + Conjugate | 0.058 | 96% |
| Engineered SLC#2 (V213C) + Conjugate | 0.156 | 99% |

Several Surrobody-drug conjugates (bivalent, bispecific, and monovalent) were analyzed for potency and for the ability to inhibit cell proliferation of cell lines with high receptor expression (Figure 11A-B and Table 4.2).

All of the constructs in Table 4.2 contain a surrogate light chain with a V213C mutation. The R1 (or EGFR) bivalent conjugate is a Surrobody-conjugate that binds EGFR and is conjugated to MMAE via an MC-vc-PAB linker. The R2/R1 (or ErbB2/EGFR) bispecific construct is a Surrobody-conjugate that binds to both ErbB2 and EGFR and is conjugated to MMAE via an MC-vc-PAB linker. The R1 (or EGFR) monovalent conjugate is a Surrobody-conjugate having one functionally null moiety and a monovalent EGFR binding moiety, which is conjugated to MMAE via an MC-vc-PAB linker. The R2 (or ErbB2) monovalent conjugate is a Surrobody-conjugate having one functionally null moiety and a monovalent ErbB2 binding moiety, which is conjugated to MMAE via an MC-vc-PAB linker.

Briefly, these molecules were tested for their ability, in vitro, to inhibit growth of A431 cells. The assay involved plating 7,500 cells per well in a 96-well culture dish and 3-6 hours after plating the cells treating them with control surrobodies and drug conjugated surrobodies. After 4 days of treatment the cell viability was assessed by Cell titer Glo (Promega) assay and the results plotted and analyzed using Prism (Graphpad) software. Figure 11A depicts the following conjugate constructs: R1 bivalent (2 identical SLCs and heavy chains bivalent for R1, or EGFR); R2/R1 bispecific (SLC + R2 or ErbB2 heavy chain and SLC + R1 or EGFR heavy chain); R1 or EGFR monovalent (SLC + heavy chain for R1 or EGFR and null); and R2 monovalent (SLC + heavy chain for R2 or ErbB2 and null). Figure 11B shows the percent inhibition of proliferation. Overall, the results indicate that it is possible to modify an SLC to increase a cytotoxic payload and increase both the potency and efficacy of monospecific and bispecific surrobodies. Specifically,the bispecfic SLC + conjugates were observed to perform better than either monovalent monospecific an antibody-drug conjugate and possibly provide a greater index of killing compared to single specificities that may predominate undesirable targeting to healthy tissue compared to tumor tissue, which may only be achieved by bispecific targeting compared to monospecific targeting that may be further improved by affinity tuning bispecifics to bind significantly better when both targets are present compared to when either or both are reduced or absent on healthy tissue.

**Table 4.2**

| | Potency [pM] |
|---|---|
| R1 bivalent conjugate | 0.067 |
| R2/R1 bispecific conjugate | 0.704 |
| R1 monovalent conjugate | 3.595 |
| R2 monovalent conjugate | 202.6 |

### Example 5. Immunoconjugates with improved therapeutic utility

Immunostimulatory cytokines, such as IL-2 can enhance tumor cell killing, but have deleterious side effects when administered systemically. As such, it is desirable to conjugate IL-2 to surrogate light chains to enhance tumor cell killing, while restricting broad systemic availability. To create such a molecule one can recombinantly engineer a surrogate light chain/IL-2 fusion. We created such molecules where human IL-2 was cloned in frame with the N-terminus of Lambda-5, the C-terminus of VpreB, and also the N-terminus of a VpreB-Lambda-5 fusion construct. The resulting proteins were coexpressed with a heavy chain from a surrobody that specifically recognizes human HGF.

We specifically assessed the bioactivity of the IL-2 fusions. Briefly, the IL-2 bearing Surrobodies were tested for the bioactivity in an HT-2 proliferation assay and compared with control Surrobodies and recombinant human IL-2. Results in Figure 1 show that activity is comparable to a recombinant reference standard. As IL-2 is a rather large protein and the site for conjugated fusion could be proximal to surfaces facing the Heavy Chain CDRs we wished to see if the IL-2 proteins impeded target binding (HGF). As seen in Figure 2, the IL-2 bearing Surrobodies maintained excellent high affinity binding characteristics, indicating that no significant steric issues were created by any of the fusions.

### Example 6. Radiolabeled Surrobodies with improved utility

Targeting Surrobodies can be created such that diagnostic or therapeutic radioactive conjugates could be incorporated specifically into the surrogate light chain. One such method would be to utilize naturally occurring side chain chemistries, such as phenols on tyrosines to incorporate Iodine-based radionuclides. Alternatively, tyrosines could be systematically substituted throughout either the VpreB or lambda-5 domains of a surrogate light chain to incorporate an optimally positioned amount of radionuclide. Alternatively, other nuclides may be used and additional amenable sites derivatized. Also several chelate methods that utilize direct association, or bispecific components, fused or incorporated within either the VpreB or lambda-5 domains of a surrogate light chain could be used.

### Example 7. Targeted enzymatic prodrug activation

Prodrugs can be specifically activated by enzymes. As Surrobodies have been shown to maintain specific binding properties, even in the presence of significantly-sized protein fusions, we would take the opportunity to conjugate a prodrug activating enzyme to enables specific activation at the tumor targeted site. As an example, we would fuse an enzymatic prodrug activator such as beta-lactamase or carboxypeptidase G2 to the Surrogate light chain. The resulting Surrobody would be administered and next a prodrug capable of activation by the conjugated enzyme would be administered.

### Example 8. Surrobody targeted drug-loaded particles

Either drug-loaded liposomes or nanoparticles could be specifically derived through naturally occurring chemistries in the Surrogate Light Chain to enable stable association of targeted Surrobodies on their surface. In this instance one would conjugate bifunctional agents that incorporate into naturally occurring or engineered sites on the surrogate light chain and also specifically and stably associate with the drug-loaded liposomes or nanoparticles. Such drug-loaded complexes would then be administered to localized the liposome or nanoparticle cargo to the tumor site.

### Example 9. Bispecific and monospecific Surrobody-Drug conjugates

The following Surrobody-drug conjugates (bispecific and monospecific) were constructed and evaluated for the percentage of drug conjugated (Figure 10 and Table 9.1). The R3/null V213C is a monvalent Surrobody having one functionally null moiety, a monvalent ErbB3 binding moiety, and a surrogate light chain with a V213C mutation. The R2/null V213C is a monvalent Surrobody having one functionally null moiety, a monvalent ErbB2 binding moiety, and a surrogate light chain with a V213C mutation. The R1/null V213C is a monvalent Surrobody having one functionally null moiety, a monvalent EGFR binding moiety, and a surrogate light chain with a V213C mutation. The R2/R3 null V213C is a monvalent Surrobody having one functionally null moiety, a bispecific ErbB2/ErbB3 binding moiety (Stacked Variable Domain), and a surrogate light chain with a V213C mutation. The R1/R3 null V213C is a monvalent Surrobody having one functionally null moiety, a bispecific EGFR/ErbB3 binding moiety (Stacked Variable Domain), and a surrogate light chain with a V213C mutation. The R1/R2 null V213C is a monvalent Surrobody having one functionally null moiety, a bispecific EGFR/ErbB2 binding moiety (Stacked Variable Domain), and a surrogate light chain with a V213C mutation.

In general the described surrobodies were transiently produced in HEK293 cells and purified using Protein A chromatography. The resulting proteins were reduced with TCEP (tris(2-carboxyethyl)phosphine) to reveal unpaired cysteine thiols and then allowed to reform parental inter and intrachain disulfide bonds. Next the proteins were conjugated through maleimide chemistries with MMAE (Monomethyl auristatin) toxin and subsequently subjected to size exclusion chromatography to remove unreacted MMAE toxin. Finally, the resulting proteins were examined by reverse phase HPLC. Toxin conjugated entities were identified by their slower elution properties and their relative amounts quantitated by A280 absorption.

**Table 9.1**

| Bispecific | # toxins conjugated | % Conjugated |
|---|---|---|
| 1: R3/null V213C | zero to 2 | 97 |
| 2: R2/null V213C | zero to 2 | 92 |
| 3: R1/null V213C | zero to 2 | 95 |
| 4: R2/R3 null V213C | zero to 2 | 96 |
| 5: R1/R3 null V213C | zero to 2 | 94 |
| 6: R1/R2 null V213C | zero to 2 | 93 |
| 7: R3/null V213C | zero to 2 | 95 |
| 8: R1/null V213C | zero to 2 | 94 |
| 9: R2/null V213C | zero to 2 | 97 |
| 10: Herception/V205C | zero to 2 | 74 |

The results indicate that unoptimized reactions generate surrobodies that are more completely conjugated than a Herceptin™ control monoclonal antibody. The Surrobody-drug constructs described herein demonstrate drug conjugation that can exceed antibody efficiencies. In addition, this efficiency of conjugation has the potential to be beneficial for subsequent development steps.

### Example 10. Serum stability of Surrobody-drug conjugates

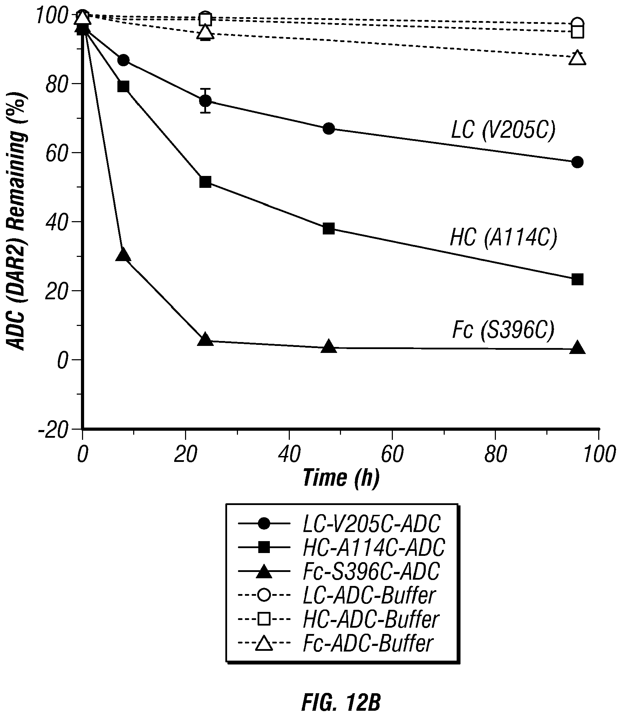

Surrobody-drug conjugates were observed for serum stability. In previous unoptimized conjugation reactions almost all the surrobodies are conjugated, with most carrying two cytotoxic molecules. Figure 12A depicts serum conjugate stability (biotin) for the following constructs: ErbB2 SLC (N180C), ErbB2 SLC (T21C), and ErbB2 SLC (V213C). Figure 12B depicts serum stability for various Herceptin™ conjugates (V205C, A114C, and S296C). Briefly specific proteins were transiently produced in HEK293 cells and purified using Protein A chromatography, as previously described. The resulting proteins were reduced with TCEP (tris(2-carboxyethyl)phosphine) to reveal unpaired cysteine thiols and then allowed to reform parental inter and intrachain disulfide bonds. Next the proteins were conjugated through maleimide chemistries with biotin and subsequently subjected to size exclusion chromatography to remove unreacted biotin. Next, the resulting purified biotin conjugated surrobodies were mixed with serum for various periods of time at 37 degrees centigrade. After incubation with serum the resulting surrobodies were captured in ErbB2 coated ELISA format and then detected with either anti-Fc antibodies or Streptavidin. With the differences between the relative strengths of these two methods of binding detection being reflective of site specific conjugate stability.

### Example 11. Analysis of aggregation of Surrobody-drug conjugates

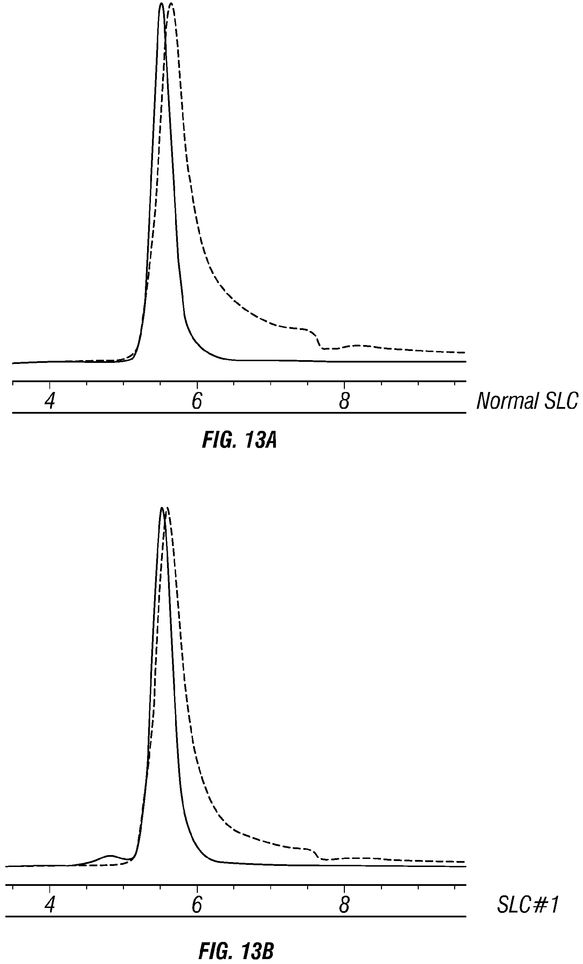

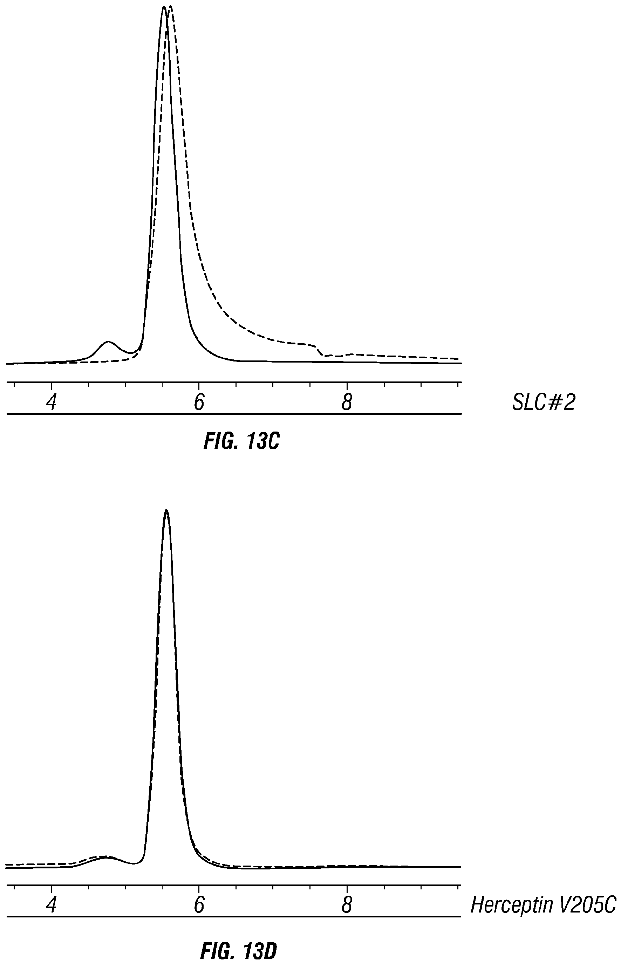

Surrobody constructs containing new site specific cysteines introduced into the surrogate light chain constucts were generated and following protein A purification the unpaired cysteine thiols were reduced and allowed to react in either an intermolecular or intramolecular mode under nonreducing conditions at high protein concentration. We then examined proteins having such free thiol exposures from newly engineered cysteines for aggregation following concentration (Table 11.1 and Figure 13A-B - dashed line = 0.5 mg/mL; solid line = -22 mg/mL). The SLC#1 "λ5" is a bivalent Surrobody-conjugate that binds ErbB2 and contains a surrogate light chain with a V213C mutation, which is conjugated to MMAE via an MC-vc-PAB linker. The SLC#2 "VpreB" is a bivalent Surrobody-conjugate that binds ErbB2 and contains a surrogate light chain with a T21C mutation, which is conjugated to MMAE via an MC-vc-PAB linker.

**Table 11.1**

| | Normal SLC | SLC #1 "λ5" - Cys | SLC #2 "VpreB" - Cys | Herceptin™ V205C |
|---|---|---|---|---|
| % Aggregate @ 0.5 mg/mL | 0.0 | 0.0 | 1.0 | 4.2 |
| % Aggregate @ 22 mg/mL | 1.2 | 2.8 | 8.3 | 5.2 |

Figure 13A-B depicts Normal SLC and SLC#1 ("λ5" - Cys). Figure 13C-D depicts SLC#2 ("VpreB" - Cys) and Herceptin™ V205C. Experiment approach used: Surrobodies were reduced, concentrated in spin columns, and examined by SEC. It was observed that following storage at >20mg/mL for 3 days at 4C, those with free thiols accumulated 10-13% aggregate. Dilution reduced it for Surrobodies to -5%, but not for Herceptin™.

Figure 13A-B depicts Normal SLC and SLC#1 ("λ5" - Cys). Figure 13C-D depicts SLC#2 ("VpreB" - Cys) and Herceptin™ V205C. Experiment analysis involved size exclusion chromatography analysis following storage at >20mg/mL for 3 days at 4°C. In this example, the resulting analyzed aggregate content for Surrobody conjugation was capable of being lower than that seen for Herceptin™.

## Claims

1. A binding molecule conjugate comprising a surrogate light chain (SLC) polypeptide having at least one agent linked to an amino acid residue of the SLC polypeptide at a predetermined amino acid position, wherein the amino acid residue is either:
(i) normally present in the SLC polypeptide at said amino acid position; or
(ii) introduced into the amino acid sequence of said SLC polypeptide at said amino acid position,
wherein the agent is a therapeutic agent and is selected from the group consisting of amaytansinoid, a monomethyl auristatin E (MMAE) and a monomethyl auristatin F (MMAF).

2. The binding molecule conjugate of claim 1, wherein the amino acid residue is located at:
(i) at least one of position 16 or 21 of a mature VpreB 1 sequence; and/or
(ii) at one or more positions selected from the group consisting of 60, 74, 78, 79, 85, 91, 110, 123, 131, 133, 166, and 170 of a mature λ5 sequence.

3. The binding molecule conjugate of claim 1 or claim 2, wherein the binding molecule conjugate binds at least one target or at least two targets.

4. The binding molecule conjugate of claim 1 or claim 2, wherein the amino acid residue is introduced into the amino acid sequence of the SLC polypeptide .

5. The binding molecule conjugate of claim 1 or claim 2, wherein the amino acid residue is introduced into the amino acid sequence of the SLC polypeptide by a substitution.

6. The binding molecule conjugate of claim 1 or claim 2, wherein the introduced amino acid is a cysteine or a lysine.

7. The binding molecule conjugate of claim 1 or claim 2, comprising a surrogate light chain (SLC) polypeptide having at least one amino acid sequence selected from the group consisting of:
SSALGCTIRLT (SEQ ID NO: 37),
TTIRLCCTLRN (SEQ ID NO: 38),
SSVTHCFGSGT (SEQ ID NO: 39),
VLSQPCATPSV (SEQ ID NO: 40),
PKATPCVTLFP (SEQ ID NO: 41),
KATPSCTLFPP (SEQ ID NO: 42),
TLFPPCSEELQ (SEQ ID NO: 43),
SEELQCNKATL (SEQ ID NO: 44),
PGILTCTWKAD (SEQ ID NO: 45),
PITQGCEMTTP (SEQ ID NO: 46),
TTPSKCSNNKY (SEQ ID NO 47),
PSKQSCNKYAA (SEQ ID NO: 48),
HEGSTCEKTVA (SEQ ID NO: 49), and
TCEKTCAPAEC (SEQ ID NO: 50).

8. The binding molecule conjugate of any one of claims 1-7, further comprising a non-cleavable linker or a cleavable linker.

9. The binding molecule conjugate of any one of claims 1-7 further comprising a non-cleavable linker comprising a maleimido-based moiety or a haloacetyl-based moiety.

10. The binding molecule conjugate of any one of claims 1-7, further comprising a cleavable linker selected from the group consisting of a peptidyl linker, a pH-sensitive linker, and a linker cleavable under reducing conditions.

11. The binding molecule conjugate of any one of claims 1-7, further comprising a cleavable linker comprising a dipeptide linker.

12. The binding molecule conjugate of any one of claims 1-7, further comprising a non-cleavable linker or a cleavable linker selected from the group consisting of: N-succinimidyl 4-(maleimidomethyl)cyclohexanecarboxylate (SMCC), N-succinimidyl-4-(N-maleimidomethyl)-cyclohexane-1-carboxy-(6-amidocaproate), (LC-SMCC), κ-maleimidoundecanoic acid N-succinimidyl ester (KMUA), γ-maleimidobutyric acid N-succinimidyl ester (GMBS), ε-maleimidocaproic acid N-hydroxysuccinimide ester (EMCS), m-maleimidobenzoyl-N-hydroxysuccinimide ester (MBS), N-(α-maleimidoacetoxy)-succinimide ester [AMAS], succinimidyl-6-(β-maleimidopropionamido)hexanoate (SMPH), N-succinimidyl 4-(p-maleimidophenyl)-butyrate (SMPB), N-(p-maleimidophenyl)isocyanate (PMPI), 6-maleimidocaproyl (MC), maleimidopropanoyl (MP), valine-citrulline (val-cit), alanine-phenylalanine (ala-phe), paminobenzyloxycarbonyl (PAB), N-Succinimidyl 4-(2-pyridylthio) pentanoate (SPP), maleimidocaproyl-L-valine-L-citrulline-p-aminobenzyl alcohol p-nitrophenylcarbonate (MC-val-cit-PAB), N-succinimidyl-4-(iodoacetyl)-aminobenzoate (SIAB), N-succinimidyl iodoacetate (SIA), N-succinimidyl bromoacetate (SBA), N-succinimidyl 3-(bromoacetamido)propionate (SBAP), N-succinimidyl-5-acetylthioacetate (SATA), N-succinimidyl-3-(2-pyridyldithio)propionate (SPDP), N-succinimidyl-3-(2-pyridyldithio)butyrate) (SPDB), and N-succinimidyl-oxycarbonyl-alpha-methyl-alpha-(2-pyridyl-dithio)toluene) (SMPT).

## Patentansprüche

1. Bindemolekülkonjugat, umfassend ein Surrogat-Leichtketten-Polypeptid (SLC-Polypeptid), das mindestens ein Mittel aufweist, das an einer vorbestimmten Aminosäureposition mit einem Aminosäurerest des SLC-Polypeptids verbunden ist, wobei der Aminosäurerest entweder wie folgt ist:
(i) normalerweise an der Aminosäureposition in dem SLC-Polypeptid vorhanden; oder
(ii) an der Aminosäureposition in die Aminosäuresequenz des SLC-Polypeptids eingeführt,
wobei das Mittel ein therapeutisches Mittel ist und ausgewählt ist aus der Gruppe bestehend aus Amaytansinoid, einem Monomethylauristatin E (MMAE) und einem Monomethylauristatin F (MMAF).

2. Bindemolekülkonjugat nach Anspruch 1, wobei sich der Aminosäurerest befindet an:
(i) mindestens einer von Position 16 oder 21 einer reifen VpreB1-Sequenz; und/oder
(ii) an einer oder mehreren Positionen ausgewählt aus der Gruppe bestehend aus 60, 74, 78, 79, 85, 91, 110, 123, 131, 133, 166 und 170 einer reifen λ5-Sequenz.

3. Bindemolekülkonjugat nach Anspruch 1 oder Anspruch 2, wobei das Bindemolekülkonjugat mindestens ein Ziel oder mindestens zwei Ziele bindet.

4. Bindemolekülkonjugat nach Anspruch 1 oder Anspruch 2, wobei der Aminosäurerest in die Aminosäuresequenz des SLC-Polypeptids eingeführt ist.

5. Bindemolekülkonjugat nach Anspruch 1 oder Anspruch 2, wobei der Aminosäurerest durch eine Substitution in die Aminosäuresequenz des SLC-Polypeptids eingeführt ist.

6. Bindemolekülkonjugat nach Anspruch 1 oder Anspruch 2, wobei die eingeführte Aminosäure ein Cystein oder ein Lysin ist.

7. Bindemolekülkonjugat nach Anspruch 1 oder Anspruch 2, umfassend ein Surrogat-Leichtketten-Polypeptid (SLC-Polypeptid), das mindestens eine Aminosäuresequenz aufweist, ausgewählt aus der Gruppe bestehend aus:
SSALGCTIRuT (SEQ ID NO: 37),
TTIRLCCTLRN (SEQ ID NO: 38),
SSVTHCFGSGT (SEQ ID NO: 39),
VLSQPCATPSV (SEQ ID NO: 40),
PKATPCVTLFP (SEQ ID NO: 41),
KATPSCTLFPP (SEQ ID NO: 42),
TLFPPCSEELQ (SEQ ID NO: 43),
SEELQCNKATL (SEQ ID NO: 44),
PGILTCTWKAD (SEQ ID NO: 45),
PITQGCEMTTP (SEQ ID NO: 46),
TTPSKCSNNKY (SEQ ID NO 47),
PSKQSCNKYAA (SEQ ID NO: 48),
HEGSTCEKTVA (SEQ ID NO: 49), und
TCEKTCAPAEC (SEQ ID NO: 50).

8. Bindemolekülkonjugat nach einem der Ansprüche 1-7, ferner umfassend einen nicht spaltbaren Linker oder einen spaltbaren Linker.

9. Bindemolekülkonjugat nach einem der Ansprüche 1-7, ferner umfassend einen nicht spaltbaren Linker, umfassend eine auf Maleimido basierende Gruppierung oder eine auf Haloacetyl basierende Gruppierung.

10. Bindemolekülkonjugat nach einem der Ansprüche 1-7, ferner umfassend einen spaltbaren Linker, ausgewählt aus der Gruppe bestehend aus einem Peptidyl-Linker, einem pH-sensitiven Linker und einem unter reduzierenden Bedingungen spaltbaren Linker.

11. Bindemolekülkonjugat nach einem der Ansprüche 1-7, ferner umfassend einen spaltbaren Linker, der einen Dipeptid-Linker umfasst.

12. Bindemolekülkonjugat nach einem der Ansprüche 1-7, ferner umfassend einen nicht spaltbaren Linker oder einen spaltbaren Linker, ausgewählt aus der Gruppe bestehend aus: Nsuccinimidyl 4-(Maleimidomethyl)cyclohexancarboxylat (SMCC), N-Succinimidyl-4-(N-maleimidomethyl)-cyclohexan-1-carboxy-(6-amidocaproat), (LC-SMCC), κ-Maleimidoundecansäure-N-succinimidylester (KMUA), γ-Maleimidobuttersäure-N-succinimidylester (GMBS), ε-Maleimidocapronsäure-N-hydroxysuccinimidester (EMCS), m-Maleimidobenzoyl-N-hydroxysuccinimidester (MBS), N-(α-Maleimidoacetoxy)-succinimidester [AMAS], Succinimidyl-6-(-β-maleimidopropionamido)hexanoat (SMPH), N-Succinimidyl-4-(p-maleimidophenyl)-butyrat (SMPB), N-(p-Maleimidophenyl)-isocyanat (PMPI), 6-Maleimidocaproyl (MC), Maleimidopropanoyl (MP), Valin-Citrullin (val-cit), Alanin-Phenylalanin (ala-phe), Paminobenzyloxycarbonyl (PAB), N-Succinimidyl-4-(2-pyridylthio)-pentanoat (SPP), Maleinimidocapronyl-L-Valin-L-citrullin-p-Aminobenzylalkohol-p-nitrophenylcarbonat (MC-val-cit-PAB), N-Succinimidyl-4-(iodacetyl)-aminobenzoat (SIAB), Nsuccinimidyliodacetat (SIA), N-Succinimidylbromacetat (SBA), N-Succinimidyl-3-(bromacetamido)propionat (SBAP), N-Succinimidyl-5-acetylthioacetat (SATA), N-Succinimidyl-3-(2-pyridyldithio)propionat (SPDP), N-Succinimidyl-3-(2-pyridyldithio)butyrat) (SPDB) und N-Succinimidyl-oxycarbonyl-alpha-methyl-alpha(2-pyridyl-dithio)toluol) (SMPT).

## Revendications

1. Un conjugué de molécule de liaison comprenant un polypeptide de chaine légère de substitution (SLC) ayant au moins un agent lié à un résidu d'acide aminé du polypeptide SLC dans une position d'acide aminé prédéterminée, dans lequel le résidu d'acide aminé est soit :
(i) normalement présent dans le polypeptide SLC dans ladite position d'acide aminé ;
(ii) introduit dans la séquence d'acide aminé dudit polypeptide SLC dans ladite position d'acide aminé.
Dans lequel l'agent est un agent thérapeutique sélectionné dans le groupe composé d'un maytansinoïde, d'une monométhylauristatine E (MMAE) et d'une monométhylauristatine F (MMAF).

2. Le conjugué de molécule de liaison de la revendication 1, dans lequel le résidu d'acide aminé est situé dans :
(i) au moins la position 16 ou 21 d'une séquence VpreB1 mature ; et/ou
(ii) au moins une ou plusieurs des positions sélectionnées dans le groupe composé de 60, 74, 78, 79, 85, 91, 110, 123, 131, 133, 166 et 170 d'une séquence λ5 mature.

3. Le conjugué de molécule de liaison des revendications 1 ou 2, dans lequel le conjugué de molécule de liaison relie au moins une cible ou au moins deux cibles.

4. Le conjugué de molécule de liaison des revendications 1 ou 2, dans lequel le résidu d'acide aminé est introduit dans la séquence d'acide aminé du polypeptide SLC.

5. Le conjugué de molécule de liaison des revendications 1 ou 2, dans lequel le résidu d'acide aminé est introduit dans la séquence d'acide aminé du polypeptide SLC par une substitution.

6. Le conjugué de molécule de liaison des revendications 1 ou 2, dans lequel l'acide aminé introduit est une cystéine ou une lysine.

7. Le conjugué de molécule de liaison des revendications 1 ou 2, comprenant un polypeptide de chaîne légère de substitution (SLC) ayant au moins une séquence d'acide aminé sélectionnée dans le groupe composé de :
SSALG**C**TIRLT (ID. SÉQ. N° : 37)
TTIRL**C**CTLRN (ID. SÉQ. N° : 38)
SSVTH**C**FGSGT (ID. SÉQ. N° : 39)
VLSQP**C**ATPSV (ID. SÉQ. N° : 40)
PKATP**C**VTLFP (ID. SÉQ. N° : 41)
KATPS**C**TLFPP (ID. SÉQ. N° : 42)
TLFPP**C**SEELQ (ID. SÉQ. N° : 43)
SEELQ**C**NKATL (ID. SÉQ. N° : 44)
PGILT**C**TWKAD (ID. SÉQ. N° : 45)
PITQG**C**EMTTP (ID. SÉQ. N° : 46)
TTPSK**C**SNNKY (ID. SÉQ. N° : 47)
PSKQS**C**NKYAA (ID. SÉQ. N° : 48)
HEGST**C**EKTVA (ID. SÉQ. N° : 49)
TCEKT**C**APAEC (ID. SÉQ. N° : 50)

8. Le conjugué de molécule de liaison de l'une quelconque des revendications 1 à 7, comprenant également un lieur clivable ou non clivable.

9. Le conjugué de molécule de liaison de l'une quelconque des revendications 1 à 7 comprenant un lieur non clivable comprenant une fraction à base de maléimide ou une fraction à base d'haloacétyle.

10. Le conjugué de molécule de liaison de l'une quelconque des revendications 1 à 7, comprenant également un lieur clivable sélectionné dans le groupe composé d'un lieur peptidyle, d'un lieur sensible au pH et d'un lieur clivable en conditions réductrices.

11. Le conjugué de molécule de liaison de l'une quelconque des revendications 1 à 7, comprenant également un lieu clivable comprenant un lieur dipeptide.

12. Le conjugué de molécule de liaison de l'une quelconque des revendications 1 à 7, comprenant également un lieur non clivable ou un lieur clivable sélectionné dans le groupe composé de : N-succinimide 4-(maléimidométhyl)cyclohexane carboxylate (SMCC), N-succinimidel-4-(N-maléimidométhyl)-cyclohexane-1-carboxy-(6-amidocaproate), (LC-SMCC), ester N-succinimide d'acide κ-maléimidoundécanoïque (KMUA), ester N-succinimide d'acide γ-maléimidobutyrique (GMBS), ester N-hydroxysuccinime d'acide ε-maléimidocaproïque (EMCS), ester de m-maléimidobenzoyl-N-hydroxysuccinimide (MBS), ester de Nα(maléimidoacétoxy)succinimide) [AMAS], succinimidyl-6-[β-maléïmidopropionamido]hexanoate (SMPH), N-succinimide 4-(p-maléimidophényl) butyrate (SMPB), N-(p-maléimidophényl) isocyanate (PMPI), 6-maléimidocaproyl (MC), maléimidopropanoyl (MP), valine-citrulline (val-cit), alanine-phénylalanine (ala-phé), paminobenzyloxycarbonyl (PAB), N-succinimide 4-(2-pyridylthio) pentanoate (SPP), carbonate de p-nitrophényl maléimidocaproyl-L-valine-L-citrulline-p-alcool aminobenzyle (MC-val-cit-PAB), N-succinimide-4-(iodoacétyl)-aminobenzoate (SIAB), N-succinimide iodoacétate (SIA), N-succinimide bromoacetate (SBA), N-succinimide 3-(bromoacétamido)propionate (SBAP), N-succinimide-5-acétylthioacétate (SATA), N-succinimide-3-(2-pyridylthio)propionate (SPDP), N-succinimide-3-(2-pyridylthio)butyrate) (SPDB) et N-succinimide-oxycarbonyl-alpha-méthyl-alpha-(2-pyridyl-dithio)toluène) (SMPT).


### SEQUENCE LISTING

<110> SEA LANE BIOTECHNOLOGIES, LLC
<120> BINDING MOLECULE CONJUGATES
<130> SLN-0025 PR1
<140>
   <141>
<150> 61/848,379
   <151> 2013-01-02
<150> 61/589,272
   <151> 2012-01-20
<160> 71
<170> PatentIn version 3.5
<210> 1
   <211> 145
   <212> PRT
   <213> Homo sapiens
<400> 1 Pro
145
<210> 2
   <211> 142
   <212> PRT
   <213> Mus sp.
<400> 2
   Met Ala Trp Thr Ser Val Leu Leu Met Leu Leu Ala His Leu Thr Gly
   1               5                   10                  15
   Cys Gly Pro Gln Pro Met Val His Gln Pro Pro Ser Ala Ser Ser Ser
               20                  25                  30
   Leu Gly Ala Thr Ile Arg Leu Ser Cys Thr Leu Ser Asn Asp His Asn
           35                  40                  45
   Ile Gly Ile Tyr Ser Ile Tyr Trp Tyr Gln Gln Arg Pro Gly His Pro
       50                  55                  60
   Pro Arg Phe Leu Leu Arg Tyr Phe Ser His Ser Asp Lys His Gln Gly
   65                  70                  75                  80
   Pro Asp Ile Pro Pro Arg Phe Ser Gly Ser Lys Asp Thr Ala Arg Asn
                   85                  90                  95
   Leu Gly Tyr Leu Ser Ile Ser Glu Leu Gln Pro Glu Asp Glu Ala Val
               100                 105                 110
   Tyr Tyr Cys Ala Val Gly Leu Arg Ser His Glu Lys Lys Arg Met Glu
           115                 120                 125
   Arg Glu Trp Glu Gly Glu Lys Ser Tyr Thr Asp Leu Gly Ser
       130                 135                 140
<210> 3
   <211> 171
   <212> PRT
   <213> Mus sp.
<400> 3
   Met Ala Trp Thr Ser Val Leu Leu Met Leu Leu Ala His Leu Thr Gly
   1               5                   10                  15
   Lys Gly Thr Leu Gly Val Gln Gly Phe Leu Ala Pro Pro Val Ala Leu
               20                  25                  30
   Leu Cys Pro Ser Asp Gly His Ala Ser Ile Phe Ser Gly Cys Gly Pro
           35                  40                  45
   Gln Pro Met Val His Gln Pro Pro Ser Ala Ser Ser Ser Leu Gly Ala
       50                  55                  60
   Thr Ile Arg Leu Ser Cys Thr Leu Ser Asn Asp His Asn Ile Gly Ile
   65                  70                  75                  80
   Tyr Ser Ile Tyr Trp Tyr Gln Gln Arg Pro Gly His Pro Pro Arg Phe
                   85                  90                  95
   Leu Leu Arg Tyr Phe Ser His Ser Asp Lys His Gln Gly Pro Asp Ile
               100                 105                 110
   Pro Pro Arg Phe Ser Gly Ser Lys Asp Thr Ala Arg Asn Leu Gly Tyr
           115                 120                 125
   Leu Ser Ile Ser Glu Leu Gln Pro Glu Asp Glu Ala Val Tyr Tyr Cys
       130                 135                 140
   Ala Val Gly Leu Arg Ser His Glu Lys Lys Arg Met Glu Arg Glu Trp
   145                 150                 155                 160
   Glu Gly Glu Lys Ser Tyr Thr Asp Leu Gly Ser
                   165                 170
<210> 4
   <211> 123
   <212> PRT
   <213> Homo sapiens
<400> 4
   Met Ala Cys Arg Cys Leu Ser Phe Leu Leu Met Gly Thr Phe Leu Ser
   1               5                   10                  15
   Val Ser Gln Thr Val Leu Ala Gln Leu Asp Ala Leu Leu Val Phe Pro
               20                  25                  30
   Gly Gln Val Ala Gln Leu Ser Cys Thr Leu Ser Pro Gln His Val Thr
           35                  40                  45
   Ile Arg Asp Tyr Gly Val Ser Trp Tyr Gln Gln Arg Ala Gly Ser Ala
       50                  55                  60
   Pro Arg Tyr Leu Leu Tyr Tyr Arg Ser Glu Glu Asp His His Arg Pro
   65                  70                  75                  80
   Ala Asp Ile Pro Asp Arg Phe Ser Ala Ala Lys Asp Glu Ala His Asn
                   85                  90                  95
   Ala Cys Val Leu Thr Ile Ser Pro Val Gln Pro Glu Asp Asp Ala Asp
               100                 105                 110
   Tyr Tyr Cys Ser Val Gly Tyr Gly Phe Ser Pro
           115                 120
<210> 5
   <211> 119
   <212> PRT
   <213> Homo sapiens
<400> 5
   Met Ser Trp Ala Pro Val Leu Leu Met Leu Phe Val Tyr Cys Thr Gly
   1               5                   10                  15
   Cys Gly Pro Gln Pro Val Leu His Gln Pro Pro Ala Met Ser Ser Ala
               20                  25                  30
   Leu Gly Thr Thr Ile Arg Leu Thr Cys Thr Leu Arg Asn Asp His Asp
           35                  40                  45
   Ile Gly Val Tyr Ser Val Tyr Trp Tyr Gln Gln Arg Pro Gly His Pro
       50                  55                  60
   Pro Arg Phe Leu Leu Arg Tyr Phe Ser Gln Ser Asp Lys Ser Gln Gly
   65                  70                  75                  80
   Pro Gln Val Pro Pro Arg Phe Ser Gly Ser Lys Asp Val Ala Arg Asn
                   85                  90                  95
   Arg Gly Tyr Leu Ser Ile Ser Glu Leu Gln Pro Glu Asp Glu Ala Met
               100                 105                 110
   Tyr Tyr Cys Ala Met Gly Ala
           115
<210> 6
   <211> 146
   <212> PRT
   <213> Artificial Sequence
<220>
   <221> source
   <223> /note="Description of Artificial Sequence: Synthetic polypeptide"
<400> 6
   Met Glu Thr Asp Thr Leu Leu Leu Trp Val Leu Leu Leu Trp Val Pro
   1               5                   10                  15
   Gly Ser Thr Gly Gln Pro Val Leu His Gln Pro Pro Ala Met Ser Ser
               20                  25                  30
   Ala Leu Gly Thr Thr Ile Arg Leu Thr Cys Thr Leu Arg Asn Asp His
           35                  40                  45
   Asp Ile Gly Val Tyr Ser Val Tyr Trp Tyr Gln Gln Arg Pro Gly His
       50                  55                  60
   Pro Pro Arg Phe Leu Leu Arg Tyr Phe Ser Gln Ser Asp Lys Ser Gln
   65                  70                  75                  80
   Gly Pro Gln Val Pro Pro Arg Phe Ser Gly Ser Lys Asp Val Ala Arg
                   85                  90                  95
   Asn Arg Gly Tyr Leu Ser Ile Ser Glu Leu Gln Pro Glu Asp Glu Ala
               100                 105                 110
   Met Tyr Tyr Cys Ala Met Gly Ala Arg Ser Ser Glu Lys Glu Glu Arg
           115                 120                 125
   Glu Arg Glu Trp Glu Glu Glu Met Glu Pro Thr Ala Ala Arg Thr Arg
       130                 135                 140
   Val Pro
   145
<210> 7
   <211> 209
   <212> PRT
   <213> Mus sp.
<400> 7
   Met Lys Leu Arg Val Gly Gln Thr Leu Gly Thr Ile Pro Arg Gln Cys
   1               5                   10                  15
   Glu Val Leu Leu Leu Leu Leu Leu Leu Gly Leu Val Asp Gly Val His
               20                  25                  30
   His Ile Leu Ser Pro Ser Ser Ala Glu Arg Ser Arg Ala Val Gly Pro
           35                  40                  45
   Gly Ala Ser Val Gly Ser Asn Arg Pro Ser Leu Trp Ala Leu Pro Gly
       50                  55                  60
   Arg Leu Leu Phe Gln Ile Ile Pro Arg Gly Ala Gly Pro Arg Cys Ser
   65                  70                  75                  80
   Pro His Arg Leu Pro Ser Lys Pro Gln Phe Trp Tyr Val Phe Gly Gly
                   85                  90                  95
   Gly Thr Gln Leu Thr Ile Leu Gly Gln Pro Lys Ser Asp Pro Leu Val
               100                 105                 110
   Thr Leu Phe Leu Pro Ser Leu Lys Asn Leu Gln Pro Thr Arg Pro His
           115                 120                 125
   Val Val Cys Leu Val Ser Glu Phe Tyr Pro Gly Thr Leu Val Val Asp
       130                 135                 140
   Trp Lys Val Asp Gly Val Pro Val Thr Gln Gly Val Glu Thr Thr Gln
   145                 150                 155                 160
   Pro Ser Lys Gln Thr Asn Asn Lys Tyr Met Val Ser Ser Tyr Leu Thr
                   165                 170                 175
   Leu Ile Ser Asp Gln Trp Met Pro His Ser Arg Tyr Ser Cys Arg Val
               180                 185                 190
   Thr His Glu Gly Asn Thr Val Glu Lys Ser Val Ser Pro Ala Glu Cys
           195                 200                 205
   Ser
<210> 8
   <211> 213
   <212> PRT
   <213> Homo sapiens
<400> 8
   Met Arg Pro Gly Thr Gly Gln Gly Gly Leu Glu Ala Pro Gly Glu Pro
   1               5                   10                  15
   Gly Pro Asn Leu Arg Gln Arg Trp Pro Leu Leu Leu Leu Gly Leu Ala
               20                  25                  30
   Val Val Thr His Gly Leu Leu Arg Pro Thr Ala Ala Ser Gln Ser Arg
           35                  40                  45
   Ala Leu Gly Pro Gly Ala Pro Gly Gly Ser Ser Arg Ser Ser Leu Arg
       50                  55                  60
   Ser Arg Trp Gly Arg Phe Leu Leu Gln Arg Gly Ser Trp Thr Gly Pro
   65                  70                  75                  80
   Arg Cys Trp Pro Arg Gly Phe Gln Ser Lys His Asn Ser Val Thr His
                   85                  90                  95
   Val Phe Gly Ser Gly Thr Gln Leu Thr Val Leu Ser Gln Pro Lys Ala
               100                 105                 110
   Thr Pro Ser Val Thr Leu Phe Pro Pro Ser Ser Glu Glu Leu Gln Ala
           115                 120                 125
   Asn Lys Ala Thr Leu Val Cys Leu Met Asn Asp Phe Tyr Pro Gly Ile
       130                 135                 140
   Leu Thr Val Thr Trp Lys Ala Asp Gly Thr Pro Ile Thr Gln Gly Val
   145                 150                 155                 160
   Glu Met Thr Thr Pro Ser Lys Gln Ser Asn Asn Lys Tyr Ala Ala Ser
                   165                 170                 175
   Ser Tyr Leu Ser Leu Thr Pro Glu Gln Trp Arg Ser Arg Arg Ser Tyr
               180                 185                 190
   Ser Cys Gln Val Met His Glu Gly Ser Thr Val Glu Lys Thr Val Ala
           195                 200                 205
   Pro Ala Glu Cys Ser
       210
<210> 9
   <211> 158
   <212> PRT
   <213> Artificial Sequence
<220>
   <221> source
   <223> /note="Description of Artificial Sequence: Synthetic polypeptide"
<400> 9
   Met Arg Pro Gly Thr Gly Gln Gly Gly Leu Glu Ala Pro Gly Glu Pro
   1               5                   10                  15
   Gly Pro Asn Leu Arg Gln Arg Trp Pro Leu Leu Leu Leu Gly Leu Ala
               20                  25                  30
   Val Val Thr His Gly Ser Val Thr His Val Phe Gly Ser Gly Thr Gln
           35                  40                  45
   Leu Thr Val Leu Ser Gln Pro Lys Ala Thr Pro Ser Val Thr Leu Phe
       50                  55                  60
   Pro Pro Ser Ser Glu Glu Leu Gln Ala Asn Lys Ala Thr Leu Val Cys
   65                  70                  75                  80
   Leu Met Asn Asp Phe Tyr Pro Gly Ile Leu Thr Val Thr Trp Lys Ala
                   85                  90                  95
   Asp Gly Thr Pro Ile Thr Gln Gly Val Glu Met Thr Thr Pro Ser Lys
               100                 105                 110
   Gln Ser Asn Asn Lys Tyr Ala Ala Ser Ser Tyr Leu Ser Leu Thr Pro
           115                 120                 125
   Glu Gln Trp Arg Ser Arg Arg Ser Tyr Ser Cys Gln Val Met His Glu
       130                 135                 140
   Gly Ser Thr Val Glu Lys Thr Val Ala Pro Ala Glu Cys Ser
   145                 150                 155
<210> 10
   <211> 141
   <212> PRT
   <213> Artificial Sequence
<220>
   <221> source
   <223> /note="Description of Artificial Sequence: Synthetic polypeptide"
<400> 10
   Met Glu Thr Asp Thr Leu Leu Leu Trp Val Leu Leu Leu Trp Val Pro
   1               5                   10                  15
   Gly Ser Thr Gly Ser Val Thr His Val Phe Gly Ser Gly Thr Gln Leu
               20                  25                  30
   Thr Val Leu Ser Gln Pro Lys Ala Thr Pro Ser Val Thr Leu Phe Pro
           35                  40                  45
   Pro Ser Ser Glu Glu Leu Gln Ala Asn Lys Ala Thr Leu Val Cys Leu
       50                  55                  60
   Met Asn Asp Phe Tyr Pro Gly Ile Leu Thr Val Thr Trp Lys Ala Asp
   65                  70                  75                  80
   Gly Thr Pro Ile Thr Gln Gly Val Glu Met Thr Thr Pro Ser Lys Gln
                   85                  90                  95
   Ser Asn Asn Lys Tyr Ala Ala Ser Ser Tyr Leu Ser Leu Thr Pro Glu
               100                 105                 110
   Gln Trp Arg Ser Arg Arg Ser Tyr Ser Cys Gln Val Met His Glu Gly
           115                 120                 125
   Ser Thr Val Glu Lys Thr Val Ala Pro Ala Glu Cys Ser
       130                 135                 140
<210> 11
   <211> 711
   <212> DNA
   <213> Homo sapiens
<220>
   <221> CDS
   <222> (8)..(547)
<400> 11
   cagcaag atg gtg ttg cag acc cag gtc ttc att tct ctg ttg ctc tgg          49
           Met Val Leu Gln Thr Gln Val Phe Ile Ser Leu Leu Leu Trp
           1               5                   10
   atc tct ggt gcc tac ggg gac atc gtg atg acc cag tct cca gac tcc          97
   Ile Ser Gly Ala Tyr Gly Asp Ile Val Met Thr Gln Ser Pro Asp Ser
   15                  20                  25                  30
   ctg gct gtg tct ctg ggc gag agg gcc acc atc aac tgc aag tcc agc         145
   Leu Ala Val Ser Leu Gly Glu Arg Ala Thr Ile Asn Cys Lys Ser Ser
                   35                  40                  45
   cag agt gtt tta tac agc tcc aac aat aag aac tac tta gct tgg tac         193
   Gln Ser Val Leu Tyr Ser Ser Asn Asn Lys Asn Tyr Leu Ala Trp Tyr
               50                  55                  60
   cag cag aaa cca gga cag cct cct aag ctg ctc att tac tgg gca tct         241
   Gln Gln Lys Pro Gly Gln Pro Pro Lys Leu Leu Ile Tyr Trp Ala Ser
           65                  70                  75
   acc cgg gaa tcc ggg gtc cct gac cga ttc agt ggc agc ggg tct ggg         289
   Thr Arg Glu Ser Gly Val Pro Asp Arg Phe Ser Gly Ser Gly Ser Gly
       80                  85                  90
   aca gat ttc act ctc acc atc agc agc ctg cag gct gaa gat gtg gca         337
   Thr Asp Phe Thr Leu Thr Ile Ser Ser Leu Gln Ala Glu Asp Val Ala
   95                  100                 105                 110
   gtt tat tac tgt cag caa tat tat agt act cct ccc aca gtg ctt cag         385
   Val Tyr Tyr Cys Gln Gln Tyr Tyr Ser Thr Pro Pro Thr Val Leu Gln
                   115                 120                 125
   cct cga aca caa acc tcc tcc cca tac gct ggg cca gta ggt ctt tgc         433
   Pro Arg Thr Gln Thr Ser Ser Pro Tyr Ala Gly Pro Val Gly Leu Cys
               130                 135                 140
   tgc agc agc tgc ttc ctc tgc aca cag ccc cca aca tgc atg ctt cct         481
   Cys Ser Ser Cys Phe Leu Cys Thr Gln Pro Pro Thr Cys Met Leu Pro
           145                 150                 155
   ctg tgt gtt ggg gag gtc act ctc ttg att tat tcg ttg gag ggt ttg         529
   Leu Cys Val Gly Glu Val Thr Leu Leu Ile Tyr Ser Leu Glu Gly Leu
       160                 165                 170
   cag ggc cca gga tta aat taagagactt gacttttgct ggatctcttt                577
   Gln Gly Pro Gly Leu Asn
   175                 180
   ttgtagaaga ttattaaagc aaaatgttgt aaagatccct tagagacatt gtcaggagtt       637
   tttgtgttac aggaacctgc atgtttcaca tggacacatc acatgaccga gccaaataga       697
   tttatcttta ctct                                                         711
<210> 12
   <211> 180
   <212> PRT
   <213> Homo sapiens
<400> 12
   Met Val Leu Gln Thr Gln Val Phe Ile Ser Leu Leu Leu Trp Ile Ser
   1               5                   10                  15
   Gly Ala Tyr Gly Asp Ile Val Met Thr Gln Ser Pro Asp Ser Leu Ala
               20                  25                  30
   Val Ser Leu Gly Glu Arg Ala Thr Ile Asn Cys Lys Ser Ser Gln Ser
           35                  40                  45
   Val Leu Tyr Ser Ser Asn Asn Lys Asn Tyr Leu Ala Trp Tyr Gln Gln
       50                  55                  60
   Lys Pro Gly Gln Pro Pro Lys Leu Leu Ile Tyr Trp Ala Ser Thr Arg
   65                  70                  75                  80
   Glu Ser Gly Val Pro Asp Arg Phe Ser Gly Ser Gly Ser Gly Thr Asp
                   85                  90                  95
   Phe Thr Leu Thr Ile Ser Ser Leu Gln Ala Glu Asp Val Ala Val Tyr
               100                 105                 110
   Tyr Cys Gln Gln Tyr Tyr Ser Thr Pro Pro Thr Val Leu Gln Pro Arg
           115                 120                 125
   Thr Gln Thr Ser Ser Pro Tyr Ala Gly Pro Val Gly Leu Cys Cys Ser
       130                 135                 140
   Ser Cys Phe Leu Cys Thr Gln Pro Pro Thr Cys Met Leu Pro Leu Cys
   145                 150                 155                 160
   Val Gly Glu Val Thr Leu Leu Ile Tyr Ser Leu Glu Gly Leu Gln Gly
                   165                 170                 175
   Pro Gly Leu Asn
               180
<210> 13
   <211> 103
   <212> PRT
   <213> Artificial Sequence
<220>
   <221> source
   <223> /note="Description of Artificial Sequence: Synthetic polypeptide"
<400> 13
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala Asn Ser Phe Pro Pro
                   85                  90                  95
   Thr Val Leu Pro Thr Arg Thr
               100
<210> 14
   <211> 103
   <212> PRT
   <213> Artificial Sequence
<220>
   <221> source
   <223> /note="Description of Artificial Sequence: Synthetic polypeptide"
<400> 14
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Tyr
               20                  25                  30
   Leu Ala Trp Phe Gln Gln Lys Pro Gly Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Pro Pro
                   85                  90                  95
   Thr Val Leu His Thr Gln Thr
               100
<210> 15
   <211> 110
   <212> PRT
   <213> Artificial Sequence
<220>
   <221> source
   <223> /note="Description of Artificial Sequence: Synthetic polypeptide"
<400> 15
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Pro
                   85                  90                  95
   Thr Val Leu His Thr Arg Thr Thr Pro Arg Glu Ala Asp Val
               100                 105                 110
<210> 16
   <211> 98
   <212> PRT
   <213> Artificial Sequence
<220>
   <221> source
   <223> /note="Description of Artificial Sequence: Synthetic polypeptide"
<400> 16
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Gln Gln Tyr Asp Asn Leu Pro Pro
                   85                  90                  95
   Thr Val
<210> 17
   <211> 101
   <212> PRT
   <213> Artificial Sequence
<220>
   <221> source
   <223> /note="Description of Artificial Sequence: Synthetic polypeptide"
<400> 17
   Asp Ile Gln Met Ile Gln Ser Pro Ser Phe Leu Ser Ala Ser Val Gly
   1               5                   10                  15
   Asp Arg Val Ser Ile Ile Cys Trp Ala Ser Glu Gly Ile Ser Ser Asn
               20                  25                  30
   Leu Ala Trp Tyr Leu Gln Lys Pro Gly Lys Ser Pro Lys Leu Phe Leu
           35                  40                  45
   Tyr Asp Ala Lys Asp Leu His Pro Gly Val Ser Ser Arg Phe Ser Gly
       50                  55                  60
   Arg Gly Ser Gly Thr Asp Phe Thr Leu Thr Ile Ile Ser Leu Lys Pro
   65                  70                  75                  80
   Glu Asp Phe Ala Ala Tyr Tyr Cys Lys Gln Asp Phe Ser Tyr Pro Pro
                   85                  90                  95
   Thr Gly Leu Gln Ala
   100
<210> 18
   <211> 135
   <212> PRT
   <213> Artificial Sequence
<220>
   <221> source
   <223> /note="Description of Artificial Sequence: Synthetic polypeptide"
<400> 18
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Pro Thr Val Val Gln Pro Leu Thr Glu Thr Ser Ser
               100                 105                 110
   Trp Gly Cys Pro Val Ala His Met Cys Cys Leu Ser Gly Glu Gln Leu
           115                 120                 125
   Ser Arg Val Ser Glu Ser Ala
       130                 135
<210> 19
   <211> 106
   <212> PRT
   <213> Artificial Sequence
<220>
   <221> source
   <223> /note="Description of Artificial Sequence: Synthetic polypeptide"
<400> 19
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Pro Thr Val Val Gln Pro
               100                 105
<210> 20
   <211> 132
   <212> PRT
   <213> Artificial Sequence
<220>
   <221> source
   <223> /note="Description of Artificial Sequence: Synthetic polypeptide"
<400> 20
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Pro
                   85                  90                  95
   Thr Val Ile Pro His Glu Thr Lys Thr Pro Thr Arg Pro Ser Val Phe
               100                 105                 110
   Thr Arg Leu Leu Tyr Gln Leu Leu Pro Leu Gln Thr Ala Ser Gly Val
           115                 120                 125
   Ala Thr Gln Cys
       130
<210> 21
   <211> 118
   <212> PRT
   <213> Artificial Sequence
<220>
   <221> source
   <223> /note="Description of Artificial Sequence: Synthetic polypeptide"
<400> 21
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Gly Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Pro Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp His Pro
                   85                  90                  95
   Thr Val Ile Pro His Glu Thr Lys Thr Pro Thr Arg Pro Ser Val Phe
               100                 105                 110
   Thr Arg Leu Leu Tyr Gln
           115
<210> 22
   <211> 115
   <212> PRT
   <213> Artificial Sequence
<220>
   <221> source
   <223> /note="Description of Artificial Sequence: Synthetic polypeptide"
<400> 22
   Glu Thr Thr Leu Thr Gln Ser Pro Ala Phe Met Ser Ala Thr Pro Gly
   1               5                   10                  15
   Asp Lys Val Asn Ile Ser Cys Lys Ala Ser Gln Asp Ile Asp Asp Asp
               20                  25                  30
   Met Asn Trp Tyr Gln Gln Lys Pro Gly Glu Ala Ala Ile Phe Ile Ile
           35                  40                  45
   Gln Glu Ala Thr Thr Leu Val Pro Gly Ile Pro Pro Arg Phe Ser Gly
       50                  55                  60
   Ser Gly Tyr Gly Thr Asp Phe Thr Leu Thr Ile Asn Asn Ile Glu Ser
   65                  70                  75                  80
   Glu Asp Ala Ala Tyr Tyr Phe Cys Leu Gln His Asp Asn Phe Pro Leu
                   85                  90                  95
   Thr Val Ile His Pro Val Gln Lys Pro Pro Ser Ser Leu Ser Gly Ile
               100                 105                 110
   Ala Ser Ala
           115
<210> 23
   <211> 158
   <212> PRT
   <213> Artificial Sequence
<220>
   <221> source
   <223> /note="Description of Artificial Sequence: Synthetic polypeptide"
<400> 23
   Asp Val Val Met Thr Gln Ser Pro Ala Phe Leu Ser Val Thr Pro Gly
   1               5                   10                  15
   Glu Lys Val Thr Ile Thr Cys Gln Ala Ser Glu Gly Ile Gly Asn Tyr
               20                  25                  30
   Leu Tyr Trp Tyr Gln Gln Lys Pro Asp Gln Ala Pro Lys Leu Leu Ile
           35                  40                  45
   Lys Tyr Ala Ser Gln Ser Ile Ser Gly Val Pro Ser Arg Phe Ser Gly
       50                  55                  60
   Ser Gly Ser Gly Thr Asp Phe Thr Phe Thr Ile Ser Ser Leu Glu Ala
   65                  70                  75                      80
   Glu Asp Ala Ala Thr Tyr Tyr Cys Gln Gln Gly Asn Lys His Pro His
                   85                  90                  95
   Thr Val Leu Gln Pro Lys Thr Lys Ile Ser Ser Ala Trp Arg Asn Arg
               100                 105                 110
   Glu Thr Glu Gln Tyr Pro Val Phe Met Ile Leu Ala Gly Ala Val Gly
           115                 120                 125
   Glu Ile Ile Tyr Gln Ile Pro Ser His Met Ala His Ser Ala Glu Leu
       130                 135                 140
   Thr Pro Lys Ser Gln Cys Leu Thr Leu Ser Ser Leu Pro Thr
   145                 150                 155
<210> 24
   <211> 103
   <212> PRT
   <213> Artificial Sequence
<220>
   <221> source
   <223> /note="Description of Artificial Sequence: Synthetic polypeptide"
<400> 24
   Asp Ile Val Leu Thr Gln Ser Pro Ala Ser Leu Ala Val Ser Pro Gly
   1               5                   10                  15
   Gln Arg Ala Thr Ile Thr Cys Arg Ala Ser Glu Ser Val Ser Phe Leu
               20                  25                  30
   Gly Ile Asn Leu Ile His Trp Tyr Gln Gln Lys Pro Gly Gln Pro Pro
           35                  40                  45
   Lys Leu Leu Ile Tyr Gln Ala Ser Asn Lys Asp Thr Gly Val Pro Ala
       50                  55                  60
   Arg Phe Ser Gly Ser Gly Ser Gly Thr Asp Phe Thr Leu Thr Ile Asn
   65                  70                  75                  80
   Pro Val Glu Ala Asn Asp Thr Ala Asn Tyr Tyr Cys Leu Gln Ser Lys
                   85                  90                  95
   Asn Phe Pro Pro Thr Val Leu
               100
<210> 25
   <211> 408
   <212> DNA
   <213> Homo sapiens
<220>
   <221> CDS
   <222> (1)..(405)
<400> 25
   gtg aga agg gtt ttt gtt cag caa gac aat gga gag ctc aca ctg tgg          48
   Val Arg Arg Val Phe Val Gln Gln Asp Asn Gly Glu Leu Thr Leu Trp
   1               5                   10                  15
   tgg acg ttc ggc caa ggg acc aag gtg gaa atc aaa cga act gtg gct          96
   Trp Thr Phe Gly Gln Gly Thr Lys Val Glu Ile Lys Arg Thr Val Ala
               20                  25                  30
   gca cca tct gtc ttc atc ttc ccg cca tct gat gag cag ttg aaa tct         144
   Ala Pro Ser Val Phe Ile Phe Pro Pro Ser Asp Glu Gln Leu Lys Ser
           35                  40                  45
   gga act gcc tct gtt gtg tgc ctg ctg aat aac ttc tat ccc aga gag         192
   Gly Thr Ala Ser Val Val Cys Leu Leu Asn Asn Phe Tyr Pro Arg Glu
       50                  55                  60
   gcc aaa gta cag tgg aag gtg gat aac gcc ctc caa tcg ggt aac tcc         240
   Ala Lys Val Gln Trp Lys Val Asp Asn Ala Leu Gln Ser Gly Asn Ser
   65                  70                  75                  80
   cag gag agt gtc aca gag cag gac agc aag gac agc acc tac agc ctc         288
   Gln Glu Ser Val Thr Glu Gln Asp Ser Lys Asp Ser Thr Tyr Ser Leu
                   85                  90                  95
   agc agc acc ctg acg ctg agc aaa gca gac tac gag aaa cac aaa ctc         336
   Ser Ser Thr Leu Thr Leu Ser Lys Ala Asp Tyr Glu Lys His Lys Leu
               100                 105                 110
   tac gcc tgc gaa gtc acc cat cag ggc ctg agc tcg ccc gtc aca aag         384
   Tyr Ala Cys Glu Val Thr His Gln Gly Leu Ser Ser Pro Val Thr Lys
           115                 120                 125
   agc ttc aac agg gga gag tgt tag                                         408
   Ser Phe Asn Arg Gly Glu Cys
       130                 135
<210> 26
   <211> 135
   <212> PRT
   <213> Homo sapiens
<400> 26
   Val Arg Arg Val Phe Val Gln Gln Asp Asn Gly Glu Leu Thr Leu Trp
   1               5                   10                  15
   Trp Thr Phe Gly Gln Gly Thr Lys Val Glu Ile Lys Arg Thr Val Ala
               20                  25                  30
   Ala Pro Ser Val Phe Ile Phe Pro Pro Ser Asp Glu Gln Leu Lys Ser
           35                  40                  45
   Gly Thr Ala Ser Val Val Cys Leu Leu Asn Asn Phe Tyr Pro Arg Glu
   50 55 60
   Ala Lys Val Gln Trp Lys Val Asp Asn Ala Leu Gln Ser Gly Asn Ser
   65 70 75 80
   Gln Glu Ser Val Thr Glu Gln Asp Ser Lys Asp Ser Thr Tyr Ser Leu
   85 90 95
   Ser Ser Thr Leu Thr Leu Ser Lys Ala Asp Tyr Glu Lys His Lys Leu
   100 105 110
   Tyr Ala Cys Glu Val Thr His Gln Gly Leu Ser Ser Pro Val Thr Lys
   115 120 125
   Ser Phe Asn Arg Gly Glu Cys
   130 135
<210> 27
   <211> 119
   <212> PRT
   <213> Artificial Sequence
<220>
   <221> source
   <223> /note="Description of Artificial Sequence: Synthetic polypeptide"
<400> 27
   Trp Thr Phe Gly Gln Gly Thr Lys Val Glu Ile Lys Arg Thr Val Ala
   1 5 10 15
   Ala Pro Ser Val Phe Ile Phe Pro Pro Ser Asp Glu Gln Leu Lys Ser
   20 25 30
   Gly Thr Ala Ser Val Val Cys Leu Leu Asn Asn Phe Tyr Pro Arg Glu
   35 40 45
   Ala Lys Val Gln Trp Lys Val Asp Asn Ala Leu Gln Ser Gly Asn Ser
   50 55 60
   Gln Glu Ser Val Thr Glu Gln Asp Ser Lys Asp Ser Thr Tyr Ser Leu
   65 70 75 80
   Ser Ser Thr Leu Thr Leu Ser Lys Ala Asp Tyr Glu Lys His Lys Leu
   85 90 95
   Tyr Ala Cys Glu Val Thr His Gln Gly Leu Ser Ser Pro Val Thr Lys
   100 105 110
   Ser Phe Asn Arg Gly Glu Cys
   115
<210> 28
   <211> 119
   <212> PRT
   <213> Artificial Sequence
<220>
   <221> source
   <223> /note="Description of Artificial Sequence: Synthetic polypeptide"
<400> 28
   Tyr Thr Phe Gly Gln Gly Thr Lys Leu Glu Ile Lys Arg Thr Val Ala
   1 5 10 15
   Ala Pro Ser Val Phe Ile Phe Pro Pro Ser Asp Glu Gln Leu Lys Ser
   20 25 30
   Gly Thr Ala Ser Val Val Cys Leu Leu Asn Asn Phe Tyr Pro Arg Glu
   35 40 45
   Ala Lys Val Gln Trp Lys Val Asp Asn Ala Leu Gln Ser Gly Asn Ser
   50 55 60
   Gln Glu Ser Val Thr Glu Gln Asp Ser Lys Asp Ser Thr Tyr Ser Leu
   65 70 75 80
   Ser Ser Thr Leu Thr Leu Ser Lys Ala Asp Tyr Glu Lys His Lys Leu
   85 90 95
   Tyr Ala Cys Glu Val Thr His Gln Gly Leu Ser Ser Pro Val Thr Lys
   100 105 110
   Ser Phe Asn Arg Gly Glu Cys
   115
<210> 29
   <211> 119
   <212> PRT
   <213> Artificial Sequence
<220>
   <221> source
   <223> /note="Description of Artificial Sequence: Synthetic polypeptide"
<400> 29
   Phe Thr Phe Gly Pro Gly Thr Lys Val Asp Ile Lys Arg Thr Val Ala
   1 5 10 15
   Ala Pro Ser Val Phe Ile Phe Pro Pro Ser Asp Glu Gln Leu Lys Ser
               20                  25                  30
   Gly Thr Ala Ser Val Val Cys Leu Leu Asn Asn Phe Tyr Pro Arg Glu
           35                  40                  45
   Ala Lys Val Gln Trp Lys Val Asp Asn Ala Leu Gln Ser Gly Asn Ser
       50                  55                  60
   Gln Glu Ser Val Thr Glu Gln Asp Ser Lys Asp Ser Thr Tyr Ser Leu
   65                  70                  75                      80
   Ser Ser Thr Leu Thr Leu Ser Lys Ala Asp Tyr Glu Lys His Lys Leu
                   85                  90                  95
   Tyr Ala Cys Glu Val Thr His Gln Gly Leu Ser Ser Pro Val Thr Lys
               100                 105                 110
   Ser Phe Asn Arg Gly Glu Cys
           115
<210> 30
   <211> 119
   <212> PRT
   <213> Artificial Sequence
<220>
   <221> source
   <223> /note="Description of Artificial Sequence: Synthetic polypeptide"
<400> 30
   Leu Thr Phe Gly Gly Gly Thr Lys Val Glu Ile Lys Arg Thr Val Ala
   1               5                   10                  15
   Ala Pro Ser Val Phe Ile Phe Pro Pro Ser Asp Glu Gln Leu Lys Ser
               20                  25                  30
   Gly Thr Ala Ser Val Val Cys Leu Leu Asn Asn Phe Tyr Pro Arg Glu
           35                  40                  45
   Ala Lys Val Gln Trp Lys Val Asp Asn Ala Leu Gln Ser Gly Asn Ser
       50                  55                  60
   Gln Glu Ser Val Thr Glu Gln Asp Ser Lys Asp Ser Thr Tyr Ser Leu
   65                  70                  75                  80
   Ser Ser Thr Leu Thr Leu Ser Lys Ala Asp Tyr Glu Lys His Lys Leu
                   85                  90                  95
   Tyr Ala Cys Glu Val Thr His Gln Gly Leu Ser Ser Pro Val Thr Lys
               100                 105                 110
   Ser Phe Asn Arg Gly Glu Cys
           115
<210> 31
   <211> 119
   <212> PRT
   <213> Artificial Sequence
<220>
   <221> source
   <223> /note="Description of Artificial Sequence: Synthetic polypeptide"
<400> 31
   Ile Thr Phe Gly Gln Gly Thr Arg Leu Glu Ile Lys Arg Thr Val Ala
   1 5 10 15
   Ala Pro Ser Val Phe Ile Phe Pro Pro Ser Asp Glu Gln Leu Lys Ser
   20 25 30
   Gly Thr Ala Ser Val Val Cys Leu Leu Asn Asn Phe Tyr Pro Arg Glu
   35 40 45
   Ala Lys Val Gln Trp Lys Val Asp Asn Ala Leu Gln Ser Gly Asn Ser
   50 55 60
   Gln Glu Ser Val Thr Glu Gln Asp Ser Lys Asp Ser Thr Tyr Ser Leu
   65 70 75 80
   Ser Ser Thr Leu Thr Leu Ser Lys Ala Asp Tyr Glu Lys His Lys Leu
   85 90 95
   Tyr Ala Cys Glu Val Thr His Gln Gly Leu Ser Ser Pro Val Thr Lys
   100 105 110
   Ser Phe Asn Arg Gly Glu Cys
   115
<210> 32
   <211> 154
   <212> PRT
   <213> Artificial Sequence
<220>
   <221> source
   <223> /note="Description of Artificial Sequence: Synthetic polypeptide"
<400> 32
<210> 33
   <211> 138
   <212> PRT
   <213> Artificial Sequence
<220>
   <221> source
   <223> /note="Description of Artificial Sequence: Synthetic polypeptide"
<400> 33
   Met Glu Thr Asp Thr Leu Leu Leu Trp Val Leu Leu Leu Trp Val Pro
   1               5                   10                  15
   Gly Ser Thr Gly Trp Thr Phe Gly Gln Gly Thr Lys Val Glu Ile Lys
               20                  25                  30
   Arg Thr Val Ala Ala Pro Ser Val Phe Ile Phe Pro Pro Ser Asp Glu
           35                  40                  45
   Gln Leu Lys Ser Gly Thr Ala Ser Val Val Cys Leu Leu Asn Asn Phe
       50                  55                  60
   Tyr Pro Arg Glu Ala Lys Val Gln Trp Lys Val Asp Asn Ala Leu Gln
   65                  70                  75                  80
   Gly Asn Ser Gln Glu Ser Val Thr Glu Gln Asp Ser Lys Asp Ser Thr
                   85                  90                  95
   Tyr Ser Leu Ser Ser Thr Leu Thr Leu Ser Lys Ala Asp Tyr Glu Lys
               100                 105                 110
   His Lys Leu Tyr Ala Cys Glu Val Thr His Gln Gly Leu Ser Ser Pro
           115                 120                 125
   Val Thr Lys Ser Phe Asn Arg Gly Glu Cys
       130                 135
<210> 34
   <211> 132
   <212> PRT
   <213> Artificial Sequence
<220>
   <221> source
   <223> /note="Description of Artificial Sequence: Synthetic polypeptide"
<400> 34
   Met Glu Thr Asp Thr Leu Leu Leu Trp Val Leu Leu Leu Trp Val Pro
   1               5                   10                  15
   Gly Ser Thr Gly Thr Lys Val Glu Ile Lys Arg Thr Val Ala Ala Pro
               20                  25                  30
   Ser Val Phe Ile Phe Pro Pro Ser Asp Glu Gln Leu Lys Ser Gly Thr
           35                  40                  45
   Ala Ser Val Val Cys Leu Leu Asn Asn Phe Tyr Pro Arg Glu Ala Lys
       50                  55                  60
   Val Gln Trp Lys Val Asp Asn Ala Leu Gln Gly Asn Ser Gln Glu Ser
   65                  70                  75                  80
   Val Thr Glu Gln Asp Ser Lys Asp Ser Thr Tyr Ser Leu Ser Ser Thr
                   85                  90                  95
   Leu Thr Leu Ser Lys Ala Asp Tyr Glu Lys His Lys Leu Tyr Ala Cys
               100                 105                 110
   Glu Val Thr His Gln Gly Leu Ser Ser Pro Val Thr Lys Ser Phe Asn
                 115                 120                 125
   Arg Gly Glu Cys
       130
<210> 35
   <211> 243
   <212> PRT
   <213> Artificial Sequence
<220>
   <221> source
   <223> /note="Description of Artificial Sequence: Synthetic polypeptide"
<400> 35
   Met Glu Thr Asp Thr Leu Leu Leu Trp Val Leu Leu Leu Trp Val Pro
   1               5                   10                  15
   Gly Ser Thr Gly Gln Pro Val Leu His Gln Pro Pro Ala Met Ser Ser
               20                  25                  30
   Ala Leu Gly Thr Thr Ile Arg Leu Thr Cys Thr Leu Arg Asn Asp His
           35                  40                  45
   Asp Ile Gly Val Tyr Ser Val Tyr Trp Tyr Gln Gln Arg Pro Gly His
       50                  55                  60
   Pro Pro Arg Phe Leu Leu Arg Tyr Phe Ser Gln Ser Asp Lys Ser Gln
   65                  70                  75                  80
   Gly Pro Gln Val Pro Pro Arg Phe Ser Gly Ser Lys Asp Val Ala Arg
                   85                  90                  95
   Asn Arg Gly Tyr Leu Ser Ile Ser Glu Leu Gln Pro Glu Asp Glu Ala
               100                 105                 110
   Met Tyr Tyr Cys Ala Met Gly Ala Arg Ser Ser Val Thr His Val Phe
           115                 120                 125
   Gly Ser Gly Thr Gln Leu Thr Val Leu Ser Gln Pro Lys Ala Thr Pro
       130                 135                 140
   Ser Val Thr Leu Phe Pro Pro Ser Ser Glu Glu Leu Gln Ala Asn Lys
   145                 150                 155                 160
   Ala Thr Leu Val Cys Leu Met Asn Asp Phe Tyr Pro Gly Ile Leu Thr
                   165                 170                 175
   Val Thr Trp Lys Ala Asp Gly Thr Pro Ile Thr Gln Gly Val Glu Met
                   180                 185                 190
   Thr Thr Pro Ser Lys Gln Ser Asn Asn Lys Tyr Ala Ala Ser Ser Tyr
           195                 200                 205
   Leu Ser Leu Thr Pro Glu Gln Trp Arg Ser Arg Arg Ser Tyr Ser Cys
       210                 215                 220
   Gln Val Met His Glu Gly Ser Thr Val Glu Lys Thr Val Ala Pro Ala
   225                 230                 235                 240
   Glu Cys Ser
<210> 36
   <211> 149
   <212> PRT
   <213> Artificial Sequence
<220>
   <221> source
   <223> /note="Description of Artificial Sequence: Synthetic polypeptide"
<400> 36
   Met Glu Thr Asp Thr Leu Leu Leu Trp Val Leu Leu Leu Trp Val Pro
   1               5                   10                  15
   Gly Ser Thr Gly Gln Pro Val Leu His Gln Pro Pro Ala Met Ser Ser
               20                  25                  30
   Ala Leu Gly Thr Thr Ile Arg Leu Thr Cys Thr Leu Arg Asn Asp His
           35                  40                  45
   Asp Ile Gly Val Tyr Ser Val Tyr Trp Tyr Gln Gln Arg Pro Gly His
       50                  55                  60
   Pro Pro Arg Phe Leu Leu Arg Tyr Phe Ser Gln Ser Asp Lys Ser Gln
   65                  70                  75                  80
   Gly Pro Gln Val Pro Pro Arg Phe Ser Gly Ser Lys Asp Val Ala Arg
                   85                  90                  95
   Asn Arg Gly Tyr Leu Ser Ile Ser Val Thr His Val Phe Gly Ser Gly
               100                 105                 110
   Thr Gln Leu Thr Val Leu Ser Gln Gln Val Gln Leu Val Gln Ser Val
           115                 120                 125
   Thr His Val Phe Gly Ser Gly Thr Gln Leu Thr Val Leu Ser Gln Gln
               130                 135                 140
   Val Gln Leu Val Gln
   145
<210> 37
   <211> 11
   <212> PRT
   <213> Artificial Sequence
<220>
   <221> source
   <223> /note="Description of Artificial Sequence: Synthetic peptide"
<400> 37
   Ser Ser Ala Leu Gly Cys Thr Ile Arg Leu Thr
   1               5                   10
<210> 38
   <211> 11
   <212> PRT
   <213> Artificial Sequence
<220>
   <221> source
   <223> /note="Description of Artificial Sequence: Synthetic peptide"
<400> 38
   Thr Thr Ile Arg Leu Cys Cys Thr Leu Arg Asn
   1               5                   10
<210> 39
   <211> 11
   <212> PRT
   <213> Artificial Sequence
<220>
   <221> source
   <223> /note="Description of Artificial Sequence: Synthetic peptide"
<400> 39
   Ser Ser Val Thr His Cys Phe Gly Ser Gly Thr
   1               5                   10
<210> 40
   <211> 11
   <212> PRT
   <213> Artificial Sequence
<220>
   <221> source
   <223> /note="Description of Artificial Sequence: Synthetic peptide"
<400> 40
   Val Leu Ser Gln Pro Cys Ala Thr Pro Ser Val
   1               5                   10
<210> 41
   <211> 11
   <212> PRT
   <213> Artificial Sequence
<220>
   <221> source
   <223> /note="Description of Artificial Sequence: Synthetic peptide"
<400> 41
   Pro Lys Ala Thr Pro Cys Val Thr Leu Phe Pro
   1               5                   10
<210> 42
   <211> 11
   <212> PRT
   <213> Artificial Sequence
<220>
   <221> source
   <223> /note="Description of Artificial Sequence: Synthetic peptide"
<400> 42
   Lys Ala Thr Pro Ser Cys Thr Leu Phe Pro Pro
   1               5                   10
<210> 43
   <211> 11
   <212> PRT
   <213> Artificial Sequence
<220>
   <221> source
   <223> /note="Description of Artificial Sequence: Synthetic peptide"
<400> 43
   Thr Leu Phe Pro Pro Cys Ser Glu Glu Leu Gln
   1               5                   10
<210> 44
   <211> 11
   <212> PRT
   <213> Artificial Sequence
<220>
   <221> source
   <223> /note="Description of Artificial Sequence: Synthetic peptide"
<400> 44
   Ser Glu Glu Leu Gln Cys Asn Lys Ala Thr Leu
   1               5                   10
<210> 45
   <211> 11
   <212> PRT
   <213> Artificial Sequence
<220>
   <221> source
   <223> /note="Description of Artificial Sequence: Synthetic peptide"
<400> 45
   Pro Gly Ile Leu Thr Cys Thr Trp Lys Ala Asp
   1               5                   10
<210> 46
   <211> 11
   <212> PRT
   <213> Artificial Sequence
<220>
   <221> source
   <223> /note="Description of Artificial Sequence: Synthetic peptide"
<400> 46
   Pro Ile Thr Gln Gly Cys Glu Met Thr Thr Pro
   1               5                   10
<210> 47
   <211> 11
   <212> PRT
   <213> Artificial Sequence
<220>
   <221> source
   <223> /note="Description of Artificial Sequence: Synthetic peptide"
<400> 47
   Thr Thr Pro Ser Lys Cys Ser Asn Asn Lys Tyr
   1               5                   10
<210> 48
   <211> 11
   <212> PRT
   <213> Artificial Sequence
<220>
   <221> source
   <223> /note="Description of Artificial Sequence: Synthetic peptide"
<400> 48
   Pro Ser Lys Gln Ser Cys Asn Lys Tyr Ala Ala
   1               5                   10
<210> 49
   <211> 11
   <212> PRT
   <213> Artificial Sequence
<220>
   <221> source
   <223> /note="Description of Artificial Sequence: Synthetic peptide"
<400> 49
   His Glu Gly Ser Thr Cys Glu Lys Thr Val Ala
   1               5                   10
<210> 50
   <211> 11
   <212> PRT
   <213> Artificial Sequence
<220>
   <221> source
   <223> /note="Description of Artificial Sequence: Synthetic peptide"
<400> 50
   Thr Cys Glu Lys Thr Cys Ala Pro Ala Glu Cys
   1 5 10
<210> 51
   <211> 4
   <212> PRT
   <213> Artificial Sequence
<220>
   <221> source
   <223> /note="Description of Artificial Sequence: Synthetic peptide"
<400> 51
   Gly Phe Leu Gly
   1
<210> 52
   <211> 4
   <212> PRT
   <213> Artificial Sequence
<220>
   <221> source
   <223> /note="Description of Artificial Sequence: Synthetic peptide"
<400> 52
   Gly Ser Val Glu
   1
<210> 53
   <211> 20
   <212> PRT
   <213> Mus sp.
<400> 53
   Met Glu Thr Asp Thr Leu Leu Leu Trp Val Leu Leu Leu Trp Val Pro
   1 5 10 15
   Gly Ser Thr Gly
   20
<210> 54
   <211> 126
   <212> PRT
   <213> Homo sapiens
<400> 54
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Glu Lys Glu Glu Arg Glu Arg Glu Trp
               100                 105                 110
   Glu Glu Glu Met Glu Pro Thr Ala Ala Arg Thr Arg Val Pro
           115                 120                 125
<210> 55
   <211> 176
   <212> PRT
   <213> Homo sapiens
<400> 55
   Leu Leu Arg Pro Thr Ala Ala Ser Gln Ser Arg Ala Leu Gly Pro Gly
   1               5                   10                  15
   Ala Pro Gly Gly Ser Ser Arg Ser Ser Leu Arg Ser Arg Trp Gly Arg
               20                  25                  30
   Phe Leu Leu Gln Arg Gly Ser Trp Thr Gly Pro Arg Cys Trp Pro Arg
           35                  40                  45
   Gly Phe Gln Ser Lys His Asn Ser Val Thr His Val Phe Gly Ser Gly
       50                  55                  60
   Thr Gln Leu Thr Val Leu Ser Gln Pro Lys Ala Thr Pro Ser Val Thr
   65                  70                  75                  80
   Leu Phe Pro Pro Ser Ser Glu Glu Leu Gln Ala Asn Lys Ala Thr Leu
                   85                  90                  95
   Val Cys Leu Met Asn Asp Phe Tyr Pro Gly Ile Leu Thr Val Thr Trp
               100                 105                 110
   Lys Ala Asp Gly Thr Pro Ile Thr Gln Gly Val Glu Met Thr Thr Pro
           115                 120                 125
   Ser Lys Gln Ser Asn Asn Lys Tyr Ala Ala Ser Ser Tyr Leu Ser Leu
       130                 135                 140
   Thr Pro Glu Gln Trp Arg Ser Arg Arg Ser Tyr Ser Cys Gln Val Met
   145                 150                 155                 160
   His Glu Gly Ser Thr Val Glu Lys Thr Val Ala Pro Ala Glu Cys Ser
                   165                 170                 175
<210> 56
   <211> 223
   <212> PRT
   <213> Artificial Sequence
<220>
   <221> source
   <223> /note="Description of Artificial Sequence: Synthetic polypeptide"
<400> 56
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
   85 90 95
   Ala Met Gly Ala Arg Ser Ser Val Thr His Val Phe Gly Ser Gly Thr
   100 105 110
   Gln Leu Thr Val Leu Ser Gln Pro Lys Ala Thr Pro Ser Val Thr Leu
   115 120 125
   Phe Pro Pro Ser Ser Glu Glu Leu Gln Ala Asn Lys Ala Thr Leu Val
   130 135 140
   Cys Leu Met Asn Asp Phe Tyr Pro Gly Ile Leu Thr Val Thr Trp Lys
   145 150 155 160
   Ala Asp Gly Thr Pro Ile Thr Gln Gly Val Glu Met Thr Thr Pro Ser
   165 170 175
   Lys Gln Ser Asn Asn Lys Tyr Ala Ala Ser Ser Tyr Leu Ser Leu Thr
   180 185 190
   Pro Glu Gln Trp Arg Ser Arg Arg Ser Tyr Ser Cys Gln Val Met His
   195 200 205
   Glu Gly Ser Thr Val Glu Lys Thr Val Ala Pro Ala Glu Cys Ser
   210 215 220
<210> 57
   <211> 223
   <212> PRT
   <213> Artificial Sequence
<220>
   <221> source
   <223> /note="Description of Artificial Sequence: Synthetic polypeptide"
<400> 57
   Gln Pro Val Leu His Gln Pro Pro Ala Met Ser Ser Ala Leu Gly Cys
   1 5 10 15
   Thr Ile Arg Leu Thr Cys Thr Leu Arg Asn Asp His Asp Ile Gly Val
   20 25 30
   Tyr Ser Val Tyr Trp Tyr Gln Gln Arg Pro Gly His Pro Pro Arg Phe
   35 40 45
   Leu Leu Arg Tyr Phe Ser Gln Ser Asp Lys Ser Gln Gly Pro Gln Val
   50 55 60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 58
   <211> 223
   <212> PRT
   <213> Artificial Sequence
<220>
   <221> source
   <223> /note="Description of Artificial Sequence: Synthetic polypeptide"
<400> 58
   Gln Pro Val Leu His Gln Pro Pro Ala Met Ser Ser Ala Leu Gly Thr
   1               5                   10                  15
   Thr Ile Arg Leu Cys Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 59
   <211> 223
   <212> PRT
   <213> Artificial Sequence
<220>
   <221> source
   <223> /note="Description of Artificial Sequence: Synthetic polypeptide"
<400> 59
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Cys Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 60
   <211> 223
   <212> PRT
   <213> Artificial Sequence
<220>
   <221> source
   <223> /note="Description of Artificial Sequence: Synthetic polypeptide"
<400> 60
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Cys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 61
   <211> 223
   <212> PRT
   <213> Artificial Sequence
<220>
   <221> source
   <223> /note="Description of Artificial Sequence: Synthetic polypeptide"
<400> 61
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Cys Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 62
   <211> 223
   <212> PRT
   <213> Artificial Sequence
<220>
   <221> source
   <223> /note="Description of Artificial Sequence: Synthetic polypeptide"
<400> 62
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Cys Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 63
   <211> 223
   <212> PRT
   <213> Artificial Sequence
<220>
   <221> source
   <223> /note="Description of Artificial Sequence: Synthetic polypeptide"
<400> 63
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Cys Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
             210                 215                 220
<210> 64
   <211> 223
   <212> PRT
   <213> Artificial Sequence
<220>
   <221> source
   <223> /note="Description of Artificial Sequence: Synthetic polypeptide"
<400> 64
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Cys Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
             195                 200                 205
   Glu Gly Ser Thr Val Glu Lys Thr Val Ala Pro Ala Glu Cys Ser
       210                 215                 220
<210> 65
   <211> 223
   <212> PRT
   <213> Artificial Sequence
<220>
   <221> source
   <223> /note="Description of Artificial Sequence: Synthetic polypeptide"
<400> 65
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Cys Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
                   180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 66
   <211> 223
   <212> PRT
   <213> Artificial Sequence
<220>
   <221> source
   <223> /note="Description of Artificial Sequence: Synthetic polypeptide"
<400> 66
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Cys Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 67
   <211> 223
   <212> PRT
   <213> Artificial Sequence
<220>
   <221> source
   <223> /note="Description of Artificial Sequence: Synthetic polypeptide"
<400> 67
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Cys Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 68
   <211> 223
   <212> PRT
   <213> Artificial Sequence
<220>
   <221> source
   <223> /note="Description of Artificial Sequence: Synthetic polypeptide"
<400> 68
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                      80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
           115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                     160
   Ala Asp Gly Thr Pro Ile Thr Gln Gly Val Glu Met Thr Thr Pro Ser
                   165                 170                 175
   Lys Gln Ser Cys Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Val Glu Lys Thr Val Ala Pro Ala Glu Cys Ser
       210                 215                 220
<210> 69
   <211> 223
   <212> PRT
   <213> Artificial Sequence
<220>
   <221> source
   <223> /note="Description of Artificial Sequence: Synthetic polypeptide"
<400> 69
   Gln Pro Val Leu His Gln Pro Pro Ala Met Ser Ser Ala Leu Gly Thr
   1               5                   10                  15
   Thr Ile Arg Leu Thr Cys Thr Leu Arg Asn Asp His Asp Ile Gly Val
               20                  25                  30
   Tyr Ser Val Tyr Trp Tyr Gln Gln Arg Pro Gly His Pro Pro Arg Phe
           35                  40                  45
   Leu Leu Arg Tyr Phe Ser Gln Ser Asp Lys Ser Gln Gly Pro Gln Val
       50                  55                  60
   Pro Pro Arg Phe Ser Gly Ser Lys Asp Val Ala Arg Asn Arg Gly Tyr
   65                  70                  75                  80
   Leu Ser Ile Ser Glu Leu Gln Pro Glu Asp Glu Ala Met Tyr Tyr Cys
                   85                  90                  95
   Ala Met Gly Ala Arg Ser Ser Val Thr His Val Phe Gly Ser Gly Thr
               100                 105                 110
   Gln Leu Thr Val Leu Ser Gln Pro Lys Ala Thr Pro Ser Val Thr Leu
                   115                 120                 125
   Phe Pro Pro Ser Ser Glu Glu Leu Gln Ala Asn Lys Ala Thr Leu Val
       130                 135                 140
   Cys Leu Met Asn Asp Phe Tyr Pro Gly Ile Leu Thr Val Thr Trp Lys
   145                 150                 155                 160
   Ala Asp Gly Thr Pro Ile Thr Gln Gly Val Glu Met Thr Thr Pro Ser
                   165                 170                 175
   Lys Gln Ser Asn Asn Lys Tyr Ala Ala Ser Ser Tyr Leu Ser Leu Thr
               180                 185                 190
   Pro Glu Gln Trp Arg Ser Arg Arg Ser Tyr Ser Cys Gln Val Met His
           195                 200                 205
   Glu Gly Ser Thr Cys Glu Lys Thr Val Ala Pro Ala Glu Cys Ser
       210                 215                 220
<210> 70
   <211> 223
   <212> PRT
   <213> Artificial Sequence
<220>
   <221> source
   <223> /note="Description of Artificial Sequence: Synthetic polypeptide"
<400> 70
   Gln Pro Val Leu His Gln Pro Pro Ala Met Ser Ser Ala Leu Gly Thr
   1 5 10 15
   Thr Ile Arg Leu Thr Cys Thr Leu Arg Asn Asp His Asp Ile Gly Val
   20 25 30
   Tyr Ser Val Tyr Trp Tyr Gln Gln Arg Pro Gly His Pro Pro Arg Phe
   35 40 45
   Leu Leu Arg Tyr Phe Ser Gln Ser Asp Lys Ser Gln Gly Pro Gln Val
   50 55 60
   Pro Pro Arg Phe Ser Gly Ser Lys Asp Val Ala Arg Asn Arg Gly Tyr
   65 70 75 80
   Leu Ser Ile Ser Glu Leu Gln Pro Glu Asp Glu Ala Met Tyr Tyr Cys
   85 90 95
   Ala Met Gly Ala Arg Ser Ser Val Thr His Val Phe Gly Ser Gly Thr
   100 105 110
   Gln Leu Thr Val Leu Ser Gln Pro Lys Ala Thr Pro Ser Val Thr Leu
   115 120 125
   Phe Pro Pro Ser Ser Glu Glu Leu Gln Ala Asn Lys Ala Thr Leu Val
   130 135 140
   Cys Leu Met Asn Asp Phe Tyr Pro Gly Ile Leu Thr Val Thr Trp Lys
   145 150 155 160
   Ala Asp Gly Thr Pro Ile Thr Gln Gly Val Glu Met Thr Thr Pro Ser
   165 170 175
   Lys Gln Ser Asn Asn Lys Tyr Ala Ala Ser Ser Tyr Leu Ser Leu Thr
   180 185 190
   Pro Glu Gln Trp Arg Ser Arg Arg Ser Tyr Ser Cys Gln Val Met His
   195 200 205
   Glu Gly Ser Thr Val Glu Lys Thr Cys Ala Pro Ala Glu Cys Ser
   210 215 220
<210> 71
   <211> 6
   <212> PRT
   <213> Artificial Sequence
<220>
   <221> source
   <223> /note="Description of Artificial Sequence: Synthetic 6xHis tag"
<400> 71
   His His His His His His
   1               5